# EUROPEAN PATENT SPECIFICATION

(11) **EP 1 423 389 B1**
(45) Date of publication and mention of the grant of the patent: **06.06.2007**
(21) Application number: 02759464.7
(22) Date of filing: 27.08.2002
(51) Int. Cl.: C07D 471/04, A61K 31/437, A61P 31/22

(54) **PYRAZOLO-PYRIDINES FOR THE TREATMENT OF HERPES INFECTIONS**
PYRAZOLO-PYRIDINE FÜR DIE BEHANDLUNG VON HERPES-ANSTECKUNGEN
PYRAZOLO-PYRIDINES SERVANT AU TRAITEMENT D'INFECTIONS HERPETIQUES

(30) Priority: 07.09.2001 US 318203 P
(43) Date of publication of application: 02.06.2004
(73) Proprietor: SMITHKLINE BEECHAM CORPORATION, Philadelphia, PA 19101 (US)
(72) Inventor: JOHNS, Brian A.; c/o GlaxoSmithKline, Research Triangle Park, NC 27709 (US); GUDMUNDSSON, Kristjan; c/o GlaxoSmithKline, Research Triangle Park, NC 27709 (US)
(74) Representative: Cooke, Tracey
(86) International application number: PCT/US2002/027251
(87) International publication number: WO 2003/022845

(56) References cited:
- WO-A-00/26216
- WO-A-00/52008
- WO-A-02/48147
- WO-A-02/48148
- WO-A-98/56377
- US-B1- 6 207 675
- A. AKAHANE ET. AL.: "Discovery of 6-Oxo-3-(2-phenylpyrazolo[1,5-a]pyridin-3- yl)- 1(6H)-pyridazinebutanoic Acid (FK838). A Novel Non-Xanthine Adenosine A1 Receptor Antagonist with Potent Diuretic Activity." JOURNAL OF MEDICINAL CHEMISTRY, vol. 42, no. 5, 11 March 1999 (1999-03-11), pages 779-83, XP001118346

## Description

### BACKGROUND OF THE INVENTION

The present invention relates to novel compounds, pharmaceutical formulations comprising these compounds, and the use of these compounds in therapy. More particularly, the present invention relates to compounds for the prophylaxis and treatment of herpes viral infections.

Of the DNA viruses, those of the herpes group are the sources of the most common viral illnesses in man. The group includes herpes simplex virus types 1 and 2 (HSV-1 and HSV-2), varicella zoster virus (VZV), cytomegalovirus (CMV), Epstein-Barr virus (EBV), human herpes virus type 6 (HHV-6), human herpes virus type 7 (HHV-7) and human herpes virus type 8 (HHV-8). HSV-1 and HSV-2 are some of the most common infectious agents of man. Most of these viruses are able to persist in the host's neural cells; once infected, individuals are at risk of recurrent clinical manifestations of infection which can be both physically and psychologically distressing.

Herpes simplex viruses (HSV-1 and -2) are the causative agents of herpes labialis and genital herpes. HSV infection is often characterised by extensive and debilitating lesions of the skin, mouth and/or genitals. Primary infections may be subclinical although tend to be more severe than infections in individuals previously exposed to the virus. Ocular infection by HSV can lead to keratitis or cataracts thereby endangering the host's sight Infection in the new-born, in immunocompromised patients or penetration of the infection into the central nervous system can prove fatal. In the US alone, 40 million individuals are infected with HSV-2, a number that is expected to increase to 60 million by 2007. Over 80% of individuals infected with HSV-2 are unaware they carry and spread the virus, and of those diagnosed less than 20% received oral therapies. The net result is that less than 5% of the infected population are treated. Likewise of the 530 million individuals worldwide who carry the HSV-1 virus, 81% of the symptomatic population remain untreated. No cure exists for HSV infection, and once infected, individuals carry the virus for life in a dormant state. Reactivation of the virus from latency occurs periodically and may be triggered by stress, environmental factors, and/or suppression of the host immune system. Currently, the use of nucleoside analogs such as valaciclovir (VALTREX®) and aciclovir (ZOVIRAX®) is the standard of care for managing genital herpes virus outbreaks.

VZV is a herpes virus which causes chickenpox and shingles. Chickenpox is the primary disease produced in a host without immunity, and in young children is usually a mild illness characterised by a vesicular rash and fever. Shingles or zoster is the recurrent form of the disease which occurs in adults who were previously infected with VZV. The clinical manifestations of shingles are characterised by neuralgia and a vesicular skin rash that is unilateral and dermatomal in distribution. Spread of inflammation may lead to paralysis or convulsions. Coma can occur if the meninges become affected. VZV is of serious concern in patients receiving immunosuppressive drugs for transplant purposes or for treatment of malignant neoplasia and is a serious complication of AIDS patients due to their impaired immune system.

In common with other herpes viruses, infection with CMV leads to a lifelong association of virus and host Congenital infection following infection of the mother during pregnancy may give rise to clinical effects such as death or gross disease (microcephaly, hepatosplenomegaly, jaundice, mental retardation), retinitis leading to blindness or, in less severe forms, failure to thrive, and susceptibility to chest and ear infections. CMV infection in patients who are immunocompromised for example as a result of malignancy, treatment with immunosuppressive drugs following transplantation or infection with Human Immunodeficiency Virus, may give rise to retinitis, pneumonitis, gastrointestinal disorders and neurological diseases. CMV infection is also associated with cardiovascular diseases and conditions including restenosis and atherosclerosis.

The main disease caused by EBV is acute or chronic infectious mononucleosis (glandular fever). Examples of other EBV or EBV associated diseases include lymphoproliferative disease which frequently occurs in persons with congenital or acquired cellular immune deficiency, X-linked lymphoproliferative disease which occurs namely in young boys, EBV-associated B-cell tumours, Hodgkin's disease, nasopharyngeal carcinoma, Burkitt lymphoma, non-Hodgkin's lymphoma, thymomas and oral hairy leukoplakia. EBV infections have also been found in association with a variety of epithelial-cell-derived tumours of the upper and lower respiratory tracts including the lung. EBV infection has also been associated with other diseases and conditions including chronic fatigue syndrome, multiple sclerosis and Alzheimer's disease.

HHV-6 has been shown to be a causative agent of infantum subitum in children and of kidney rejection and interstitial pneumonia in kidney and bone marrow transplant patients, respectively, and may be associated with other diseases such as multiple sclerosis. There is also evidence of repression of stem cell counts in bone marrow transplant patients. HHV-7 is of undetermined disease aetiology.

Hepatitis B virus (HBV) is a viral pathogen of world-wide major importance. The virus is aetiologically associated with primary hepatocellular carcinoma and is thought to cause 80% of the world's liver cancer. Clinical effects of infection with HBV range from headache, fever, malaise, nausea, vomiting, anorexia and abdominal pains. Replication of the virus is usually controlled by the immune response, with a course of recovery lasting weeks or months in humans, but infection may be more severe leading to persistent chronic liver disease outlined above.

U.S. Patent No. 5,498,774 and European Patent No. 0 404 190 to Mitsudera et al., relates to condensed heterocyclic compounds of the general formula (I): 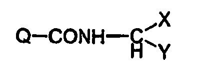 wherein Q is a condensed heterocyclic group having a nitrogen atom in the bridgehead which is unsubstituted or substituted, X is a hydrogen atom or a group attached through C, 0, S or N, and Y is an electron attractive group; or its salt which is useful as an agricultural chemical.

WO-A-00 26216 (Campbell *et al,* Glaxo Group Limited) relates to compounds of formula (I) 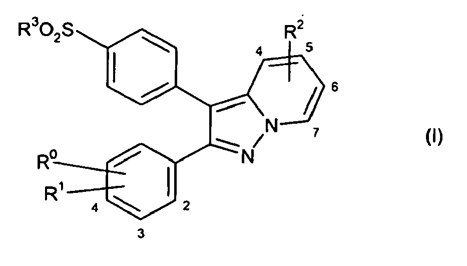 and pharmaceutically acceptable derivatives thereof in which:
R⁰ and R¹ are independently selected from H, halogen, C₁₋₆alkyl, C₁₋₆alkoxy, or C₁₋₆alkoxy substituted by one or more fluorine atoms;
R² is H, C₁₋₆alkyl. C₁₋₆alkyl substituted by one or more fluorine atoms, C₁₋₆alkoxy, C₁₋₆shydroxyalkyl, SC₁₋₆alkyl, C(O)H, C(O)C₁₋₆alkyl, C₁₋₆alkylsulphonyl, C₁₋₆alkoxy substituted by one or more fluorine atoms; and
R³ is C₁₋₆alkyl or NH₂.

Compounds of formula (I) are potent and selective inhibitors of COX-2 and are of use in the treatment of the pain, fever, inflammation of a variety of conditions and diseases.

WO-A-00 52008 (Campbell *et al,* Glaxo Group Limited) relates to compounds of formula (I) 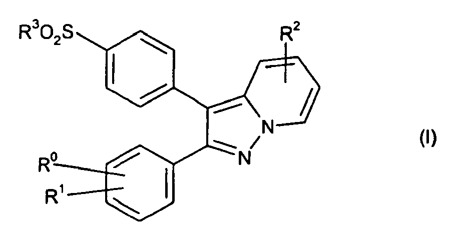 and pharmaceutically acceptable derivatives thereof in which:
R⁰ and R¹ are independently selected from H, halogen, C₁₋₆alkyl, C₁₋₆alkoxy, or C₁₋₆alkoxy substituted by one or more fluorine atoms;
R² is halogen, CN, CONR⁴R⁵, CO₂H, CO₂C₁₋₆alkyl, or NHSO₂R⁴;
R³ is C₁₋₆alkyl or NH₂; and

R⁴ and R⁵ are independently selected from H, C₁₋₆alkyl, phenyl, phenyl substituted by one or more atoms or groups (selected from halogen, C₁₋₆alkyl, C₁₋₆alkoxy, or C₁₋₆alkoxy substituted by one or more fluorine atoms), or together with the nitrogen atom to which they are attached form a saturated 4 to 8 membered ring.

Compounds of formula (I) are potent and selective inhibitors of COX-2 and are of use in the treatment of the pain, fever, inflammation of a variety of conditions and diseases.

US-B1-6,207,675 (Carry *et al,* Rhone-Poulenc Rorer S. A.) relates to pyrrole derivatives of general formula (I) useful for the treatment and prevention of diseases in which are involved viruses of the herpes family and/or cytokines in particular TNFα.

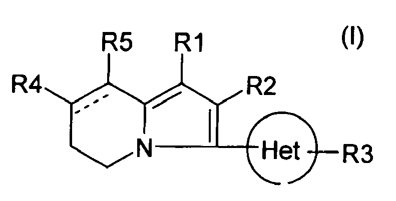

In the Journal of Medicinal Chemistry, vol 42, number 5, page 779-783 (1999) Akahane *et al* describe the design and synthesis of a series of 3-heteroaryl-2-phenylpyrazolo[1,5-a]pyridines and their pharmacological evaluation as selective adenosine A1 receptor antagonists for use as diuretics.

WO-A-98 56377 (Adams *et al,* SmithKline Beecham Corporation) relates to pyridyl or pyrimidinyl substituted pyrazole and pyrazoline compounds and compositions for use in therapy, in particular a method of treating a CSBP/RK/p38 kinase mediated disease in a mammal in need thereof.

### BRIEF SUMMARY OF THE INVENTION

According to a first aspect of the invention there is provided a compound of formula (I): 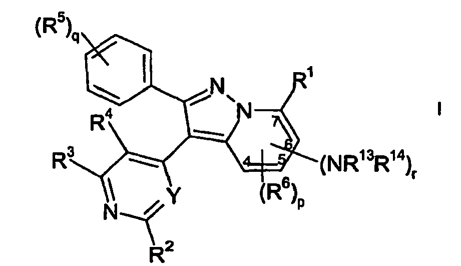 wherein:
R¹ is selected from the group consisting of alkyl, cycloalkyl, -OR⁷, -OAy, - C(O)R⁹, -CO₂R⁹, -C(O)NR⁷R⁸, -S(O)ₙR⁹, -S(O)ₙAy, -S(O)ₙHet, -S(O)₂NR⁷R⁸, R¹⁰cycloalkyl, -R¹⁰OR⁹, -R¹⁰NR⁷R⁸, cyano, nitro and azido;
   each R⁷ and R⁸ are the same or different and are each independently selected from the group consisting of H, alkyl, alkenyl, cycloalkyl, cycloalkenyl, -OR⁹, -C(O)R⁹, -CO₂R⁹, -C(O)NR⁹R¹¹, -C(S)NR⁹R¹¹, - C(NH)NR⁹R¹¹, -SO₂R¹⁰, -SO₂NR⁹R¹¹, -R¹⁰cycloalkyl, -R¹⁰OR⁹, - R¹⁰C(O)R⁹, -R¹⁰CO₂R⁹, -R¹⁰C(O)NR⁹R¹¹, -R¹⁰C(S)NR⁹R¹¹, -R¹⁰C(NH)NR⁹R¹¹, -R¹⁰SO₂R¹⁰, -R¹⁰SO₂NR⁹R¹¹, -R¹⁰NR⁹R¹¹, -R¹⁰NHCOR⁹, -R¹⁰NHC(NH)NR⁹R¹¹, -R¹⁰NHSO₂R⁹ and -R¹⁰SO₂NHCOR⁹;
   each R⁹ and R¹¹ are the same or different and are each independently selected from the group consisting of H, alkyl, cycloalkyl, - R¹⁰cycloalkyl, -R¹⁰OH, -R¹⁰(OR¹⁰)_{w} where w is 1-10, and -R¹⁰NR¹⁰R¹⁰;
   each R¹⁰ is the same or different and is independently selected from the group consisting of alkyl, alkenyl, alkynyl, cycloalkyl and cycloalkenyl;
   Ay is aryl;
   Het is a 5- or 6-membered heterocyclic or heteroaryl group;
   n is 0, 1 or 2;
R² is selected from the group consisting of Het, -OR⁷, -S(O)ₙR⁹, -NR⁷R⁸, - NHHet and -NHR¹⁰Het;
Y is N or CH;
R³ and R⁴ are the same or different and are each independently selected from the group consisting of H, halo, alkyl, alkenyl, cycloalkyl, Ay, Het, -OR⁷, -OAy, -C(O)R⁷, C(O)Ay, -CO₂R⁷, -CO₂Ay, -SO₂NHR⁹, -NR⁷R⁸, -NR⁷Ay, -NHHet, -NHR¹⁰Het, -R¹⁰OR⁷, -R¹⁰OAy, -R¹⁰NR⁷R⁸ and -R¹⁰NR⁷Ay;
q is 0, 1, 2, 3, 4 or 5;
each R⁵ is the same or different and is independently selected from the group consisting of halo, alkyl, alkenyl, alkynyl, cycloalkyl, cycloalkenyl, Ay, Het, -OR⁷, -OAy, -OHet, -C(O)R⁹, -C(O)Ay, -C(O)Het, -C(O)NR⁷R⁸,-C(S)NR⁹R¹¹, -C(O)NR⁷Ay, -C(O)NHR¹⁰Het, -CO₂R⁹, -C(NH)NR⁷R⁸, -C(NH)NR⁷Ay, -S(O)ₙR⁹, -S(O)ₙAy, -S(O)ₙHet, -S(O)₂NR⁷R⁸, -S(O)₂NR⁷Ay, -NR⁷R⁸, -NR⁷Ay, -NHHet, -NHR¹⁰Ay, -NHR¹⁰Het, -R¹⁰cycloalkyl, -R¹⁰OR⁹, -R¹⁰C(O)R⁹, -R¹⁰CO₂R⁹, -R¹⁰C(O)NR⁹R¹¹, -R¹⁰C(S)NR⁹R¹¹,-R¹⁰C(NH)NR⁹R¹¹, -R¹⁰SO₂R⁹, -R¹⁰SO₂NR⁹R¹¹, -R¹⁰SO₂NHCOR⁹, -R¹⁰NR⁷R⁸, -R¹⁰NR⁷Ay, -R¹⁰NHC(NH)NR⁹R¹¹, cyano, azido and nitro; or two adjacent R⁵ groups together with the atoms to which they are bonded form a C₅₋₆cycloalkyl or aryl;
r is 1,2 or 3;
each R¹³ and R¹⁴ are the same or different and are each independently selected from the group consisting of H, alkyl, alkenyl, cycloalkyl, cycloalkenyl, Ay, Het, -C(O)R⁹, -CO₂R⁹, -C(O)NR⁹R¹¹, -C(S)NR⁹R¹¹, -C(NH)NR⁹R¹¹, -SO₂R¹⁰,-SO₂NR⁹R¹¹, -R¹⁰cycloalkyl, -R¹⁰OR⁹, -R¹⁰C(O)R⁹, -R¹⁰CO₂R⁹, -R¹⁰C(O)NR⁹R¹¹,-R¹⁰C(S)NR⁹R¹¹, -R¹⁰C(NH)NR⁹R¹¹, -R¹⁰SO₂R¹⁰, -R¹⁰SO₂NR⁹R¹¹, -R¹⁰NR⁹R¹¹, -R¹⁰NHCOR⁹, -R¹⁰NHC(NH)NR⁹R¹¹, -R¹⁰NHSO₂R⁹ and -R¹⁰SO₂NHCOR⁹;
p is 0, 1 or 2, wherein p + r ≤ 3; and
each R⁶ is the same or different and is independently selected from the group consisting of halo, alkyl, Het, -OR⁷, -C(O)NR⁷R⁸, -S(O)ₙR⁹ -S(O)₂NR⁷R⁸, and cyano; or two adjacent R⁶ groups together with the atoms to which they are bonded form a C₅₋₆cycloalkyl or 5- or 6-membered heterocyclic group containing 1 or 2 heteroatoms;
wherein when Y is CH, R³ is not -NR⁷Ay;
and pharmaceutically acceptable salts and solvates thereof.

In another aspect of the invention there is provided a pharmaceutical composition comprising a compound of formula (I). In one particular embodiment, the pharmaceutical composition further comprises a pharmaceutically acceptable carrier or diluent In one embodiment, the pharmaceutical formulation further comprises an antiviral agent such as aciclovir, valaciclovir or a pharmaceutically acceptable salt thereof.

In another aspect, the present invention provides a process for preparing a compound of formula (I), wherein Y is N; R² is selected from the group consisting of Het, -OR⁷, -S(O)ₙR⁹, -NR⁷R⁸, -NHHet, and -NHR¹⁰Het. The process comprises the steps of:
a) reacting a compound of formula (XI): 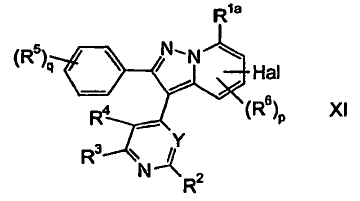 wherein Hal is halo; and
   R^{1a} is selected from the group consisting of H, alkyl, cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyl, -R¹⁰OR⁹, -R¹⁰NR⁷R⁸, cyano, azido and nitro;
   with an amine or imine to prepare a compound of formula (XII): 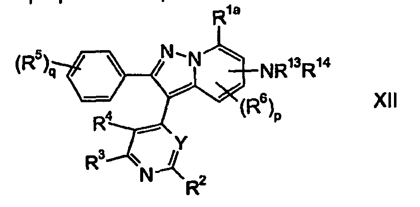 and
b) in the embodiment wherein R^{1a} is H, converting the compound of formula (XII) to a compound of formula (I).

In another embodiment, the present invention provides a process for preparing a compound of formula (I) wherein Y is N; R² is selected from the group consisting of Het, -OR⁷, -S(O)ₙR⁹, -NR⁷R⁸, -NHHet, and -NHR¹⁰Het; R³ is H and R⁴ is H. The process comprises the steps of:
a) reacting a compound of formula (IX): 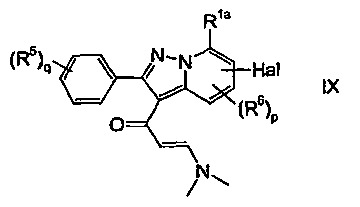 wherein Hal is halo; and
   R^{1a} is selected from the group consisting of H, alkyl, cycloalkyl. -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyl, -R¹⁰OR⁹, -R¹⁰NR⁷R⁸, cyano, azido and nitro;
   with a compound of formula (X): 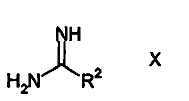 to prepare a compound of formula (XI);
b) reacting the compound of formula (XI) with an amine or imine to prepare a compound of formula (XII); and
c) in the embodiment wherein R^{1a} is H, converting the compound of formula (XII) to a compound of formula (I).

In another embodiment, the present invention provides a process for preparing a compound of formula (I) wherein Y is N; R² is selected from the group consisting of Het, -OR⁷, -S(O)nR⁹, -NR⁷R⁸, -NHHet, and -NHR¹⁰Het; R³ is selected from the group consisting of H, alkyl, alkenyl, cycloalkyl, Ay, Het, -C(O)R⁷, -C(O)Ay, -CO₂R⁷, -CO₂Ay, -SO₂NHR⁹ -NR⁷R⁸ where R⁷ and R⁸ are not H, -NR⁷Ay where R⁷ is not H, -R¹⁰OR⁷, -R¹⁰OAy, -R¹⁰NR⁷R⁸ and -R¹⁰NR⁷Ay; and R⁴ is H. The process comprises the steps of:
a) reacting a compound of formula (XXIX): 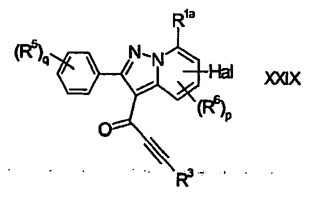 wherein Hal is halo; and
   R^{1a} is selected from the group consisting of H, alkyl, cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyl, -R¹⁰NR⁷R⁸, cyano, azido and nitro;
   with a compound of formula (X) to prepare a compound of formula (XI);
b) reacting the compound of formula (XI) with an amine or imine to prepare a compound of formula (XII); and
c) in the embodiment wherein R^{1a} is H, converting the compound of formula (XII) to a compound of formula (I).

In yet another embodiment, the present invention provides a process for preparing a compound of formula (I), wherein Y is N and R² is selected from the group consisting of Het, -OR⁷, -S(O)ₙR⁹, -NR⁷R⁸, -NHHet, and -NHR¹⁰Het. The process comprises the steps of:
a) reacting a compound of formula (XXXV): 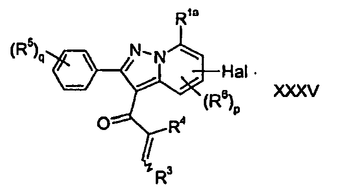 wherein Hal is halo; and
   R^{1a} is selected from the group consisting of H, alkyl, cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyl, -R¹⁰OR⁹, -R¹⁰NR⁷R⁸, cyano, azido and nitro;
   with a compound of formula (X) followed by oxidative aromatization, to prepare a compound of formula (XI);
b) reacting the compound of formula (XI) with an amine or imine to prepare a compound of formula (XII); and
c) in the embodiment wherein R^{1a} is H, converting the compound of formula (XII) to a compound of formula (I).

In another embodiment, the present invention provides a process for preparing a compound according of formula (I). The process comprises the steps of:
a) reacting a compound of formula (XL): 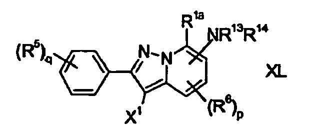 wherein X¹ is halo; and
   R^{1a} is selected from the group consisting of H, alkyl, cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyl, -R¹⁰OR⁹, -R¹⁰NR⁷R⁸, cyano, azido and nitro;
   with a compound of formula (XLI) 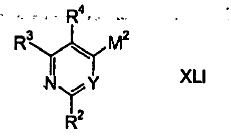 wherein M² is selected from the group consisting of -B(OH)₂, -B(ORa)₂, -B(Ra)₂, -Sn(Ra)₃, Zn-halide, ZnRa, Mg-halide where Ra is alkyl or cycloalkyl and halide is halo;
   to prepare a compound of formula (XII) and
c) in the embodiment wherein R^{1a} is H, converting the compound of formula (XII) to a compound of formula (I).

The process may further comprise the step of converting a compound of formula (XII) to a compound of formula (XII-D): 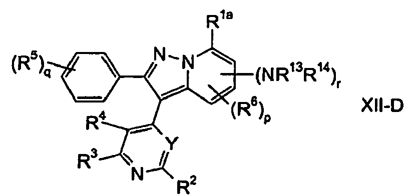 wherein r is 2 or 3.

The present invention also provides processes for converting a compound of formula (XII-E): 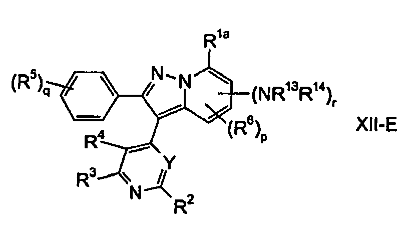 wherein r is 1, 2 or 3;
or a pharmaceutically acceptable salt or solvate thereof to another compound of formula (XII-E) or a pharmaceutically acceptable salt or solvate thereof and processes for converting a compound of formula (I) or a pharmaceutically acceptable salt or solvate thereof to another compound of formula (I) or a pharmaceutically acceptable salt or solvate thereof.

In another aspect, the present invention provides a radiolabeled compound of formula (I) or a pharmaceutically acceptable salt or solvate thereof. In one embodiment, the present invention provides a tritiated compound of formula (I) or a pharmaceutically acceptable salt or solvate thereof. In another embodiment, the present invention provides a biotinylated compound of formula (I) or a pharmaceutically acceptable salt or solvate thereof.

In another aspect, the present invention provides a compound of formula (I) for use in therapy.

In yet another aspect, the present invention provides a compound of formula (I) for use in the prophylaxis or treatment of a herpes viral infection in an animal such as a mammal, particularly a human.

In another aspect, the present invention provides a pharmaceutical composition comprising a compound of formula (I) for use in the prophylaxis or treatment of a herpes viral infection in an animal such as a mammal, particularly a human.

In yet another aspect, the present invention provides a compound of formula (I) for use in the prophylaxis or treatment of a condition or disease associated with a herpes viral infection in an animal, such as a mammal, particularly a human.

In yet another aspect, the present invention provides a pharmaceutical composition comprising a compound of formula (I) for use in the prophylaxis or treatment of a condition or disease associated with a herpes viral infection in an animal, such as a mammal, particularly a human.

In yet another aspect, the present invention provides the use of a compound of formula (I) for the preparation of a medicament for the prophylaxis or treatment of a herpes viral infection in an animal, such as a mammal, particularly a human.

In yet another aspect, the present invention provides the use of a compound of formula (I) for the preparation of a medicament for the treatment or prophylaxis of a condition or disease associated with a herpes viral infection in an animal, such as a mammal, particularly a human.

### DETAILED DESCRIPTION OF THE INVENTION

As used herein, "a compound of the invention" or "a compound of formula (I)" means a compound of formula (I) and pharmaceutically acceptable salts and solvates thereof. Similarly, with respect to isolatable intermediates such as compounds of formula (XI) (XII), (XII-D) and (XII-E), the phrase "a compound of formula (*number*)" means a compound having that formula and pharmaceutically acceptable salts and solvates thereof.

As used herein, the terms "alkyl" and "alkylene" refer to straight or branched hydrocarbon chains containing from 1 to 8 carbon atoms. Examples of "alkyl" as used herein include, but are not limited to, methyl, ethyl, n-propyl, n-butyl, n-pentyl, isobutyl, isopropyl, and tert-butyl. Examples of "alkylene" as used herein include, but are not limited to, methylene, ethylene, propylene, butylene, and isobutylene. "Alkyl" and "alkylene" also include substituted alkyl and substituted alkylene. The alkyl groups may be optionally substituted with one or more substituents selected from the group consisting of mercapto, nitro, cyano, azido and halo. Perhalo alkyl, such as trifluoromethyl is one particular alkyl group.

As used herein, the term "cycloalkyl" refers to a non-aromatic carbocyclic ring having from 3 to 8 carbon atoms (unless otherwise specified) and no carbon-carbon double bonds. "Cycloalkyl" includes by way of example cyclopropyl, cyclobutyl, cyclopentyl, cyclohexyl, cycloheptyl and cyclooctyl. "Cycloalkyl" also includes substituted cycloalkyl. The cyclalkyl may optionally be substituted on any available carbon with one or more substituents selected from the group consisting of mercapto, nitro, cyano, halo, and alkyl.

As used herein, the term "alkenyl" refers to straight or branched hydrocarbon chains containing from 2 to 8 carbon atoms and at least one and up to three carbon-carbon double bonds. Examples of "alkenyl" as used herein include, but are not limited to ethenyl and propenyl. "Alkenyl" also includes substituted alkenyl. The alkenyl groups may optionally be substituted on any available carbon with one or more substituents selected from the group consisting of mercapto, nitro, cyano, halo, and alkyl.

As used herein, the term "cycloalkenyl" refers to a non-aromatic carbocyclic ring having from 3 to 8 carbon atoms (unless otherwise specified) and up to 3 carbon-carbon double bonds. "Cycloalkenyl" includes by way of example cyclobutenyl, cyclopentenyl and cyclohexenyl. "Cycloalkenyl" also includes substituted cycloalkenyl. The cycloalkenyl may optionally be substituted on any available carbon(s) with one or more substituents selected from the group consisting of mercapto, nitro, cyano, halo and alkyl.

As used herein, the term "alkynyl" refers to straight or branched hydrocarbon chains containing from 2 to 8 carbon atoms and at least one and up to three carbon-carbon triple bonds. Examples of "alkynyl" as used herein include, but are not limited to ethynyl and propynyl. "Alkynyl" also includes substituted alkynyl. The alkynyl groups may optionally be substituted on any available carbon(s) with one or more substituents selected from the group consisting of mercapto, nitro, cyano, halo and alkyl.

The term "halo" or "halogen" refers to the elements fluorine, chlorine, bromine and iodine.

The term "aryl" refers to monocyclic carbocyclic groups and fused bicyclic carbocyclic groups having from 5 to 12 carbon atoms (unless otherwise specified) and having at least one aromatic ring. Examples of particular aryl groups include but are not limited to phenyl and naphthyl. "Aryl" also includes substituted aryl. Aryl groups may optionally be substituted on any available carbon(s) with one or more substituents selected from the group consisting of halo, alkyl (including haloalkyl), alkenyl, cycloalkyl, cycloalkenyl, alkoxy, cycloalkoxy, amino, mercapto, hydroxy, alkylhydroxy, alkylamine, cycloalkylamine, carboxy, carboxamide, sulfonamide, Het, amidine, cyano, nitro and azido. Particular aryl groups according to the invention include but are not limited to phenyl and substituted phenyl.

The term "heterocyclic" (or "heterocycle") refers to a monocyclic saturated or unsaturated non-aromatic groups and fused bicyclic non-aromatic groups, having the specified number of members and containing 1, 2, 3 or 4 heteroatoms selected from N, O and S. Examples of particular heterocyclic groups include but are not limited to tetrahydrofuran, dihydropyran, tetrahydropyran, pyran, oxetane, thietane, 1,4-dioxane, 1,3-dioxane, 1,3-dioxalane, piperidine, piperazine, tetrahydropyrimidine, pyrrolidine, morpholine, thiomorpholine, thiazolidine, oxazolidine, tetrahydrothiopyran, tetrahydrothiophene, and the like. "Heterocyclic" also includes substituted heterocyclic. The heterocyclic groups may optionally be substituted on any available carbon(s) or heteroatom(s) with one or more substituents selected from the group consisting of halo, alkyl (including perhaloalkyl), alkenyl, cycloalkyl, cycloalkenyl, hydroxy, alkoxy, cycloalkoxy, alkylhydroxy, mercapto, amino, alkylamine, cycloalkylamine, Het, amidine, carboxy, carboxamide, sulfonamide, cyano, nitro and azido. Particular heterocyclic groups according to the invention include but are not limited to pyrrolidine, piperidine, morpholine, thiomorpholine and piperazine, and substituted variants thereof.

The term "heteroaryl" refers to aromatic monocyclic groups and aromatic fused bicyclic groups having the specified number of members and containing 1, 2, 3, or 4 heteroatoms selected from N, O and S. Examples of particular heteroaryl groups include but are not limited to furan, thiophene, pyrrole, imidazole, pyrazole, triazole, tetrazole, thiazole, oxazole, isoxazole, oxadiazole, thiadiazole, isothiazole, pyridine, pyridazine, pyrazine, pyrimidine, quinoline, isoquinoline, benzofuran, benzothiophene, indole, and indazole. "Heteroaryl" also includes substituted heteroaryl. The heteroaryl groups may optionally be substituted on any available carbon(s) or heteroatom(s) with one or more substituents selected from the group consisting of halo, alkyl (including perhaloalkyl), alkenyl, cycloalkyl, cycloalkenyl, hydroxy, alkoxy, cycloalkoxy, alkylhydroxy, mercapto, amino, alkylamine, cycloalkylamine, Het, amidine, carboxy, carboxamide, sulfonamide, cyano, nitro and azido. Particular heteroaryl groups according to the invention include but are not limited to pyridine, furan, thiophene, pyrrole, imidazole, pyrazole, and pyrimidine, and substituted variants thereof.

The term "members" (and variants thereof e.g., "membered") in the context of heterocyclic and heteroaryl groups refers to the total atoms, carbon and heteroatoms N, O and/or S, which form the ring. Thus, an example of a 6-membered heterocyclic ring is piperidine and an example of a 6-membered heteroaryl ring is pyridine.

As used herein, the term "optionally" means that the subsequently described event(s) may or may not occur, and includes both event(s) that occur and events that do not occur.

The present invention provides compounds of formula (I): 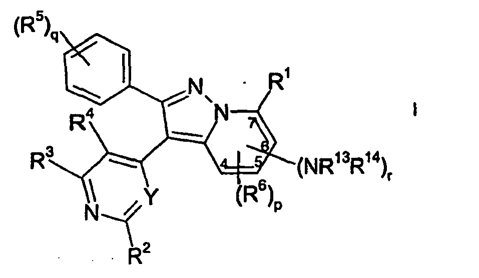 wherein:
R¹ is selected from the group consisting of alkyl, cycloalkyl, -OR⁷, -OAy, - C(O)R⁹, -CO₂R⁹, -C(O)NR⁷R⁸, -S(O)ₙR⁹, -S(O)ₙAy, -S(O)ₙHet, -S(O)₂NR⁷R⁸, - R¹⁰cycloalkyl, -R¹⁰OR⁹, -R¹⁰NR⁷R⁸, cyano, nitro and azido;
   each R⁷ and R⁸ are the same or different and are each independently selected from the group consisting of H, alkyl, alkenyl, cycloalkyl, cycloalkenyl, -OR⁹, -C(O)R⁹, -CO₂R⁹, -C(O)NR⁹R¹¹, -C(S)NR⁹R¹¹, - C(NH)NR⁹R¹¹, -SO₂R¹⁰, -SO₂NR⁹R¹¹, -R¹⁰cycloalkyl, -R¹⁰OR⁹, - R¹⁰C(O)R⁹, -R¹⁰CO₂R⁹, -R¹⁰C(O)NR⁹R¹¹, -R¹⁰C(S)NR⁹R¹¹, -R¹⁰C(NH)NR⁹R¹¹, -R¹⁰SO₂R¹⁰, -R¹⁰SO₂NR⁹R¹¹, -R¹⁰NR⁹R¹¹, -R¹⁰NHCOR⁹, -R¹⁰NHC(NH)NR⁹R¹¹, -R¹⁰NHSO₂R⁹ and -R¹⁰SO₂NHCOR⁹;
   each R⁹ and R¹¹ are the same or different and are each independently selected from the group consisting of H, alkyl, cycloalkyl, - R¹⁰cycloalkyl, -R¹⁰OH, -R¹⁰(OR¹⁰)_{w} where w is 1-10, and -R¹⁰NR¹⁰R¹⁰;
   each R¹⁰ is the same or different and is independently selected from the group consisting of alkyl, alkenyl, alkynyl, cycloalkyl and cycloalkenyl;
   Ay is aryl;
   Het is a 5- or 6-membered heterocyclic or heteroaryl group;
   n is 0, 1 or 2;
R² is selected from the group consisting of Het, -OR⁷, -S(O)ₙR⁹, -NR⁷R⁸, - NHHet and -NHR¹⁰Het;
Y is N or CH;
R³ and R⁴ are the same or different and are each independently selected from the group consisting of H, halo, alkyl, alkenyl, cycloalkyl, Ay, Het, -OR⁷, -OAy, -C(O)R¹, C(O)Ay, -CO₂R⁷, -CO₂Ay, -SO₂NHR⁹, -NR⁷R⁸, -NR⁷Ay, -NHHet, -NHR¹⁰Het, -R¹⁰OR⁷, -R¹⁰OAy, -R¹⁰NR⁷R⁸ and -R¹⁰NR⁷Ay;
q is 0, 1, 2, 3, 4 or 5;
each R⁵ is the same or different and is independently selected from the group consisting of halo, alkyl, alkenyl, alkynyl, cycloalkyl, cycloalkenyl, Ay, Het, -OR⁷, -OAy, -OHet, -C(O)R⁹, -C(O)Ay, -C(O)Het, -C(O)NR⁷R⁸, - C(S)NR⁹R¹¹, -C(O)NR⁷Ay, -C(O)NHR¹⁰Het, -CO₂R⁹, -C(NH)NR⁷R⁸, -C(NH)NR⁷Ay, -S(O)ₙR⁹, -S(O)ₙAy, -S(O)ₙHet, -S(O)₂NR⁷R⁸, -S(O)₂NR⁷Ay, -NR⁷R⁸, -NR⁷Ay, -NHHet, -NHR¹⁰Ay, -NHR¹⁰Het, -R¹⁰cycloalkyl, -R¹⁰OR⁹, -R¹⁰C(O)R⁹, -R¹⁰CO₂R⁹, -R¹⁰C(O)NR⁹R¹¹, -R¹⁰C(S)NR⁹R¹¹, - R¹⁰C(NH)NR⁹R¹¹, -R¹⁰SO₂R⁹, -R¹⁰SO₂NR⁹R¹¹, -R¹⁰SO₂NHCOR⁹, -R¹⁰NR⁷R⁸, -R¹⁰NR⁷Ay, -R¹⁰NHC(NH)NR⁹R¹¹, cyano, azido and nitro; or two adjacent R⁵ groups together with the atoms to which they are bonded form a C₅₋₆cycloalkyl or aryl;
r is 1, 2 or 3;
each R¹³ and R¹⁴ are the same or different and are each independently selected from the group consisting of H, alkyl, alkenyl, cycloalkyl, cycloalkenyl, Ay, Het, -C(O)R⁹, -CO₂R⁹, -C(O)NR⁹R¹¹, -C(S)NR⁹R¹¹, -C(NH)NR⁹R¹¹, -SO₂R¹⁰, - SO₂NR⁹R¹¹, -R¹⁰cycloalkyl, -R¹⁰OR⁹, -R¹⁰C(O)R⁹, -R¹⁰CO₂R⁹, -R¹⁰C(O)NR⁹R¹¹, - R¹⁰C(S)NR⁹R¹¹, -R¹⁰C(NH)NR⁹R¹¹, -R¹⁰SO₂R¹⁰, -R¹⁰SO₂NR⁹R¹¹, -R¹⁰NR⁹R¹¹, -R¹⁰NHCOR⁹, -R¹⁰NHC(NH)NR⁹R¹¹, -R¹⁰NHSO₂R⁹ and -R¹⁰SO₂NHCOR⁹;
p is 0, 1 or 2, wherein p + r ≤ 3; and
each R⁶ is the same or different and is independently selected from the group consisting of halo, alkyl, Het,
-OR⁷, -C(O)NR⁷R⁸, -S(O)ₙR⁹ -S(O)₂NR⁷R⁸, and cyano; or two adjacent R⁶ groups together with the atoms to which they are bonded form a C₅₋₆cycloalkyl or 5- or 6-membered heterocyclic group containing 1 or 2 heteroatoms;
wherein when Y is CH, R³ is not -NR⁷Ay;
and pharmaceutically acceptable salts and solvates thereof.

In one class of compounds of formula (I), Y is CH. In another class of compounds of formula (I), Y is N.

In one embodiment, compounds of formula (I) are defined where R¹ contains an aryl, heterocyclic or heteroaryl moiety (e.g., R¹ is selected from the group consisting of -OAy, -S(O)ₙAy and -S(O)ₙHet, or any subset thereof). In another embodiment, compounds of formula (I) are defined where R¹ contains a heterocyclic or heteroaryl moiety (e.g., R¹ is -S(O)ₙHet. In yet another embodiment, the compounds of formula (I) are defined where R¹ contains no aryl, heterocyclic or heteroaryl moiety (e.g., R¹ is selected from the group consisting of alkyl, cycloalkyl, -OR⁷, -C(O)R⁹, -CO₂R⁹, -C(O)NR⁷R⁸, -S(O)nR⁹, -S(O)₂NR⁷R⁸, -R¹⁰cycloalkyl, -R¹⁰OR⁹, cyano, azido and nitro, or any subset thereof). In another embodiment, R¹ contains no heteroaryl or heterocyclic moiety but may contain an aryl moeity (e.g., R¹ is selected from the group consisting of alkyl, cycloalkyl, -OR⁷, -OAy, -C(O)R⁹, -CO₂R⁹, -C(O)NR⁷R⁸, -S(O)nR⁹, -S(O)ₙAy, -S(O)₂NR⁷R⁸, -R¹⁰cycloalkyl, -R¹⁰OR⁹, -R¹⁰NR⁷R⁸, cyano, azido and nitro, or any subset thereof).

In one embodiment, R¹ is selected from the group consisting of alkyl, cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyl, -R¹⁰OR⁹, -R¹⁰NR⁷R⁸, cyano, nitro and azido, or any subset thereof. More particularly, R¹ is selected from the group consisting of alkyl, -OR⁷, -C(O)NR⁷R⁸, -S(O)ₙR⁹, -S(O)nAy, -S(O)ₙHet, -R¹⁰OR⁹ and -R¹⁰NR⁷R⁸, or any subset thereof. In one particular embodiment, R¹ is selected form the group consisting of alkyl, -OR⁷, -C(O)NR⁷R⁸ and S(O)nR⁹, or any subset thereof.

In one embodiment, the compounds of formula (I) are defined wherein when Y is CH and R¹ is -CO₂R⁹, R⁹ is not H. In another embodiment, the compounds of formula (I) are defined wherein when Y is CH, R¹ is -CONR⁷R⁸, and R⁷ is H, then R⁸ is not alkyl.

Specific examples of some particular R¹ groups are selected from the group consisting of methyl, ethyl, propyl, isopropyl, butyl, isobutyl, t-butyl, methoxy, ethoxy, propoxy, isopropoxy, butoxy, t-butoxy, thiomethoxy, thioethoxy, thioisopropoxy, thio-tert-butoxy, thiophenyl and -OCH₂CF₃, or any subset thereof.

In one embodiment, compounds of formula (I) are defined where R² contains an aryl, heterocyclic or heteroaryl moiety (e.g., R² is selected from the group consisting of Het, -NHHet, and -NHR¹⁰Het, or any subset thereof). In another embodiment, compounds of formula (I) are defined where R² contains a heterocyclic or heteroaryl moiety (e.g., R² is selected from the group consisting of Het, -NHHet, -NHR¹⁰Het, or any subset thereof). In yet another embodiment, the compounds of formula (I) are defined where R² contains no aryl, heterocyclic or heteroaryl moiety (e.g., R² is selected from the group consisting of -OR⁷, -S(O)ₙR⁹ and -NR⁷R⁸ or any subset thereof). In another embodiment, R² contains no heteroaryl or heterocyclic moiety but may contain an aryl moeity (e.g., R² is selected from the group consisting of -OR⁷, -S(O)ₙR⁹, and -NR⁷R⁸, or any subset thereof).

In one embodiment, R² is selected from the group consisting of Het, -OR⁷, -S(O)nR⁹, -NR⁷R⁸, -NHHet and -NHR¹⁰Het, or any subset thereof. More particularly, R² is selected from the group consisting of Het, -NR⁷R⁸, -NHR¹⁰Het and -NHHet, or any subset thereof. In one particular embodiment, the compounds of formula (I) are defined where R² is -NR⁷R⁸, Het or -NHHet, or any subset thereof. In one embodiment, R² is -NR⁷R⁸.

In one embodiment, R⁷ and R⁸ are each the same or different and are independently selected from the group consisting of H, alkyl, cycloalkyl, -C(O)R⁹, -R¹⁰-cycloalkyl, -R¹⁰OR⁹, -R¹⁰CO₂R⁹ and-R¹⁰NR⁹R¹¹, or any subset thereof. More particularly, R⁷ and R⁸ are each the same or different and are independently selected from the group consisting of H, alkyl, cycloalkyl, and -R¹⁰-cycloalkyl, or any subset thereof. In one embodiment, R⁷ and R⁸ are each the same or different and are independently selected from the group consisting of H, alkyl and cycloalkyl.

The group -R¹⁰(OR¹⁰)_{w} in the definition of R⁹ and R¹¹ refers to a PEG chain. In one embodiment, R⁹ and R¹¹ are each the same or different and are independently selected from the group consisting of H, alkyl, cycloalkyl, and -R¹⁰-cycloalkyl, or any subset thereof. More particularly, R⁹ and R¹¹ are each the same or different and are independently selected from the group consisting of H and alkyl.

In one embodiment, R¹⁰ is alkyl or cycloalkyl; more particularly alkyl.

More specifically, particular embodiments of the present invention include compounds of formula (I) wherein R² is selected from the group consisting of -NH-alkyl, -NH-cycloalkyl, Het, -NHHet and -NH-alkyl-Het, or any subset thereof. In one embodiment, the compounds of formula (I) are defined wherein R² is -NH-propyl, -NH-isopropyl, -NH-cyclopropyl, -NH-butyl, -NH-isobutyl, -NH-cyclobutyl, -NH-cyclopentyl, -NH-cyclohexyl, -NH(CH₂)₂OCH₃, pyrrolidine (e.g., pyrrolidine bonded through N), and morpholine (e.g., morpholine bonded through N), or any subset thereof.

In another embodiment, the compounds of formula (I) include those compounds defined where at least one of R³ and R⁴ contains an aryl, heterocyclic or heteroaryl moiety (or in one embodiment, a heterocyclic or heteroaryl moiety but exclude aryl moeities). A particular embodiment includes those compounds of formula (I) where neither R³ nor R⁴ contain an aryl, heterocyclic or heteroaryl moiety (or in one embodiment, neither contains a heterocyclic or heteroaryl moeity but may contain an aryl moiety). Based on the guidance given above for R¹ and R², one skilled in the art can readily determine the list of appropriate groups defining R³ and R⁴ which contain or exclude aryl, heterocyclic or heteroaryl moeities.

In one embodiment, R³ is selected from the group consisting of H, halo, alkyl, Ay, -OR⁷, -CO₂R⁷, -NR⁷R⁸, -R¹⁰OR⁷ and -R¹⁰NR⁷R⁸, or any subset thereof. More particularly, R³ is selected from the group consisting of H, halo, alkyl, -OR⁷, and -NR⁷R⁸, or any subset thereof. In one embodiment, R³ is H or alkyl. In one embodiment R³ is H.

In one particular embodiment, when Y is CH, R³ is not -NR⁷Ay.

In one embodiment, R⁴ is selected from the group consisting of H, halo, alkyl, Ay, -OR⁷, -CO₂R⁷, -NR⁷R⁸, -R¹⁰OR⁷ and -R¹⁰NR⁷R⁸, or any subset thereof. More particularly, R⁴ is selected from the group consisting of H, halo, alkyl, -OR⁷, and -NR⁷R⁸, or any subset ' thereof. In one embodiment, R⁴ is H or alkyl. In one embodiment R⁴ is H.

In one embodiment, q is 0,1 or 2. In one embodiment, q is 0. In another embodiment, q is 1.

R⁵ may be in the ortho, meta or para position.

Another class of compounds of formula (I) includes those compounds defined wherein at least one R⁵ group contains an aryl, heterocyclic or heteroaryl moiety (particularly a heterocyclic or heteroaryl moiety) and two adjacent R⁵ groups together with the atoms to which they are bonded do not form a C₅₋₆cycloalkyl or aryl. Another class of compounds of formula (I) includes those compounds defined wherein q is 3, 4 or 5, at least one R⁵ group contains an aryl, heterocyclic or heteroaryl moiety (particularly a heterocyclic or heteroaryl moiety) and two adjacent R⁵ groups together with the atoms to which they are bonded do form a C₅₋₆cycloalkyl or aryl. Another class of compounds of formula (I) includes those compounds defined where no R⁵ group contains an aryl, heterocyclic or heteroaryl moiety (or in one embodiment no R⁵ group contains a heterocyclic or heteroaryl moeity) and two adjacent R⁵ groups together with the atoms to which they are bonded do not form a C₅₋₆cycloalkyl or aryl. Another class of compounds of formula (I) includes those compounds defined wherein q is 2, 3, 4 or 5, no R⁵ group contains an aryl, heterocyclic or heteroaryl moiety (or in one embodiment no R⁵ group contains a heterocyclic or heteroaryl moiety) and two adjacent R⁵ groups together with the atoms to which they are bonded form a C₅₋₆cycloalkyl or aryl.

Based on the guidance given above for R¹ and R², one skilled in the art can readily determine the list of appropriate groups defining R⁵ which contain or exclude aryl, heterocyclic or heteroaryl moeities.

When two adjacent R⁵ groups together with the atoms to which they are bonded form a C₅₋₆cycloalkyl or aryl, q is typically 2, 3, 4 or 5; more particularly 2. By "two adjacent R⁵ groups" is meant that two R⁵ groups are bonded to adjacent carbon atoms. In such embodiments, each R⁵ group may be the same or different and is typically selected from the group consisting of alkyl and alkenyl. In one embodiment, two adjacent R⁵ groups are alkyl and together with the atoms to which they are bonded, they form a cycloalkyl group such as:

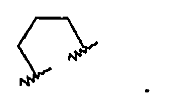

From this example, additional embodiments, including those where two adjacent R⁵ groups together with the atoms to which they are bonded form an aryl group can be readily ascertained by those skilled in the art

In one embodiment, two adjacent R⁵ groups together with the atoms to which they are bonded do not form a C₅₋₆cycloalkyl or aryl.

In one embodiment, each R⁵ is the same or different and is independently selected from the group consisting of halo, alkyl, alkenyl, Ay, Het, -OR⁷, -OAy, -CO₂R⁹, -C(O)NR⁷R⁸, -C(O)NR⁷Ay, -S(O)₂NR⁷R⁸, -NR⁷R⁸, -NR⁷Ay, -NHR¹⁰Ay, cyano, nitro and azido, or any subset thereof. More particularly, each R⁵ is the same or different and is independently selected from the group consisting of halo, alkyl, Het, -OR⁷, -C(O)NR⁷R⁸, -S(O)₂NR⁷R⁸, -NR⁷R⁸, cyano and nitro, or any subset thereof. In one embodiment, each R⁵ is the same or different and is independently selected from the group consisting of halo, alkyl, -OR⁷, -NR⁷R⁸, and cyano, or any subset thereof.

In particular, embodiments of the compounds of formula (I) are defined where R⁵ is selected from the group consisting of halo (e.g., fluoro or chloro), alkyl (e.g., methyl). 0-alkyl (e.g., O-methyl, O-isobutyl, and 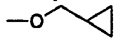 ), O-allyl, cyano, -NH-CH₃, and -M(CH₃)₂, or any subset thereof.

In one class of compounds of formula (I), p is 0, 1 or 2. More particularly, p is 0 or 1. In one embodiment, p is 0.

R⁶ may be in the C-4, C-5 or C-6 position.

One class of compounds of formula (I) includes those compounds defined wherein at least one R⁶ group contains an aryl, heterocyclic or heteroaryl moiety (particularly a heterocyclic or heteroaryl moiety) and two adjacent R⁶ groups together with the atoms to which they are bonded do not form a C₅₋₆cycloalkyl or a 5- or 6-membered heterocyclic group containing 1 or 2 heteroatoms. Another class of compounds of formula (I) includes those compounds defined where no R⁶ group contains an aryl, heterocyclic or heteroaryl moiety (or in one embodiment no R⁶ group contains a heterocyclic or heteroaryl moeity) and two adjacent R⁶ groups together with the atoms to which they are bonded do not form a C₅₋₆cycloalkyl or a 5- or 6-membered heterocyclic group containing 1 or 2 heteroatoms. Another class of compounds of formula (I) includes those compounds defined wherein p is 2, no R⁶ group contains an aryl, heterocyclic or heteroaryl moiety (or in one embodiment no R⁶ group contains a heterocyclic or heteroaryl moiety) and two adjacent R⁶ groups together with the atoms to which they are bonded do form a C₅₋₆cycloalkyl or a 5- or 6-membered heterocyclic group containing 1 or 2 heteroatoms. Based on the guidance given above for R¹ and R², one skilled in the art can readily determine the list of appropriate groups defining R⁶ which contain or exclude aryl, heterocyclic or heteroaryl moeities.

In those embodiments where two adjacent R⁶ groups together with the atoms to which they are bonded form a C₅₋₆ cycloalkyl or a 5- or 6-membered heterocyclic group having 1 or 2 heteroatoms, each R⁶ may be the same or different and is typically selected from the group consisting of alkyl, -OR⁷, and -S(O)ₙR⁹. For example, in one embodiment two adjacent R⁶ groups are -OR⁷ and together with the atoms to which they are bonded, they form a heterocyclic group such as:

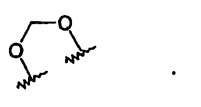

In another embodiment, two adjacent R⁶ groups are alkyl and together with the atoms to which they are bonded, they form a cycloalkyl group such as:

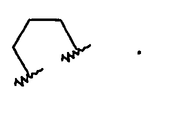

From these examples, additional embodiments can be readily ascertained by those skilled in the art In one embodiment, two R⁶ groups together with the atoms to which they are bonded do not form a C₅₋₆ cycloalkyl or a 5- or 6-membered heterocyclic group.

More particularly, each R⁶ is the same or different and is independently selected from the group consisting of halo, alkyl, Het, -OR⁷, -C(O)NR⁷R⁸, -S(O)₂NR⁷R⁸, -S(O)nR⁹ and cyano, or any subset thereof. In one embodiment, each R⁶ is the same or different and is independently selected from the group consisting of halo, Het, -OR⁷ and -S(O)nR⁹, or any subset thereof.

In another embodiment, the compounds of formula (I) are defined wherein when Y is CH, R⁶ is -CONR⁷R⁸, and R⁷ is H, then R⁸ is not alkyl.

In one embodiment, R⁶ is selected from the group consisting of Cl. Br, F, methyl, ethyl, isopropyl, pyrrolidine, morpholine, -OH, -O-alkyl, -CONH₂, -CONH-alkyl, -CON(alkyl)₂, -S-alkyl, -CF₃, and -SO₂NH₂, or any subset thereof. In one particular embodiment, R⁶ is selected from the group consisting of Cl, Br, F, methyl, ethyl, isopropyl, pyrrolidine, morpholine, -OH, -O-methyl, -O-isopropyl, -CONH₂, -CON(H)CH₃, -CON(CH₃)₂, -S-methyl, -S-ethyl, -S-isopropyl, -CF₃, and -SO₂NH₂, or any subset thereof. In one particular embodiment, R⁶ is halo, e.g., F or Cl. In one embodiment R⁶ is trifluoromethyl.

In one embodiment, r is 1.

The amine group (i.e., -(NR¹³R¹⁴)r) may be at the C-4, C-5 or C-6 position. In the embodiment wherein r is 1, the amine group is at the C-5 position.

In one embodiment, each R¹³ and R¹⁴ are the same or different and are each independently selected from the group consisting of H, alkyl, cycloalkyl, -C(O)R⁹, -CO₂R⁹, -R¹⁰cycloalkyl, -R¹⁰OR⁹ and -R¹⁰CO₂R⁹ and -R¹⁰NR⁹R¹¹, or any subset thereof. More particularly, each R¹³ and R¹⁴ are the same or different and are each independently selected from the group consisting of H, alkyl, cycloalkyl, -C(O)R⁹, -CO₂R⁹, -R¹⁰cycloalkyl and -R¹⁰OR⁹, or any subset thereof. In one particular embodiment, each R¹³ and R¹⁴ are the same or different and are each independently selected from the group consisting of H, alkyl and cycloalkyl, or any subset thereof.

Specific examples of groups defining R¹³ and R¹⁴ are selected from the group consisting of H, methyl, ethyl, isopropyl, n-butyl, cyclopropyl, cyclobutyl, cyclopentyl, and -(CH₂)₂OCH₃. In one particular embodiment, R¹³ and R¹⁴ are selected from the group consisting of H, n-butyl and cyclopentyl.

It is to be understood that the present invention includes all combinations and subsets of the particular groups defined hereinabove.

Examples of compounds of formula (I) include but are not limited to:
*N*-Cyclopentyl-3-[2-(cyclopentylamino)-4-pyrimidinyl]-7-(ethylsulfanyl)-2-(4-fluorophenyl)pyrazolo[1,5-*a*]pyridin-5-amine;
*N*-Cyclopentyl-3-[2-(cyclopentylamino)-4-pyrimidinyl]-2-(4-fluorophenyl)-7-methylpyrazolo[1,5-*a*]pyridin-5-amine;
*N*-Cyclopentyl-3-[2-(cyclopentylamino)-4-pyrimidinyl]-7-ethoxy-2-(4-fluorophenyl)pyrazolo[1,5-*a*]pyridin-5-amine;
*N*-Cyclopentyl-3-[2-(cyclopentylamino)-4-pyrimidinyl]-2-(4-fluorophenyl)-7-(methylsulfanyl)pyrazolo[1,5-*a*]pyridin-5-amine;
*N*-Cyclopentyl-3-[2-(cyclopentylamino)-4-pyrimidinyl]-2-(4-fluorophenyl)-7-(2-methoxyethoxy)pyrazolo[1,5-*a*]pyridin-5-amine;
*N*-Cyclopentyl-3-[2-(cyclopentylamino)-4-pyrimidinyl]-2-(4-methoxyphenyl)-7-(methylsulfanyl)pyrazolo[1,5-*a*]pyridin-5-amine;
*N*-Cyclopentyl-3-[2-(cyclopentylamino)-4-pyrimidinyl]-7-(ethylsulfanyl)-2-(4-methoxyphenyl)pyrazolo[1,5-*a*]pyridin-5-amine;
*N*-Cyclopentyl-3-[2-(cyclopentylamino)-4-pyrimidinyl]-7-(isopropylsulfanyl)-2-(4-methoxyphenyl)pyrazolo[1,5-*a*]pyridin-5-amine;
*N*-Cyclopentyl-3-[2-(cyclopentylamino)-4-pyrimidinyl]-2-(4-methoxyphenyl)-7-(phenylsulfanyl)pyrazolo[1,5-*a*]pyridin-5-amine; and
*N*-Cyclopentyl-3-[2-(cyclopentylamino)-4-pyrimidinyl]-2-(4-fluorophenyl)-7-(isopropylsulfanyl)pyrazolo[1,5-*a*]pyridin-5-amine; and
pharmaceutically acceptable salts and solvates thereof.

It will be appreciated by those skilled in the art that the compounds of the present invention may also be utilized in the form of a pharmaceutically acceptable salt or solvate thereof. The pharmaceutically acceptable salts of the compounds of formula (I) include conventional salts formed from pharmaceutically acceptable inorganic or organic acids or bases as well as quaternary ammonium salts. More specific examples of suitable acid salts include hydrochloric, hydrobromic, sulfuric, phosphoric, nitric, perchloric, fumaric, acetic, propionic, succinic, glycolic, formic, lactic, maleic, tartaric, citric, palmoic, malonic, hydroxymaleic, phenylacetic, glutamic, benzoic, salicylic, fumaric, toluenesulfonic, methanesulfonic (mesylate), naphthalene-2-sulfonic, benzenesulfonic hydroxynaphthoic, hydroiodic, malic, steroic, tannic and the like. In one embodiment, the compound of formula (I) is in the form of the mesylate salt Other acids such as oxalic, while not in themselves pharmaceutically acceptable, may be useful in the preparation of salts useful as intermediates in obtaining the compounds of the invention and their pharmaceutically acceptable salts. More specific examples of suitable basic salts include sodium, lithium, potassium, magnesium, aluminium, calcium, zinc, N,N'-dibenzylethylenediamine, chloroprocaine, choline, diethanolamine, ethylenediamine, N-methylglucamine and procaine salts.

The term "solvate" as used herein refers to a complex of variable stoichiometry formed by a solute (a compound of formula (I)) and a solvent Solvents, by way of example, include water, methanol, ethanol, or acetic acid.

Processes for preparing pharmaceutically acceptable salts and solvates of the compound of formula (I) are conventional in the art. See, for example Burger's Medicinal Chemistry And Drug Discovery, 5th Edition, Vol 1: Principles And Practice.

As will be apparent to those skilled in the art, in the processes descibed below for the preparation of compounds of formula (I), certain intermediates (including but not limited to compounds of formula (XI), (XII), (XII-A), (XII-B), (XII-C) and (XII-D), may be in the form of pharmaceutically acceptable salts or solvates the compound. Those terms as applied to any intermediate employed in the process of preparing compounds of formula (I) have the same meanings as noted above with respect to compounds of formula (I). Processes for preparing pharmaceutically acceptable salts and solvates of such intermediates are known in the art and are analogous to the process for preparing pharmaceutically acceptable salts, and solvates the compounds of formula (I).

Certain compounds of formula (I) and intermediates used in the processes of preparing compounds of formula (I) may exist in stereoisomeric forms (e.g. they may contain one or more asymmetric carbon atoms or may exhibit *cis-trans* isomerism). The individual stereoisomers (enantiomers and diastereomers) and mixtures of these are included within the scope of the present invention. The present invention also covers the individual isomers of the compounds represented by formula (I) as mixtures with isomers thereof in which one or more chiral centers are inverted. Likewise, it is understood that compounds of formula (I) may exist in tautomeric forms other than that shown in the formula and these are also included within the scope of the present invention.

The present invention further provides compounds of formula (I) for use in medical therapy, e.g. in the treatment or prophylaxis, including suppression of recurrence of symptoms, of a viral disease in an animal, e.g. a mammal such as a human. The compounds of formula (I) are especially useful for the treatment or prophylaxis of viral diseases such as herpes viral infections. Herpes viral infections include, for example, herpes simplex virus 1 (HSV-1), herpes simplex virus 2 (HSV-2), cytomegalovirus (CMV) (including CMV in organ transplant patients being treated with immunosuppressants), Epstein Barr virus (EBV), varicella zoster virus (VZV), human herpes virus 6 (HHV-6), human herpes virus 7 (HHV-7), and human herpes virus 8 (HHV-8). Thus, the compounds of the invention are also useful in the treatment or prophylaxis of the symptoms or effects of herpes virus infections.

The compounds of the invention are useful in the treatment or prophylaxis of a condition or disease associated with a herpes virus infection, particularly conditions or diseases associated with latent herpes virus infections in an animal, e.g., a mammal such as a human. By conditions or diseases associated with herpes viral infections is meant a condition or disease, excluding the viral infection per se, which results from the presence of the viral infection, such as chronic fatigue syndrome which is associated with EBV infection; and multiple sclerosis which has been associated with herpes viral infections such as EBV and HHV-6. Further examples of such conditions or diseases are described in the background section above.

In addition to those conditions and diseases, the compounds of the present invention may also be used for the treatment or prophylaxis of cardiovascular diseases and conditions associated with a herpes virus infection, in particular atherosclerosis, coronary artery disease and restenosis and specifically restenosis following angioplasty (RFA). Restenosis is the narrowing of the blood vessels which can occur after injury to the vessel wall, for example injury caused by balloon angioplasty or other surgical and/or diagnostic techniques, and is characterized by excessive proliferation of smooth muscle cells in the walls of the blood vessel treated. It is thought that in many patients suffering from restenosis following angioplasty, viral infection, particularly by CMV and/or HHV-6 plays a pivotal role in the proliferation of the smooth muscle cells in the coronary vessel. Restenosis can occur following a number of surgical and/or diagnostic techniques, for example, transplant surgery, vein grafting, coronary by-pass grafting and, most commonly following angioplasty.

There is evidence from work done both *in vitro* and *in vivo,* indicating that restenosis is a multifactorial process. Several cytokines and growth factors, acting in concert, stimulate the migration and proliferation of vascular smooth muscle cells (SMC) and production of extracellular matrix material, which accumulate to occlude the blood vessel. In addition growth suppressors act to inhibit the proliferation of SMC's and production of extracellular matrix material.

In addition, compounds of formula (I) may be useful in the treatment or prophylaxis of hepatitis B or hepatitis C viruses, human papilloma virus (HPV) and HIV.

As used herein, the term "prophylaxis" refers to the prevention of infection, the prevention of occurrence of symptoms in an infected subject, the prevention of recurrence of symptoms in an infected subject, or a decrease in severity or frequency of symptoms of viral infection, condition or disease in the subject

As used herein, the term "treatment" refers to the partial or total elimination of symptoms or decrease in severity of symptoms of viral infection, condition or disease in the subject, or the elimination or decrease of viral presence in the subject.

As used herein, the term "therapeutically effective amount" means an amount of a compound of formula (I) which is sufficient, in the subject to which it is administered, to treat or prevent the stated disease, condition or infection. For example, a therapeutically effective amount of a compound of formula (I) for the treatment of a herpes virus infection is an amount sufficient to treat the herpes virus infection in the subject

The present invention also provides the use of the compound of formula (I) in the preparation of a medicament for the treatment or prophylaxis of a viral infection in an animal such as a mammal (e.g., a human), particularly a herpes viral infection; the use of the compound of formula (I) in the preparation of a medicament for the treatment of a condition or disease associated with a herpes viral infection; and the use of the compound of formula (I) in the preparation of a medicament for the treatment or prophylaxis of hepatitis B or hepatitis C viruses, human papilloma virus or HIV. In particular, the present invention also provides the use of a compound of formula (I) in the preparation of a medicament for the treatment or prophylaxis of chronic fatigue syndrome or multiple sclerosis. In one embodiment, the present invention provides the use of a compound of formula (I) in the preparation of a medicament for the treatment or prophylaxis of cardiovascular disease, such as restenosis or atherosclerosis.

The compounds of formula (I) are conveniently administered in the form of pharmaceutical compositions. Such compositions may conveniently be presented for use in conventional manner in admixture with one or more physiologically acceptable carriers or diluents.

While it is possible that compounds of the present invention may be therapeutically administered as the raw chemical, it is preferable to present the active ingredient as a pharmaceutical composition. The pharmaceutical composition may comprise one or more pharmaceutically acceptable carriers or diluents. The carrier(s) or diluent(s)must be "acceptable" in the sense of being compatible with the other ingredients of the formulation and not deleterious to the recipient thereof.

Accordingly, the present invention further provides for a pharmaceutical formulation or composition comprising a compound of formula (I). In one embodiment, the pharmaceutical formulation further comprises one or more pharmaceutically acceptable carriers or dilents and, optionally, other therapeutic and/or prophylactic ingredients.

The formulations include those suitable for oral, parenteral (including subcutaneous e.g. by injection or by depot tablet, intradermal, intrathecal, intramuscular e.g. by depot and intravenous), rectal and topical (including dermal, buccal and sublingual) administration although the most suitable route may depend upon for example the condition, age, and disorder of the recipient as well as the viral infection or disease being treated. The formulations may conveniently be presented in unit dosage form and may be prepared by any of the methods well known in the art of pharmacy. All methods include the step of bringing into association the compound(s) ("active ingredient") with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation. Formulations suitable for oral administration may be presented as discrete units such as capsules (including soft-gel capsules), cachets or tablets (e.g. chewable tablets in particular for paediatric administration) each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with other conventional excipients such as binding agents, (for example, syrup, acacia, gelatin, sorbitol, tragacanth, mucilage of starch or polyvinylpyrrolidone), fillers (for example, lactose, sugar, microcrystalline cellulose, maize-starch, calcium phosphate or sorbitol), lubricants (for example, magnesium stearate, stearic acid, talc, polyethylene glycol or silica), disintegrants (for example, potato starch or sodium starch glycollate) or wetting agents, such as sodium lauryl sulfate. Moulded tablets may be made by moulding in a suitable machine a mixture of the powdered compound moistened with an inert liquid diluent. The tablets may optionally be coated or scored and may be formulated so as to provide slow or controlled release of the active ingredient therein. The tablets may be coated according to methods well-known in the art

Alternatively, the compounds of the present invention may be incorporated into oral liquid preparations such as aqueous or oily suspensions, solutions, emulsions, syrups or elixirs, for example. Moreover, formulations containing these compounds may be presented as a dry product for constitution with water or other suitable vehicle before use. Such liquid preparations may contain conventional additives such as suspending agents such as sorbitol syrup, methyl cellulose, glucose/sugar syrup, gelatin, hydroxyethylcellulose, carboxymethyl cellulose, aluminum stearate gel or hydrogenated edible fats; emulsifying agents such as lecithin, sorbitan mono-oleate or acacia; non-aqueous vehicles (which may include edible oils) such as almond oil, fractionated coconut oil, oily esters, propylene glycol or ethyl alcohol; and preservatives such as methyl or propyl p-hydroxybenzoates or sorbic acid. Such preparations may also be formulated as suppositories, e.g., containing conventional suppository bases such as cocoa butter or other glycerides. Liquid preparations may also be formulated as soft-gel capsules for oral administration, e.g., containing conventional soft-gel excipients such as polyethylene glycol.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The formulations may be presented in unit-dose or multi-dose containers, for example sealed ampoules and vials, and may be stored in a freeze-dried (lyophilised) condition requiring only the addition of a sterile liquid carrier, for example, water-for-injection, immediately prior to use. Extemporaneous injection solutions and suspensions may be prepared from sterile powders, granules and tablets of the kind previously described.

Formulations for rectal administration may be presented as a suppository with the usual carriers such as cocoa butter, hard fat or polyethylene glycol.

Formulations suitable for topical (e.g., dermal) or intranasal application include ointments, creams, lotions, pastes, gels, sprays, aerosols and oils. Suitable carriers for such formulations include petroleum jelly, lanolin, polyethyleneglycols, alcohols, and combinations thereof.

Formulations for topical administration in the mouth, for example buccally or sublingually, include lozenges comprising the active ingredient in a flavoured base such as sucrose and acacia or tragacanth, and pastilles comprising the active ingredient in a base such as gelatin and glycerin or sucrose and acacia.

The compounds may also be formulated as depot preparations. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt

In addition to the ingredients particularly mentioned above, the formulations may include other agents conventional in the art having regard to the type of formulation in question, for example those suitable for oral administration may include flavouring agents.

It will be appreciated that the amount of a compound of the invention required for use in treatment will vary with the nature of the condition being treated and the age and the condition of the patient and will be ultimately at the discretion of the attendant physician or veterinarian. In general, however, doses employed for adult human treatment will typically be in the range of 0.02-5000 mg per day, more particularly 100-1500 mg per day. The desired dose may conveniently be presented in a single dose or as divided doses administered at appropriate intervals, for example as two, three, four or more sub-doses per day. The formulations according to the invention may contain between 0.1-99% of the active ingredient, conveniently from 30-95% for tablets and capsules and 3-50% for liquid preparations.

The compound of formula (I) for use in the instant invention may be used in combination with other therapeutic agents for example, non-nucleotide reverse transcriptase inhibitors, nucleoside reverse transcriptase inhibitors, protease inhibitors and/or other antiviral agents. The invention thus provides in a further aspect the use of a combination comprising a compound of formula (I) with a further therapeutic agent in the treatment of viral infections. Particular antiviral agents which may be combined with the compounds of the present invention include aciclovir, valaciclovir, famcyclovir, ganciclovir, docosanol, miribavir, amprenavir, lamivudine, zidovudine, and abacavir, as well as pharmaceutically acceptable salts or solvates thereof. Particular antiviral agents for combining with the compounds of the present invention include aciclovir and valaciclovir. Thus the present invention provides in a further aspect, a combination comprising a compound of formula (I) and aciclovir or valaciclovir; the use of such combination in the treatment of viral infections, and the preparation of a medicament for the treatment of a viral infection, and a method of treating viral infections comprising administering a compound of formula (I) and an antiviral agent selected from the group consisting of aciclovir and valaciclovir.

When the compounds of formula (I) are used in combination with other therapeutic agents, the compounds may be administered either sequentially or simultaneously by any convenient route.

The combinations referred to above may conveniently be presented for use in the form of a pharmaceutical formulation and thus pharmaceutical formulations comprising a combination as defined above optionally together with a pharmaceutically acceptable carrier or diluent comprise a further aspect of the invention. The individual components of such combinations may be administered either sequentially or simultaneously in separate or combined pharmaceutical formulations.

When combined in the same formulation it will be appreciated that the two compounds must be stable and compatible with each other and the other components of the formulation and may be formulated for administration. When formulated separately they may be provided in any convenient formulation, in such a manner as are known for such compounds in the art

When a compound of formula (I) is used in combination with a second therapeutic agent active against the viral infection, the dose of each compound may differ from that when the compound is used alone. Appropriate doses will be readily appreciated by those skilled in the art

Compounds of formula (I) are prepared using the methods described below. The following methods and schemes describe processes for preparing compounds of formula (XII). As described below, the compounds of formula (XII) may be converted to compounds of formula (XII-E): 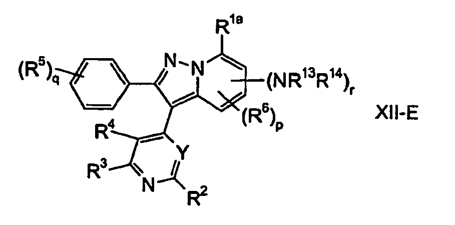 wherein r is 1, 2 or 3;
R^{1a} is selected from the group consisting of H, alkyl, cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)2NR⁷R⁸, -R¹⁰cycloalkyl, -R¹⁰OR⁹, -R¹⁰NR⁷R⁸, cyano, azido and nitro; and all other variables are as defined above.

It will be appreciated by those skilled in the art that the compounds of formula (XII-E) encompass the compounds of formula (I) and are in fact the same as the compounds of formula (I) when compounds of formula (XII-E) are defined wherein R^{1a} = R¹. When the compound of formula (XII) or (XII-E) are defined wherein R^{1a} = H, the compound of formula (XII) or (XII-E) may be converted into a compound of formula (I) using methods described below.

Compounds of formula (I) and (XII) wherein Y is N; R² is selected from the group consisting of Het, -OR⁷, -S(O)nR⁹, -NR⁷R⁸, -NHHet, and -NHR¹⁰Het ; R³ and R⁴ are both H; and r is 1, may be conveniently prepared by the process outlined in Scheme 1 below. 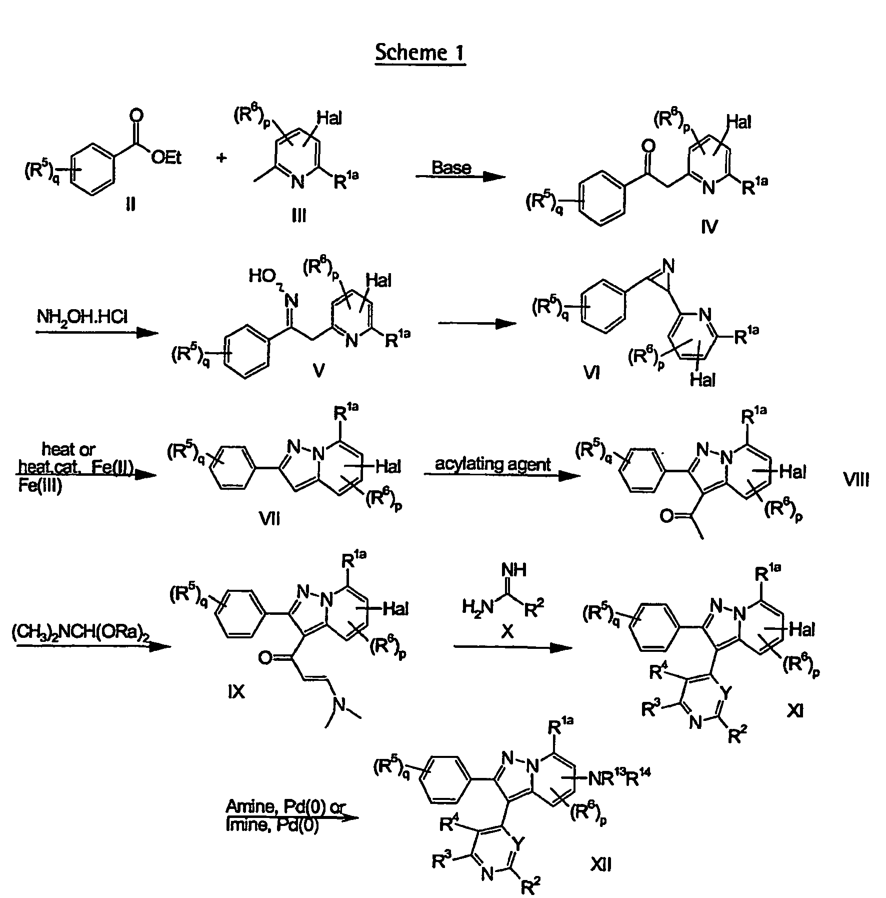 wherein:
Hal is halo;
R^{1a} is selected from the group consisting of H, alkyl, cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyl, -R¹⁰OR⁹, -R¹⁰NR⁷R⁸, cyano, azido and nitro (i.e., R^{1a} is H or R¹ above);
Y is N;
R² is selected from the group consisting of Het, -OR⁷, -S(O)ₙR⁹, -NR⁷R⁸, -NHHet, and -NHR¹⁰Het,
R³ and R⁴ are both H;
r is 1;
Ra is alkyl or cycloalkyl; and
all other variables are as defined above.

Generally, the process for preparing the compounds of formula (I) and (XII) wherein Y is N; R² is selected from the group consisting of Het, -OR⁷, -S(O)ₙR⁹, -NR⁷R⁸, -NHHet, and -NHR¹⁰Het; R³ and R⁴ are both H; and r is 1, (all formulas and all other variables having been defined above) comprises the steps of:
(a) reacting a picoline compound of formula (III) with a benzoylating agent of formula (II) to prepare a compound of formula (IV);
(b) reacting the compound of formula (IV) with a hydroxylamine source to prepare a compound of formula (V);
(c) reacting the compound of formula (V) with an acylating or sulfonylating agent to prepare a compound of formula (VI);
(d) rearranging the compound of formula (VI) to prepare a compound of formula (VII);
(e) acylating the compound of formula (VII) to prepare a compound of formula (VIII);
(f) reacting the compound of formula (VIII) with a dimethylformamide dialkyl acetal of formula (CH₃)₂NCH(ORa)₂ to prepare a compound of formula (IX);
(g) reacting the compound of formula (IX) with a compound of formula (X) to prepare a compound of formula (XI);
(h) reacting the compound of formula (XI) with an amine or imine to prepare a compound of formula (XII); and
(i) in the embodiment wherein R^{1a} is H, converting the compound of formula (XII) to a compound of formula (I).

More specifically, compounds of formula (I) and (XII) wherein Y is N; R² is selected from the group consisting of Het, -OR⁷, -S(O)ₙR⁹, -NR⁷R⁸,-NHHet, and -NHR¹⁰ Het, R³ and R⁴ are both H; and r is 1, can be prepared by reacting the compound of formula (XI) with an amine or imine to prepare a compound of formula (XII); and when R^{1a} is H, converting to a compound of formula (I). 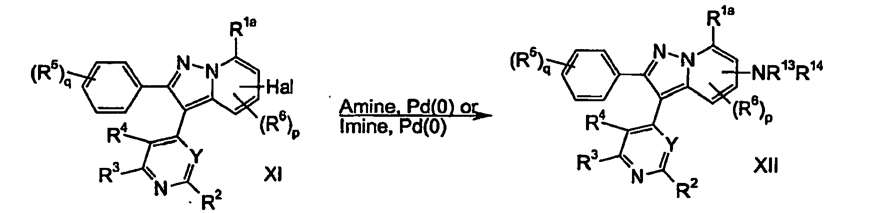 wherein all variables are as defined above.

This reaction can be carried out via an adaptation of procedures found in the literature (See, Wolfe, J. P.; Buchwald, S. L *J. Org. Chem.* **2000**, 65, 1144, the disclosure of which is incorporated herein by reference in its entirety) wherein a compound of formula (XI) is treated with an amine, a palladium (0) or nickel (0) source and a base, optionally in a suitable solvent. Suitable sources of palladium (0) include but are not limited to palladium(II) acetate and tris(dibenzylideneacetone) dipalladium (0). Typical bases for use in the reaction include, for example sodium *tert*-butoxide and cesium carbonate. Toluene is an example of a suitable solvent. Suitable amines which will give the amine group (-NR¹³R¹⁴) are commercially available or can be prepared using conventional techniques.

Alternatively, the process of reacting a compound of formula (XI) with an amine to prepare a compound of formula (XII) is carried out by reacting a compound of formula (XI) with an imine in the presence of a palladium (0) source, a base and a suitable ligand, followed by hydrolysis to give a compound of formula (XII). See J. Wolfe, et al., *Tetrahedron Letters* 38:6367-6370 (1997), the disclosure of which is incorporated herein by reference in its entirety. Typically the imine is benzophenoneimine, the palladium (0) source is tris(dibenrylideneacetone)-dipalladium(0), the base is sodium *tert*-butoxide and the ligand is *racemic*-2,2'-bis(diphenylphosphino)-1,1'-binaphthyl. Suitable solvents include *N,N*-dimethylformamide, toluene and the like. Hydrolysis can conveniently be carried out by using aqueous hydrochloric acid or the like.

The foregoing processes for converting the halogen substituent (Hal) to the amine group (-NR¹³R¹⁴) is described as occurring at the end of the synthesis, however, one skilled in the art will readily appreciate that the conversion of the halogen to the amine can occur at earlier stages both in this process and in the following synthesis routes as well. For example, any of the halogenated intermediates may be converted to the amine analogues prior to proceeding with the next step of the synthesis. This would of course eliminate the need for performing this conversion as the final step. The various permutations of the synthesis described herein wherein the conversion of the halogen to the amine occurs earlier in the synthesis are contemplated by the instant invention and encompassed within its scope. Thus, the order of the foregoing steps of the synthesis is not critical to obtaining the compounds of formula (XII).

The compounds of formula (XI) may be conveniently prepared by reacting a compound of formula (IX) with a compound of formula (X). 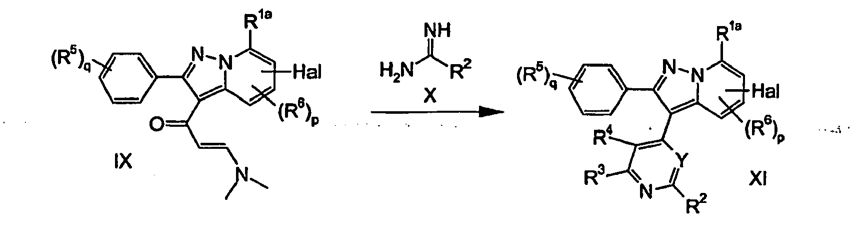 wherein all variables are as defined above.

This method can be readily carried out by mixing a compound of formula (IX) with a compound of formula (X) in a suitable solvent, optionally in the presence of a base (particularly when the amidine is in a salt form), and heating the reaction to 50-150°C. Typical solvents include lower alcohols such as methanol, ethanol, isopropanol, dimethylformamide and the like. The base is typically a sodium alkoxide, potassium carbonate, or an amine base such as triethylamine. In one embodiment, the solvent is dimethylformamide and the base is potassium carbonate, or an amine base such as triethylamine.

Compounds of the formula (IX) may be conveniently prepared by reacting a compound of formula (VIII) with a dimethylformamide dialkyl acetal of formula (CH₃)₂NCH(ORa)₂, wherein Rₐ is alkyl or cycloalkyl. 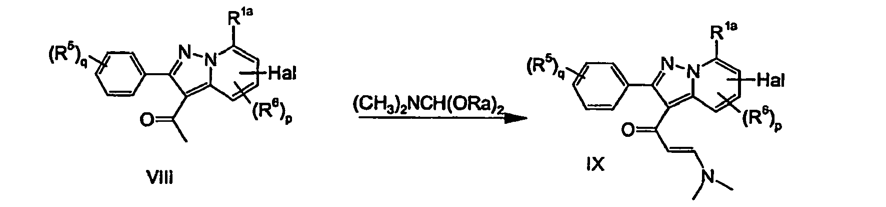 wherein all variables are as defined above.

Typical dimethylformamide dialkylacetal compounds for use in this method include but are not limited to dimethylformamide dimethylacetal and dimethylformamide di-tert-butylacetal. The reaction is carried out by mixing a compound of formula (VIII) with the dimethylformamide dialkyl acetal, optionally with heating. A compound of the formula (VIII) may be conveniently prepared from a compound of the formula (VII) using an acylation procedure. 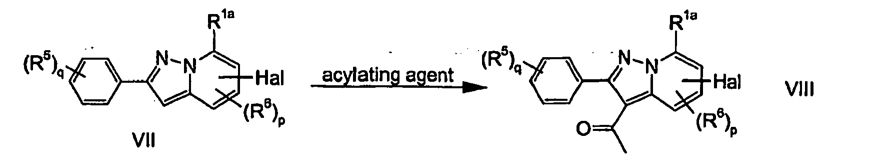 wherein all variables are as defined above.

Typically the acylation is carried out by treating the compound of formula (VII) with an acylating agent, optionally in the presence of an acid or Lewis acid catalyst in an inert solvent with optional heating. Typical acylating agents will be readily determined by those skilled in the art One particular acylating agent is acetic anhydride. Lewis acid catalysts are also known to those skilled in the art One particular Lewis acid catalyst for use in this reaction is boron trifluoride diethyl etherate. A suitable solvent is toluene.

A compound of formula (VII) is conveniently prepared by rearranging an azirine compound of formula (VI). 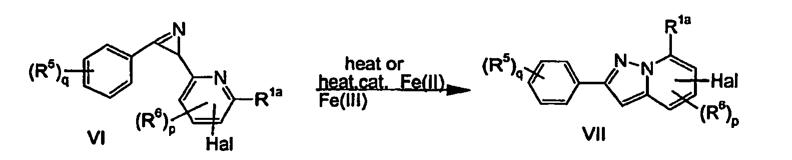 wherein all variables are as defined above.

The rearrangement of the azirine of formula (VI) can be accomplished by heating a solution of the azirine of formula (VI) in a suitable solvent at a temperature of about 160-200°C. Suitable inert solvents include, but are not limited to, 1-methyl-2-pyrrolidinone, and 1,2,4-trichlorobenzene. A more preferred method for rearrangement of the azirine of formula (VI) to a compound of formula (VII) involves reacting the compound of formula (VI) with ferrous chloride (FeCl₂) or ferric chloride (FeCl₃). This reaction is typically done in an inert solvent with heating. A suitable solvent for this reaction is 1,2-dimethoxyethane and the like.

Typically the azirines of formula (VI) are prepared from oxime compounds of formula (V) by treatment with acylating or sulfonylating agents in the presence of a base. 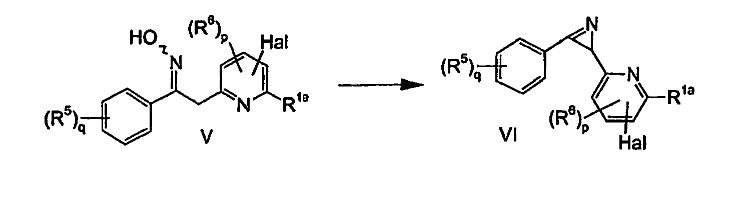 wherein all variables are as defined above.

Typical acylating or sulfonylating agents include but are not limited to, acetic anhydride, trifluoroacetic anhydride, methanesulfonyl chloride, toluenesulfonyl chloride and the like. Typical bases include, but are not limited to, triethylamine, diisopropylethylamine, pyridine, and the like. The reaction may be carried out in an inert solvent such as for example, chloroform, dichloromethane, toluene or the like.

The oxime compound of formula (V) is readily prepared by treating the ketone compound of formula (IV) with a hydroxylamine source, in a suitable solvent, and optionally with a base. 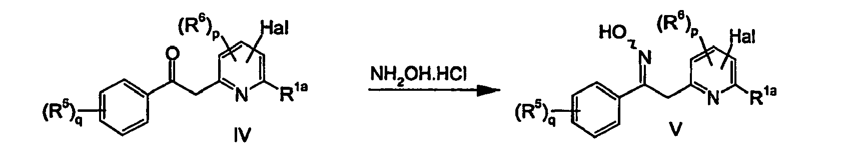 wherein all variables are as defined above.

In one embodiment, the hydroxylamine is hydroxylamine hydrochloride and the base is an aqueous solution of sodium hydroxide. Suitable solvents include lower alcohols such as methanol, ethanol, or isopropanol.

The ketone compounds of formula (IV) can be prepared by treatment of a picoline compound of formula (III) with a benzoylating agent of formula (II) in the presence of a base. 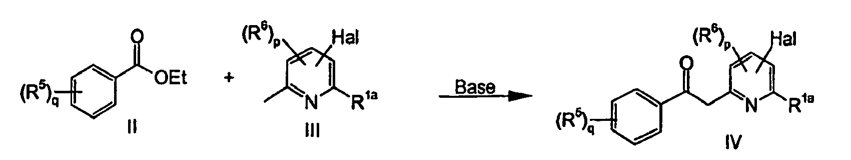 wherein all variables are as defined above.

The benzoylating agents of formula (II) and the picoline compounds of formula (III) are commercially available or may be prepared using conventional methods known to those skilled in the art. An example of a particular picoline is chloropicoline.

Benzoylating agents of formula (II) include, but are not limited to, benzoyl esters. An example of a suitable base is lithium bis(trimethylsilyl)amide in an inert solvent such as tetrahydrofuran. Ketones such as those of formula (IV) can be readily prepared using procedures known to one skilled in the art and/or described in the literature (See, Casity, R.P.; Taylor, LT.; Wolfe, J.F. *J. Org. Chem.* **1978**; 2286).

In another embodiment of the present invention, certain compounds of formula (I) and (XII) wherein Y is N; R² is selected from the group consisting of Het, -OR⁷, -S(O)ₙR⁹, -NR⁷R⁸, -NHHet, and -NHR¹⁰Het, R³ and R⁴ are both H and r is 1, may be conveniently prepared by the process outlined in Scheme 1-A below. 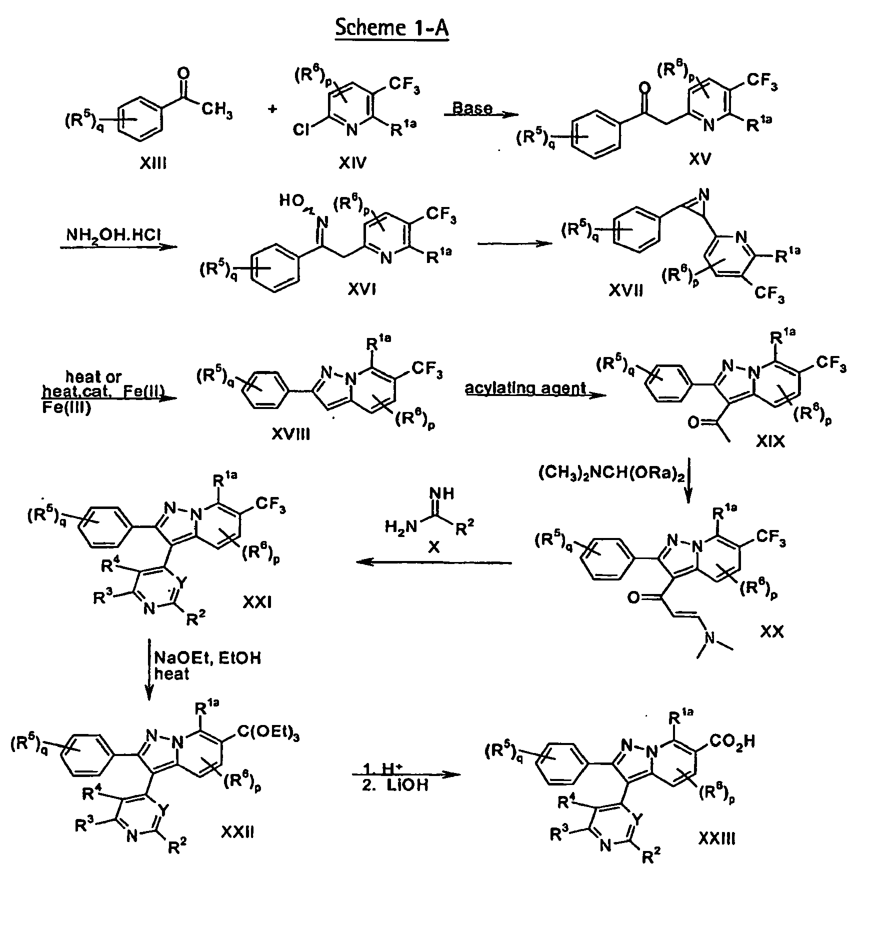 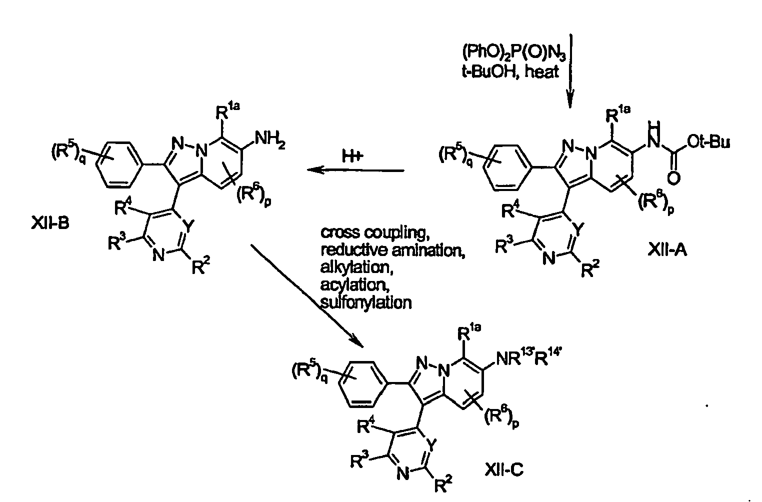 wherein:
R^{1a} is selected from the group consisting of H, alkyl, cycloalkyl, -OR⁷, - OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyl, -R¹⁰OR⁹, -R¹⁰NR⁷R⁸, cyano, azido and nitro;
Y is N;
R² is selected from the group consisting of Het, -OR⁷, -S(O)ₙR⁹, -NR⁷R⁸, -NHHet, and -NHR¹⁰Het,
R³ and R⁴ are both H;
Ph is phenyl;
Ra is alkyl or cycloalkyl;
r is 1;
each R^{13'} and R^{14'} are the same or different and are each independently selected from the group consisting of alkyl, alkenyl, cycloalkyl, cycloalkenyl, Ay, Het, -C(O)R⁹, -CO₂R⁹, -C(O)NR⁹R¹¹, -C(S)NR⁹R¹¹, -C(NH)NR⁹R¹¹, -SO₂R¹⁰, -SO₂NR⁹R¹¹, -R¹⁰cycloalkyl. -R¹⁰OR⁹, -R¹⁰C(O)R⁹, -R¹⁰CO₂R⁹, -R¹⁰C(O)NR⁹R¹¹, -R¹⁰C(S)NR⁹R¹¹, -R¹⁰C(NH)NR⁹R¹¹, -R¹⁰SO₂R¹¹, -R¹⁰SO₂NR⁹R¹¹, -R¹⁰NR⁹R¹¹, -R¹⁰NHC(NH)NR⁹R¹¹, -R¹⁰NHSO₂R⁹, -R¹⁰NHCOR⁹ and -R¹⁰SO₂NHCOR⁹; and
all other variables are as defined above.

Generally, the process for preparing these compounds of formula (I) and (XII) wherein Y is N; R² is selected from the group consisting of Het, -OR⁷, -S(O)ₙR⁹, -NR⁷R⁸, -NHHet, and -NHR¹⁰Het; R³ and R⁴ are both H; and r is 1, (all formulas and all other variables having been defined above) comprises the steps of:
(a) reacting a 2-chloro-5-trifluoromethylpyridine of formula (XIV) with an acetophenone of formula (XIII) to prepare a compound of formula (XV);
(b) reacting the compound of formula (XV) with a hydroxylamine source to prepare a compound of formula (XVI);
(c) reacting the compound of formula (XVI) with an acylating or sulfonylating agent to prepare a compound of formula (XVII);
(d) rearranging the compound of formula (XVII) to prepare a compound of formula (XVIII);
(e) acylating the compound of formula (XVIII) to prepare a compound of formula (XIX);
(f) reacting the compound of formula (XIX) with a dimethylformamide dialkyl acetal of formula (CH₃)₂NCH(ORa)₂ to prepare a compound of formula (XX);
(g) reacting the compound of formula (XX) with a compound of formula (X) to prepare a compound of formula (XXI);
(h) reacting the compound of formula (XXI) with sodium ethoxide to prepare a compound of formula (XXII);
(i) reacting the compound of formula (XXII) with an acid, followed by hydrolysis of the resulting ester to prepare a compound of formula (XXlll);
(j) reacting the compound of formula (XXIII) with diphenylphosphoryl azide in tert-butanol to prepare a compound of formula (XII-A);
(k) optionally cleaving the compound of formula (XII-A) to give a compound of formula (XII-B);
(l) optionally converting the compound of formula (XII-B) to a compound of formula (XII-C) using conditions selected from the group consisting of cross coupling, reductive amination, alkylation, acylation and sulfonylation; and
(m) in the embodiment wherein R^{1a} is H, converting the compound of formula (XII-A), (XII-B) or (XII-C) to a compound of formula (I).

More specifically, certain compounds of formula (I) and (XII) wherein Y is N; R² is selected from the group consisting of Het, -OR⁷, -S(O)ₙR⁹, -NR⁷R⁸, -NHHet; and -NHR¹⁰Het, R³ and R⁴ are both H; and r is 1, can be prepared by cleaving the compound of formula (XII-A) to prepare a compound of formula (XII-B), using acid. 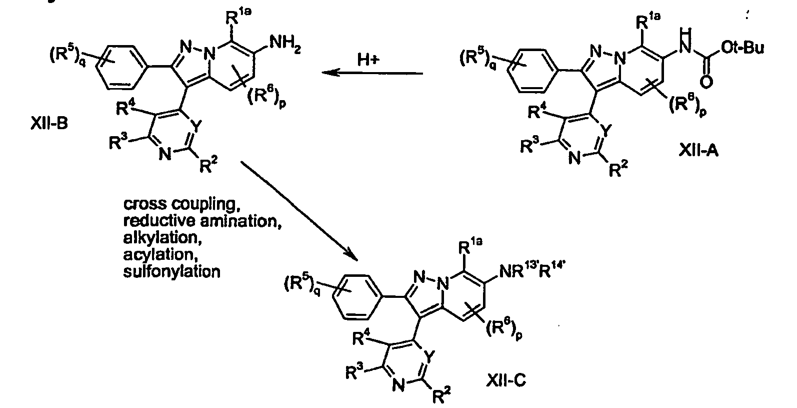 wherein all variables are as defined above.

The compound of formula (XII-A) may be further converted to a compound of formula (XII-B) by acid catalyzed hydrolysis of the *tert*-butyl carbamate in a suitable solvent

Suitable acids include hydrochloric acid and trifluoroacetic acid and the like. Suitable solvents include dioxane, diethyl ether, tetrahydrofuran, dichloromethane and the like.

The compound of formula (XII-B) may optionally be converted to another compound of formula (XII) (i.e., a compound of formula (XII-C)), by cross coupling, reductive amination, alkylation, acylation or sulfonylation, depending upon the particular compound of formula (XII) that is desired. One skilled in the art will readily be able to convert compounds of formula (XII-B) to compounds of formula (XII-C) using these general techniques.

A compound of formula (XII-A) can be conveniently prepared by a Curtius rearrangement, from a compound of formula (XXIII). 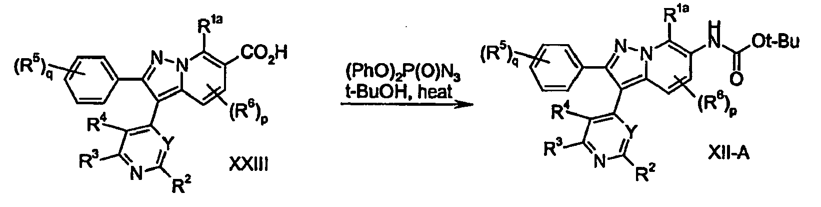 wherein all variables are as defined above.

The rearrangement can be performed by treating a compound of formula (XXIII) with diphenylphosphoryl azide in tert-butanol in the presence of a base with heating. Other carboxylic acid derived migratory rearrangements commonly known to one skilled in the art (such as the Lossen, Hofmann, and Schmidt reactions) may also be useful for this transformation.

A compound of formula (XXIII) can be conveniently prepared by reacting a compound of formula (XXII) with an acid, followed by hydrolysis of the resulting ester. 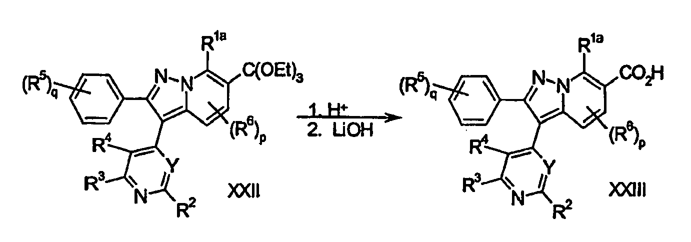 wherein all varibles are as defined above.

Suitable acids include but are not limited to p-toluenesulfonic acid, camphorsulfonic acid, pyridinium p-toluenesulfonic acid and the like. An appropriate solvent such as acetone may be used. The hydrolysis can be performed using lithium hydroxide and the like in a pure or mixed solvent system including but not limited to solvents such as tetrahydrofuran, methanol, and water.

A compound of formula (XXII) can be prepared by treating a compound of formula (XXI) with an alkoxide salt in an alcohol solvent 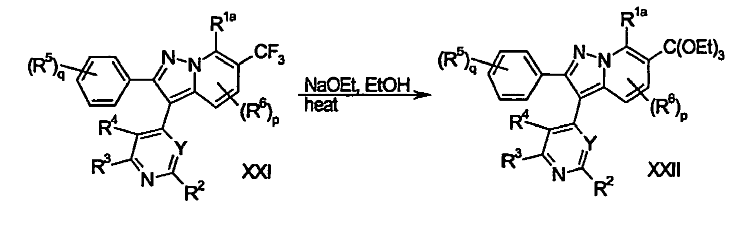 wherein all variables are as defined above.

Suitable conditions for the foregoing reaction include the use of sodium ethoxide as the alkoxide, and ethanol as the solvent The reaction may optionally be heated to 60°C.

Compounds of formula (XXI) can be prepared using methods analogous to those described above in Scheme 1 for the preparation of compounds of formula (XI), with the exception that the first step (i.e., the preparation of compounds of formula (XV)) involves the condensation of a 2-chloro-5-trifluoromethylpyridine of formula (XIV) with an acetophenone of formula (XIII) under basic conditions, in place of the reaction of the picoline of formula (III) with the benzoylating agent of formula (II) as is employed in the synthesis of the compound of formula (IV), according to Scheme 1.

In a further embodiment of the present invention, compounds of formula (I) and (XII) wherein Y is N; R² is selected from the group consisting of Het, -OR⁷, -S(O)nR⁹, -NR⁷R⁸, -NHHet, and -NHR¹⁰Het; R³ is selected from the group consisting of H, alkyl, cycloalkyl, alkenyl, Ay, Het, -C(O)R⁷, -C(O)Ay, -CO₂R⁷, -CO₂Ay, -SO₂NHR⁹, -NR⁷R⁸ (where R⁷ and R⁸ are not H), -NR⁷Ay (where R⁷ is not H), -R¹⁰OR⁷, -R¹⁰OAy, -R¹⁰NR⁷R⁸ and -R¹⁰NR⁷Ay; R⁴ is H; and r is 1, may be conveniently prepared by the process outlined in Scheme 2 below. 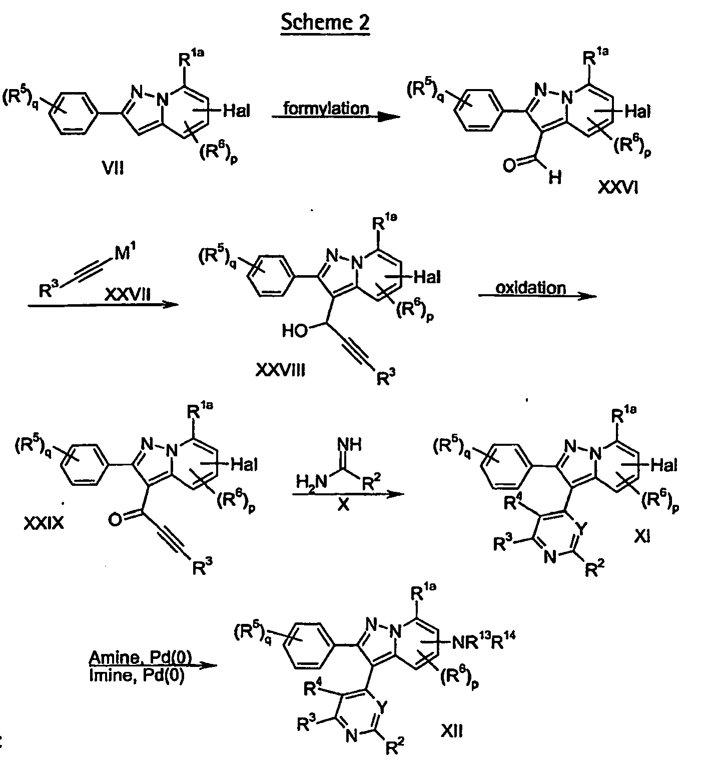 wherein:
- Hal: is halo;
- R^{1a}: is selected from the group consisting of H, alkyl, cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyl, R¹⁰OR⁹, -R¹⁰NR⁷R⁸, cyano, azido and nitro;
- Y: is N;
- R²: is selected from the group consisting of Het, -OR⁷, -S(O)ₙR⁹, -NR⁷R⁸, -NHHet, and -NHR¹⁰Het:
- R³: is selected from the group consisting of H, alkyl, cycloalkyl, alkenyl, Ay, Het, -C(O)R⁷, -C(O)Ay, -CO₂R⁷, -CO₂Ay, -SO₂NHR⁹, -NR⁷R⁸ (where R⁷ and R⁸ are not H), -NR⁷Ay (where R⁷ is not H), -R¹⁰OR⁷, -R¹⁰OAy, -R¹⁰NR⁷R⁸ and -R¹⁰NR⁷Ay;
- R⁴: is H;
- M¹: is Li, Mg-halide or cerium-halide, wherein halide is halo; and
all other variables are as defined above.

Generally, the process for preparing compounds of formula (I) and (XII) wherein Y is N; R² is selected from the group consisting of Het, -OR⁷, -S(O)ₙR⁹, -NR⁷R⁸, -NHHet, and -NHR¹⁰Het; R³ is selected from the group consisting of H, alkyl, cycloalkyl, alkenyl, Ay, Het, -C(O)R⁷, -C(O)Ay, -CO₂R⁷, -CO₂Ay, -SO₂NHR⁹, -NR⁷R⁸ (where R⁷ and R⁸ are not H), -NR⁷Ay (where R⁷ is not H), -R¹⁰OR⁷, -R¹⁰OAy, -R¹⁰NR⁷R⁸ and -R¹⁰NR⁷Ay; R⁴ is H; and r is 1, (all other variables having been defined above) comprises the following steps:
(a) formylating a compound of formula (VII) to prepare a compound of formula (XXVI);
(b) reacting the compound of formula (XXVI) with a compound of formula (XXVII) to prepare a compound of formula (XXVIII);
(c) oxidizing the compound of formula (XXVIII) to prepare a compound of formula (XXIX);
(d) reacting the compound of formula (XXIX) with a compound of formula (X) to prepare a compound of formula (XI);
(e) replacing the halogen of the compound of formula (XI) with an amine or imine to prepare a compound of formula (XII); and
(f) in the embodiment wherein R^{1a} is H, converting the compound of formula (XII) to a compound of formula (I).

More specifically, compounds of formula (I) and (XII) wherein Y is N; R² is selected from the group consisting of Het, -OR⁷, -S(O)ₙR⁹, -NR⁷R⁸, -NHHet, and -NHR¹⁰Het, R³ is selected from the group consisting of H, alkyl, cycloalkyl, alkenyl, Ay, Het, -C(O)R⁷, -C(O)Ay, -CO₂R⁷, -CO₂Ay, -SO₂NHR⁹, -NR⁷R⁸ (where R⁷ and R⁸ are not H), -NR⁷Ay (where R⁷ is not H). -R¹⁰OR⁷, -R¹⁰OAy, -R¹⁰NR⁷R⁸ and -R¹⁰NR⁷Ay; R⁴ is H; and r is 1, may be prepared by replacing the halogen on the compound of formula (XI) with an amine nucleophile as described above in connection with Scheme 1.

Compounds of formula (XI) can be prepared by reacting a compound of formula (XXIX) with a compound of formula (X). 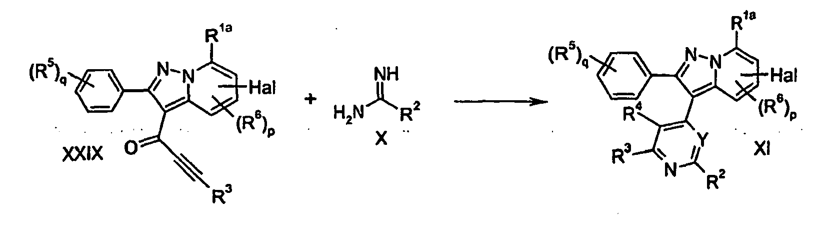 wherein all variables are as defined above.

This method can be readily carried out by mixing a compound of formula (XXIX) with a compound of formula (X) in a suitable solvent, optionally in the presence of a base. The reaction may be heated to 50-150°C or performed at ambient temperature. Typical solvents include but are not limited to lower alcohols such as methanol, ethanol, isopropanol and the like. Typical bases include for example, sodium alkoxide, potassium carbonate, or an amine base such as triethylamine. In another embodiment, the solvent is *N,N*-dimethylformamide and the base is potassium carbonate, or an amine base such as triethylamine.

A compound of formula (XXIX) may be conveniently prepared by oxidation of a compound of formula (XXVIII). 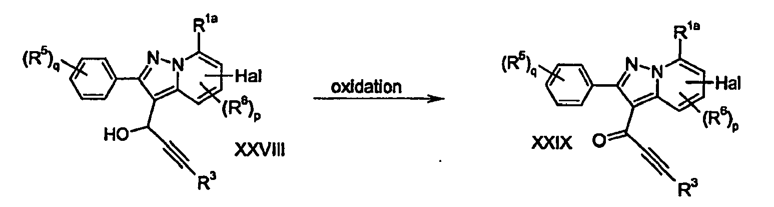 wherein all variables are as defined above.

Suitable oxidizing agents include but are not limited to, manganese dioxide, and the like, in an inert solvent Suitable inert solvents include but are not limited to, dichloromethane, chloroform, *N,N*-dimethylformamide, ether, and the like.

A compound of formula (XXVIII) may be conveniently prepared by reacting a compound of formula (XXVI) with a compound of formula (XXVII). 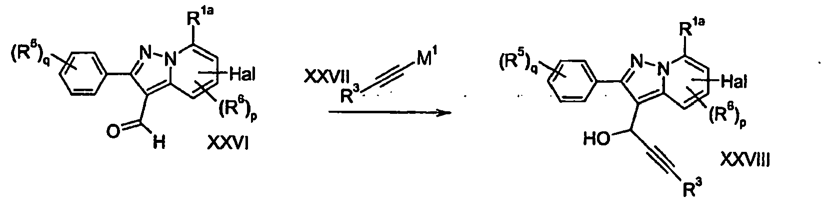 wherein all variables are as defined above.

Suitable metals (M') in the compounds of formula (XXVII) include but are not limited to, lithium, magnesium(II) halides, cerium(III) halides, and the like. A compound of formula (XXVII) may be purchased from commercial sources or prepared by methods known to one skilled in the art

A compound of formula (XXVI) may be conveniently prepared from compounds of formula (VII) by a formylation procedure. 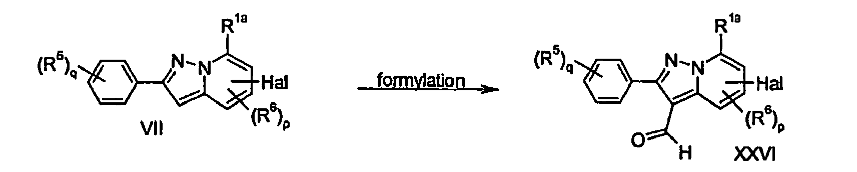 wherein all variables are as defined above.

Typically the formylation is carried out via the Vilsmeier-Haack reaction. The Vilsmeier-Haack reagents can be purchased from commercial sources or prepared *in situ.* Conditions include, but are not limited to treating compounds of formula (VII) with a premixed solution of phosphorous oxychloride in *N,N*-dimethylformamide optionally with heating the reaction to 50-150°C.

The compounds of formula (VII) are prepared by the process described above in connection with Scheme 1.

In a further embodiment of the present invention, certain compounds of formula (I) and (XII) wherein Y is N; R² is selected from the group consisting of Het, -OR⁷, -S(O)ₙR⁹, -NR⁷R⁸, -NHHet, and -NHR¹⁰Het; R³ is selected from the group consisting of H, alkyl, cycloalkyl, alkenyl, Ay, Het, -C(O)R⁷, -C(O)Ay, -CO₂R⁷, -CO₂Ay, -SO₂NHR⁹, -NR⁷R⁸ (where R⁷ and R⁸ are not H), -NR⁷Ay (where R⁷ is not H), -R¹⁰OR⁷, -R¹⁰OAy, -R¹⁰NR⁷R⁸ and -R¹⁰NR⁷Ay; R⁴ is H; and r is 1, may be conveniently prepared by a general process outlined in Scheme 2-A below. 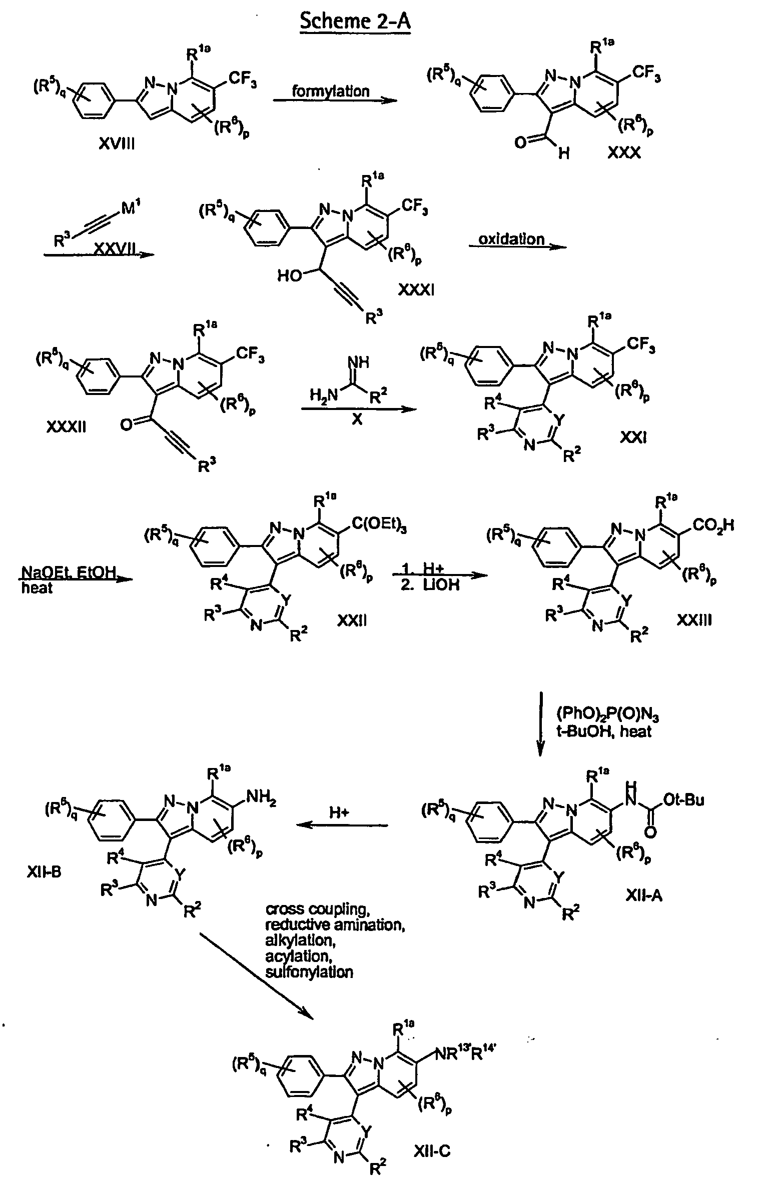 wherein:
- R^{1a}: is selected from the group consisting of H, alkyl, cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyl, -R¹⁰OR⁹, -R¹⁰NR⁷R⁸, cyano, azido and nitro;
- Y: is N ;
- R²: is selected from the group consisting of Het, -OR⁷, -S(O)nR⁹, -NR⁷R⁸, -NHHet, and -NHR¹⁰Het ;
- R³: is selected from the group consisting of H, alkyl, cycloalkyl, alkenyl, Ay, Het, -C(O)R⁷, -C(O)Ay, -CO₂R⁷. -CO₂Ay, -SO₂NHR⁹, -NR⁷R⁸ (where R⁷ and R⁸ are not H). -NR⁷Ay (where R⁷ is not H), -R¹⁰OR⁷, -R¹⁰OAy, -R¹⁰NR⁷R⁸ and -R¹⁰NR⁷Ay;
- R⁴: is H;
- Ph: is phenyl;
- each R^{13'} and R^{14'}: are the same or different and are each independently selected from the group consisting of alkyl, alkenyl, cycloalkyl, cycloalkenyl, Ay, Het, -C(O)R⁹, -CO₂R⁹, -C(O)NR⁹R¹¹, -C(S)NR⁹R¹¹, -C(NH)NR⁹R¹¹, -SO₂R¹⁰, -SO₂NR⁹R¹¹, -R¹⁰cycloalkyl, -R¹⁰OR⁹, -R¹⁰C(O)R⁹, -R¹⁰CO₂R⁹, -R¹⁰C(O)NR⁹R¹¹, -R¹⁰C(S)NR⁹R¹¹, -R¹⁰C(NH)NR⁹R¹¹, -R¹⁰SO₂R¹⁰, -R¹⁰SO₂NR⁹R¹¹, -R¹⁰SO₂NHCOR⁹, -R¹⁰NR⁹R¹¹, -R¹⁰NHC(NH)NR⁹R¹¹, -R¹⁰NHSO₂R⁹ and -R¹⁰NHCOR⁹;
- M¹: is Li, Mg-halide or cerium-halide, wherein halide is halo; and
all other variables are as defined above.

Generally, the process for preparing compounds of formula (I) and (XII) wherein Y is N; R² is selected from the group consisting of Het, -OR⁷, S(O)ₙR⁹ -NR⁷R⁸ -NHHet, and
- NHR¹⁰Het; R³ is selected from the group consisting of H, alkyl, cycloalkyl, alkenyl, Ay, Het, -C(O)R⁷, -C(O)Ay, -CO₂R⁷, -CO₂Ay, -SO₂NHR⁹, -NR⁷R⁸ (where R⁷ and R⁸ are not H), -NR⁷Ay (where R⁷ is not H), -R¹⁰OR⁷, -R¹⁰OAy, -R¹⁰NR⁷R⁸ and -R¹⁰NR⁷Ay; R⁴ is H; and r is 1, (all other variables having been defined above), comprises the following steps:
   (a) formylating the compound of formula (XVIII) to prepare a compound of formula (XXX) ;
   (b) reacting the compound of formula (XXX) with a compound of formula (XXVII) to prepare a compound of formula (XXXI);
   (c) oxidizing the compound of formula (XXXI) to prepare a compound of formula (XXXII);
   (d) reacting the compound of formula (XXXII) with a compound of formula (X) to prepare a compound of formula (XXI);
   (e) reacting the compound of formula (XXI) with sodium ethoxide to prepare a compound of formula (XXII);
   (f) reacting the compound of formula (XXII) with an acid, followed by hydrolysis of the resulting ester to prepare a compound of formula (XXIII);
   (g) reacting the compound of formula (XXIII) with diphenylphosphoryl azide in tert-butanol to prepare a compound of formula (XII-A);
   (h) optionally cleaving the compound of formula (XII-A) to prepare a compound of formula (XII-B);
   (i) optionally converting the compound of formula (XII-B) to a compound of formula (XII-C) using conditions selected from the group consisting of cross coupling, reductive amination, alkylation, acylation and sulfonylation; and
   (j) in the embodiment, wherein R^{1a} is H, converting the compound of formula (XII-A), (XII-B) or (XII-C) to a compound of formula (I).

In particular, certain compounds of formula (I) and (XII) wherein Y is N; R² is selected from the group consisting of Het, -OR⁷, -S(O)ₙR⁹, -NR⁷R⁸, -NHHet, and -NHR¹⁰Het; R³ is selected from the group consisting of H, alkyl, alkenyl, cycloalkyl, Ay, Het, -C(O)R⁷, -C(O)Ay, -CO₂R⁷, -CO₂Ay, -SO₂NHR⁹, -NR⁷R⁸ (where R⁷ and R⁸ are not H), -NR⁷Ay (where R⁷ is not H), -R¹⁰OR⁷, -R¹⁰OAy, -R¹⁰NR⁷R⁸ and -R¹⁰NR⁷Ay; R⁴ is H; and r is 1, can be prepared by converting the compound of formula (XXI) to a compound of formula (XII-A), (XII-B), or (XII-C) using the methods described above in connection with the process of Scheme 1-A.

Compounds of formula (XXI) can be prepared using a combination of process steps which are the same as or analogous to those described in Schemes 1-A and 2. Preparation of compounds of formula (XVIII) involves the condensation of a 2-chloro-5-trifluoromethylpyridine (XIV) with an acetophenone of formula (XIII) under basic conditions (according to the procedure of Scheme 1-A). Following the synthesis of the compound of formula (XVIII), the preparation proceeds according to the general steps described above for the preparation of compounds of formula (XXI) in Scheme 2.

Further compounds of formula (I) and (XII) wherein Y is N; R² is selected from the group consisting of Het, -OR⁷, -S(O)ₙR⁹, -NR⁷R⁸, -NHHet, and -NHR¹⁰Het; and r is 1, may be conveniently prepared the process outlined in Scheme 3 below. 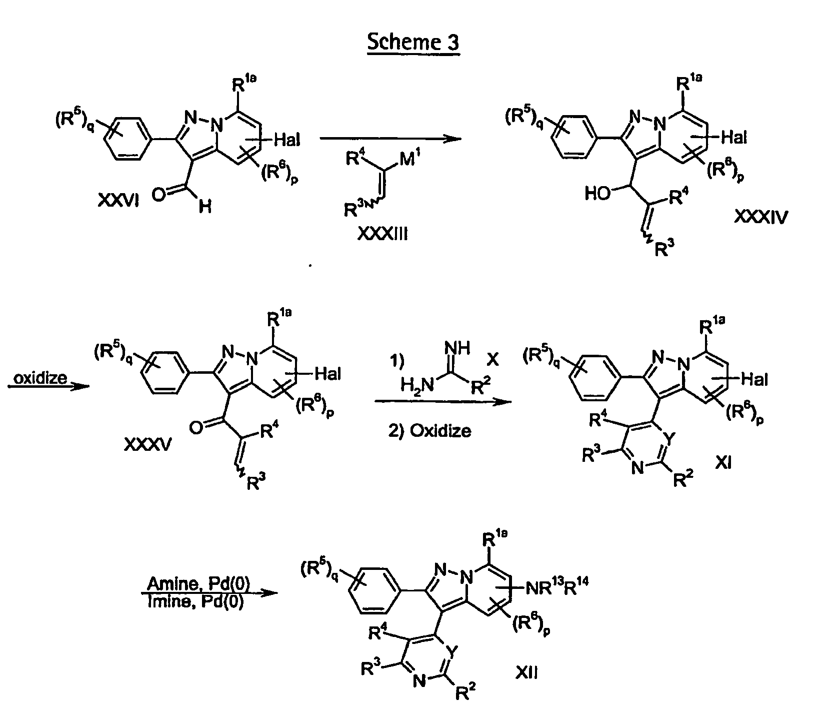 wherein:
Hal is halo;
R^{1a} is selected from the group consisting of H, alkyl, cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyl, -R¹⁰OR⁹, -R¹⁰NR⁷R⁸, cyano, azido and nitro;
YisN;
R² is selected from the group consisting of Het, -OR⁷, -S(O)ₙR⁹, -NR⁷R⁸, -NHHet, and -NHR¹⁰Het;
M¹ is Li, Mg-halide or cerium-halide, wherein halide is halo; and
all other variables are as defined above.

Generally, the process for preparing compounds of formula (I) and (XII) wherein Y is N; R² is selected from the group consisting of Het, -OR⁷, -S(O)ₙR⁹, -NR⁷R⁸, -NHHet, and -NHR¹⁰Het; and r is 1, (all formulas and all other variables having been defined above), comprises the following steps:
(a) reacting a compound of formula (XXVI) with a compound of formula (XXXIII) to prepare a compound of formula (XXXM;
(b) oxidizing the compound of formula (XXX)V) to prepare a compound of formula (XXXV);
(c) reacting the compound of formula (XXXV) with a compound of formula (X) followed by oxidative aromatization to prepare a compound of formula (XI);
(d) replacing the halogen of the compound of formula (XI) with an amine or imine to prepare a compound of formula (XII); and
(e) in the embodiment wherein R^{1a} is H, converting the compound of formula (XII) to a compound of formula (I).

More specifically, compounds of formula (I) and (XII) wherein Y is N; R² is selected from the group consisting of Het, -OR⁷, -S(O)ₙR⁹, -NR⁷R⁸, -NHHet, and -NHR¹⁰Het, and r is 1, can be prepared by replacing the halogen on the compound of formula (XI) with an amine nucleophile. Methods for the conversion of a compound of formula (XI) to a compound of formula (XII) are described above in connection with the synthesis according to Scheme 1.

A compound of formula (XI) can be prepared by reacting a compound of formula (XXXV) with a compound of formula (X) followed by oxidative aromatization. 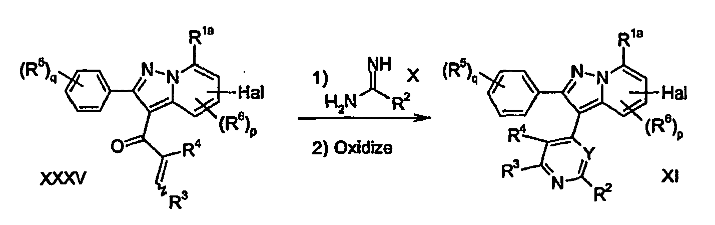 wherein all variables are as defined above.

The condensation is conveniently carried out by treating the compound of formula (XXXV) with a compound of formula (X) in an inert solvent, optionally in the presence of a base. The reaction may be heated to 50-150°C or performed at ambient temperature. Suitable inert solvents include lower alcohols such as, for example, methanol, ethanol, isopropanol and the like. The base is typically sodium alkoxide, potassium carbonate, or an amine base such as triethylamine. In another embodiment, the solvent is *N,N*-dimethylformamide and the base is potassium carbonate, or an amine base such as triethylamine. The reaction produces a dihydropyrimidine intermediate.

Conveniently, in the same reaction vessel, the dihydropyrimidine intermediate may be oxidized to a compound of formula (XI) by the addition of an oxidizing agent The reaction may be heated to 50-150°C or performed at ambient temperature. In one embodiment, the oxidizing agent is oxygen (O₂), palladium on carbon, 2,3-dichloro-5,6-dicyano-1,4-benzoquinone, or the like. A compound of formula (XXXV) may be conveniently prepared by oxidation of a compound of formula (XXXIV). 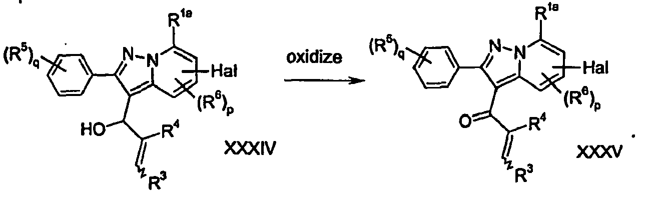 wherein all variables are as defined above.

Suitable oxidizing agents for the oxidation of compounds of formula (XXXIV) include but are not limited to manganese dioxide, and the like. The oxidation is typically carried out in an inert solvent such as for example, dichloromethane, chloroform, *N,N-*dimethylformamide, ether, and the like.

Compounds of formula (XXXIV) may be conveniently prepared by reacting a compound of formula (XXVI) with a compound of formula (XXXIII). 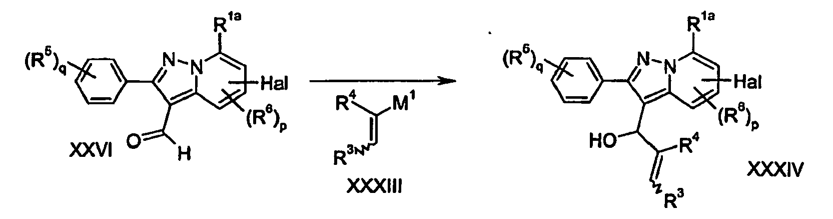 wherein M¹ is a metal such as for example, lithium, magnesium(II) halides, cerium(III) halides, and the like and all other variables are as defined above. Compounds of formula (XXXIII) may be purchased from commercial sources or prepared by methods known to one skilled in the art The compounds of formula (XXVI) may be prepared using the methods described above in connection with Scheme 2.

In another embodiment, compounds of formula (I) and (XII) wherein Y is N; R² is selected from the group consisting of Het, -OR⁷, -S(O)ₙR⁹, -NR⁷R⁸, -NHHet, and -NHR¹⁰Het, and r is 1, may be conveniently prepared by the process outlined in Scheme 3-A below. 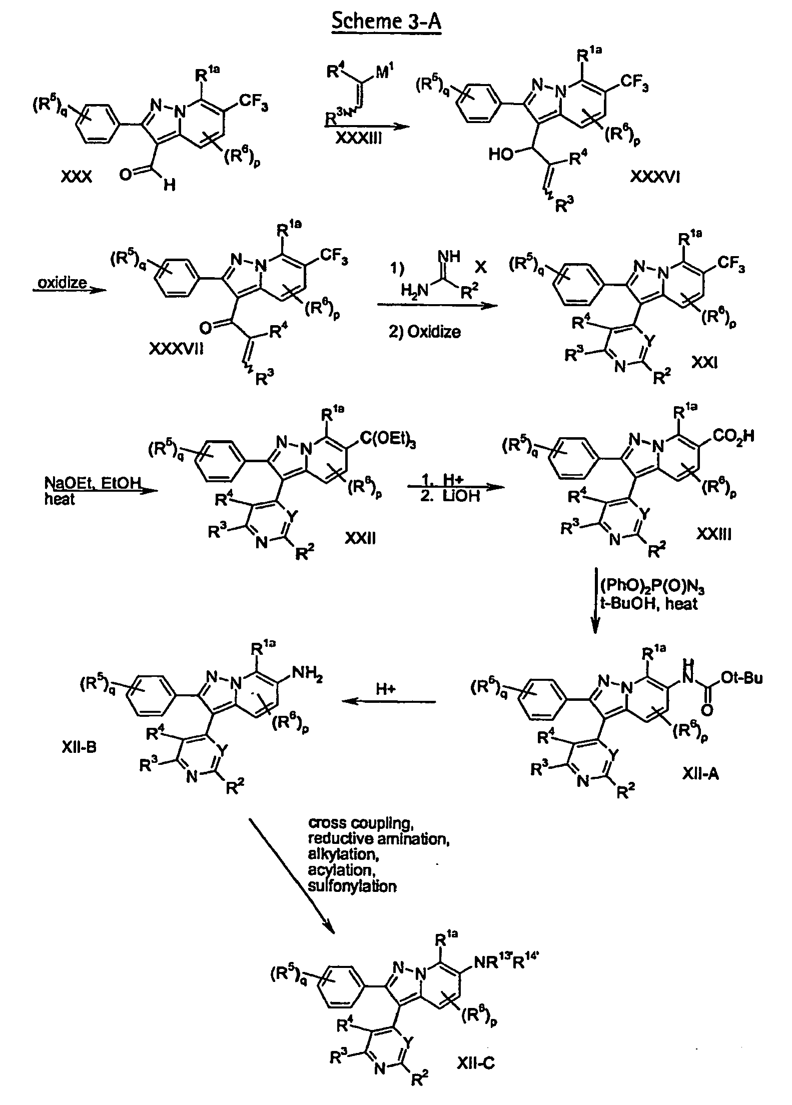 wherein:
- R^{1a}: is selected from the group consisting of H, alkyl, cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyl, -R¹⁰OR⁹, -R¹⁰NR⁷R⁸, cyano, azido and nitro;
- Y: is N;
- R²: is selected from the group consisting of Het, -OR⁷, -S(O)ₙR⁹-, -NR⁷R⁸, -NHHet, and -NHR¹⁰Het;
- each R^{13'} and R^{14'}: are the same or different and are each independently selected from the group consisting of alkyl, alkenyl, cycloalkyl, cycloalkenyl, Ay, Het, -C(O)R⁹, -CO₂R⁹, -C(O)NR⁹R¹¹, -C(NH)NR⁹R¹¹, -SO₂R¹⁰, -SO₂NR⁹R¹¹, -R¹⁰cycloalkyl, -R¹⁰OR⁹, -R¹⁰C(O)R⁹, -R¹⁰CO₂R⁹, -R¹⁰C(O)NR⁹R¹¹, -C(S)NR⁹R¹¹, -R¹⁰C(S)NR⁹R¹¹, -R¹⁰C(NH)NR⁹R¹¹, -R¹⁰SO₂R¹⁰, -R¹⁰SO₂NR⁹R¹¹, -R¹⁰SO₂NHCOR⁹, -R¹⁰NR⁹R¹¹, -R¹⁰NHC(NH)NR⁹R¹¹, -R¹⁰NHSO₂R⁹ and -R¹⁰NHCOR⁹;
- Ph: is phenyl;
- M¹: is Li, Mg-halide or cerium-halide, wherein halide is halo; and
all other variables are as defined above.

Generally, the process for preparing compounds of formula (I) and (XII) wherein Y is N; R² is selected from the group consisting of Het, -OR⁷, -S(O)ₙR⁹, -NR⁷R⁸, -NHHet, and -NHR¹⁰Het; and r is 1, (all formulas and all other variables having been defined above), comprises the following steps:
(a) reacting a compound of formula (XXX) with a compound of formula (XXXIII) to prepare a compound of formula (XXXVI);
(b) oxidizing the compound of formula (XXXVI) to prepare a compound of formula (XXXVII);
(c) reacting a compound of formula (XXXVII) with a compound of formula (X) followed by oxidative aromatization to prepare a compound of formula (XXI);
(d) reacting the compound of formula (XXI) with sodium ethoxide to prepare a compound of formula (XXII);
(e) reacting the compound of formula (XXII) with an acid, followed by hydrolysis of the resulting ester to give a compound of formula (XXIII);
(f) reacting the compound of formula (XXIII) with diphenylphosphoryl azide in tert-butanol to give a compound of formula (XII-A);
(g) optionally cleaving the compound of formula (XII-A) to give a compound of formula (XII-B);
(h) optionally converting the compound of formula (XII-B) to a compound of formula (XII-C) using conditions selected from the group consisting of cross coupling, reductive amination, alkylation, acylation and sulfonylation; and
(i) in the embodiment wherein R^{1a} is H, converting the compound of formula (XII-A), (XII-B) or (XII-C) to a compound of formula (I).

The synthesis is carried out using compounds and reactions which are the same as or analogous to those described above in connection with Schemes 1, 1-A, 2, 2-A, and 3.

Compounds of formula (I) and (XII) wherein Y is CH or N and r is 1, may be conveniently prepared by a general process outlined in Scheme 4 below. 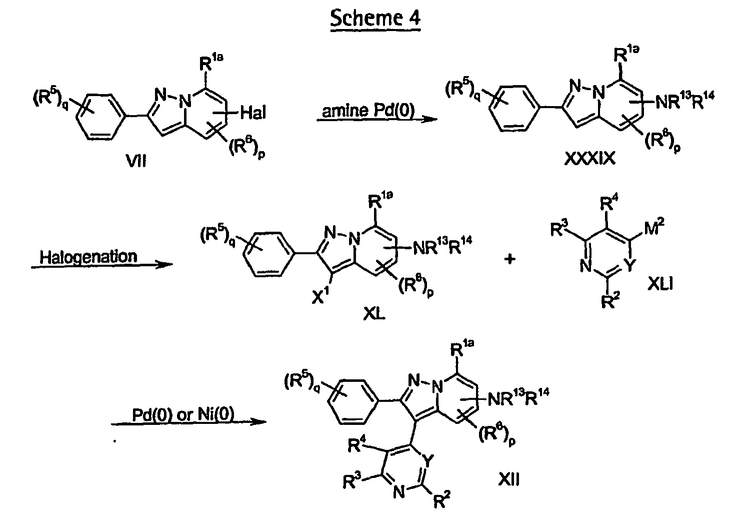 wherein:
- Hal: is halo;
- X¹: is halo, more particularly bromo or iodo;
- R^{1a}: is selected from the group consisting of H, alkyl, cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyl, -R¹⁰OR⁹, -R¹⁰NR⁷R⁸, cyano, azido and nitro;
- M²: is -B(OH)₂, -B(ORa)₂, -B(Ra)₂, -Sn(Ra)₃, Zn-halide, ZnRa, Mg-halide where Ra is alkyl or cycloalkyl and halide is halo; and
all other variables are as defined above.

Generally, the process for preparing a compound of formula (I) or (XII) wherein r is 1, (all formulas and variables having been defined above), comprises the following steps:
(a) replacing the halogen of the compound of formula (VII) with an amine to prepare a compound of formula (XXXIX);
(b) halogenating the compound of formula (XXXIX) to prepare a compound of formula (XL);
(c) reacting the compound of formula (XL) with a compound of formula (XLI) to prepare a compound of formula (XII); and
(d) in the embodiment wherein R^{1a} is H, converting the compound of formula (XII) to a compound of formula (I).

As will be apparent to one skilled in the art, the order of the foregoing steps is not critical to the process for preparing compounds of formula (XII) and as such these steps may be carried out in any desired order.

More specifically, compounds of formula (I) and (XII) wherein r is 1, can be prepared by reacting a compound of formula (XL) with a compound of formula (XLI). 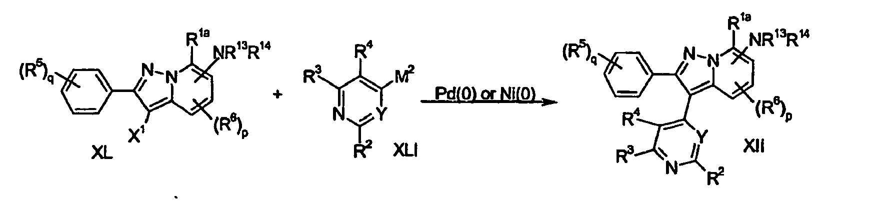 wherein all variables are as defined above.

The reaction may be carried out in an inert solvent, in the presence of a palladium (0) or nickel (0) catalyst. The reaction may optionally be heated to about 50-150°C. Typically, the reaction is performed by reacting equimolar amounts of a compound of formula (XL) with a Het-metal compound of formula (XLI), but the reaction may also be performed in the presence of an excess of compound of the formula (XLI). The palladium or nickel catalyst is typically present in 1-10 mol% compared to the compound of formula (XL). Examples of suitable palladium catalysts include but are not limited to, tetrakis(triphenylphosphine)palladium (0), dichlorobis(triphenylphosphine)palladium(II), tris(dibenzylidene acetone) dipalladium (0) and bis(diphenylphosphinoferrocene)palladium (II) dichloride. Suitable solvents include but are not limited to, *N,N*-dimethylformamide, toluene, tetrahydrofuran, dioxane, and 1-methyl-2-pyrrolidinone. When the Het-metal compound of formula (XLI) is an arylboronic acid or ester or an arylborinate the reaction is more conveniently carried out by adding a base in a proportion equivalent to, or greater than, that of the compound of formula (XL). Het-metal compounds of formula (XLI) may be obtained from commercial sources or prepared either as discreet isolated compounds or generated *in situ* using methods known to one skilled in the art (Suzuki, A. *J. Organomet. Chem.* **1999,** *576*, 147; Stille, J. *Angew. Chem. Int. Ed. Engl.* **1986**,25, 508; Snieckus, V. *J. Org. Chem.* **1995**, *60,* 292.)

Compounds of formula (XL) can be prepared from compounds of formula (XXXIX) by a halogenation procedure. 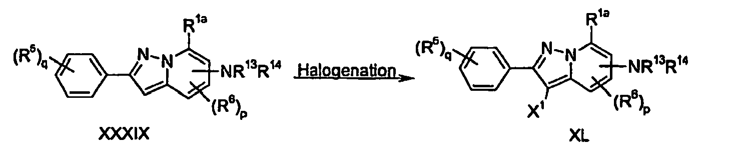 wherein all variables are as defined above.

Typically, the halogenation reaction is carried out by subjecting the compounds of formula (XXXIX) to a halogenating agent in a suitable solvent Suitable halogenating agents include but are not limited to, *N*-bromosuccinimide, trialkylammonium tribromides, bromine, *N*-chlorosuccinimide, *N*-iodosuccinimide, iodine monochloride, and the like. Suitable solvents include, for example, *N,N*-dimethylformamide, tetrahydrofuran, dioxane, 1-methyl-2-pyrrolidinone, carbon tetrachloride, toluene, dichloromethane, diethyl ether, and the like.

The compounds of formula (XXXIX) may be prepared from compounds of formula (VII) using amination methods (such as Buchwald amination) described above in connection with other Schemes.

In yet another embodiment of the present invention, certain compounds of formula (I) and (XII) wherein Y is N or CH and r is 1, may be conveniently prepared by the process outlined in Scheme 4-A below. 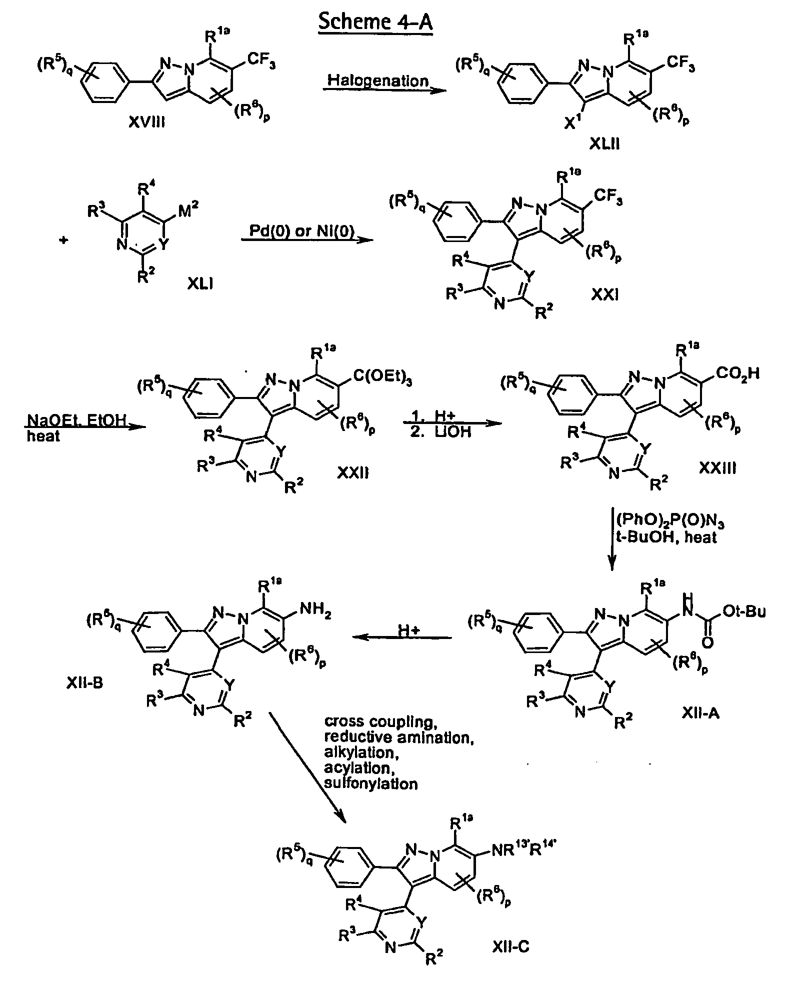 wherein:
- X¹: is halo, more particularly bromo or iodo;
- R^{1a}: is selected from the group consisting of H, alkyl, cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyl, -R¹⁰OR⁹, -R¹⁰NR⁷R⁸, cyano, azido and nitro;
- Ph: is phenyl;
- each R^{13'} and R^{14'}: are the same or different and are each independently selected from the group consisting of alkyl, alkenyl, cycloalkyl, cycloalkenyl, Ay, Het, -C(O)R⁹, -CO₂R⁹, -C(O)NR⁹R¹¹, -C(S)NR⁹R¹¹, -C(NH)NR⁹R¹¹, -SO₂R¹⁰, -SO₂NR⁹R¹¹, -R¹⁰cycloalkyl, -R¹⁰OR⁹, -R¹⁰C(O)R⁹, -R¹⁰CO₂R⁹, -R¹⁰C(O)NR⁹R¹¹, -R¹⁰C(S)NR⁹R¹¹, -R¹⁰C(NH)NR⁹R¹¹, -R¹⁰SO₂R¹⁰, -R¹⁰SO₂NR⁹R¹¹, -R¹⁰SO₂NHCOR⁹, -R¹⁰NR⁹R¹¹, -R¹⁰NHC(NH)NR⁹R¹¹, -R¹⁰NHSO₂R⁹ and -R¹⁰NHCOR⁹;
- M²: is -B(OH)₂, -B(ORa)₂, -B(Ra)₂, -Sₙ(Ra)₃, Zn-halide, ZnRa, Mg-halide where Ra is alkyl or cycloalkyl and halide is halo; and
all other variables are as defined above.

Generally, the process for preparing these compounds of formula (I) and (XII) wherein r is 1, (all formulas and variables having been defined above), comprises the following steps:
(a) halogenating a compound of formula (XVIII) to prepare a compound of formula (XLII);
(b) reacting the compound of formula (XLII) with a compound of formula (XLI) to prepare a compound of formula (XXI);
(c) reacting the compound of formula (XXI) with sodium ethoxide to prepare a compound of formula (XXII);
(d) reacting the compound of formula (XXII) with an acid, followed by hydrolysis of the resulting ester to prepare a compound of formula (XXIII);
(e) reacting the compound of formula (XXIII) with diphenylphosphoryl azide in tert-butanol to prepare a compound of formula (XII-A);
(f) optionally cleaving the compound of formula (XII-A) to prepare a compound of formula (XII-B);
(g) optionally converting the compound of formula (XII-B) to a compound of formula (XII-C) using conditions selected from the group consisting of cross coupling, reductive amination, alkylation, acylation and sulfonylation; and
(h) in the embodiment wherein R^{1a} is H, converting the compound of formula (XII-A), (XII-B) or (XII-C) to a compound of formula (I).

The synthesis is carried out using compounds and reactions which are the same as or analogous to those described above in connection with Schemes 1, 1-A, 2, 2-A, and 4.

A compound of formula (XII) (including compounds of formula (XII-A), (XII-B), and (XII-C) wherein r is 1, may be be conveniently converted to compounds of formula (XII-D): 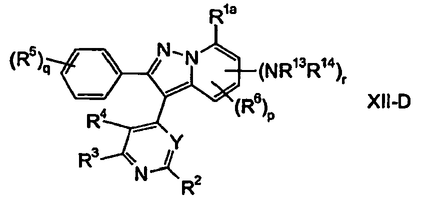 wherein r is 2 or 3 and all other variables are as defined above, using conventional procedures known to those skilled in the art For example,

A compound of formula (XII-D) wherein r is 2 or 3, can be derived from other compounds of formula (XII) using the halogenation procedures described herein, followed by a palladium (0) mediated amine coupling. Additionally, the carboxylic acid rearrangements such as the Curtius rearrangement described above can also be employed in suitably functionalized compounds of formula (XII) which can be determined readily by those skilled in the art

Similarly, as will be apparent to one skilled in the art, analogous procedures may be employed for the conversion of a compound of formula (I) wherein r is 1 to a compound of formula (I) wherein r is 2 or 3.

For the sake of brevity, the compounds of formula (XII), (XII-A), (XII-B), (XII-C) and (XII-D) (and pharmaceutically acceptable salts and solvates thereof will be collectively referred to herein as compounds of formula (XII-E) having the formula: 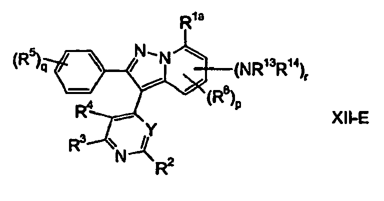 wherein r is 1, 2 or 3 and all other variables are as defined above.

The foregoing synthesis of Schemes 1, 1A, 2, 2A, 3, 3A, 4 and 4A provide the compounds of formula (I) directly, when the compound of formula (XII-E) is defined where R^{1a} is not H (i.e., where R^{1a} = R¹). In the embodiment where the compound of formula (XII-E) is defined where R^{1a} is H, the compound of formula (XII-E) may be converted to a compound of formula (I). For example, a compound of formula (XII-E) where R^{1a} is H, may be converted to a compound of formula (I) by a deprotonation/electrophile quench protocol. For example, reaction of a compound of formula (XII-E) with a base, such as n-butyllithium, followed by reacting with an electrophilic agent gives a compound of formula (I-A). 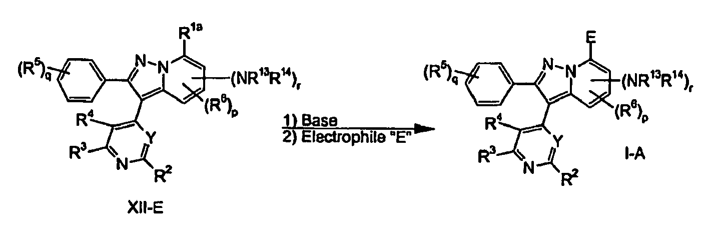 wherein E is R¹ and all other variables are as defined above in connection with any of the processes described above.

Electrophiles which may be used in this process include, but are not limited to: alkyl halides (E = methyl, benzyl etc.); aldehydes (E = CH(OH)R¹⁰); dimethylformamide (E = CHO); dialkyl disulfide (E = SMe, SEt, S-isopropyl etc); carbon dioxide (E = CO₂H); dimethylcarbamoyl chloride (E = C(O)NMe₂) and the like.

Typically a compound of formula (XII-E) in an inert solvent such as tetrahydrofuran at -78 °C is treated with a nonnucleophilic base. This reaction is subsequently quenched by addition of an electrophile. Suitable nonnucleophilic bases include, but are not limited to, n-butyllithium, lithium diisopropylamide, lithium tetramethylpiperidide and the like.

Further, a compound of formula (XII-E) wherein R^{1a} is H, may be converted to a compound of formula (I), by a deprotonation/electrophile quench/nucleophilic displacement protocol. For example, reaction of a compound of formula (XII-E) with a base, such as n-butyllithium, followed by quenching with an electrophilic halogenating agent gives a compound of formula (XLV). Treatment of compound of formula (XLV) with a nucloephile (Z) in a suitable solvent optionally with heating and optionally in the presence of a base gives a compound of formula (I-B). 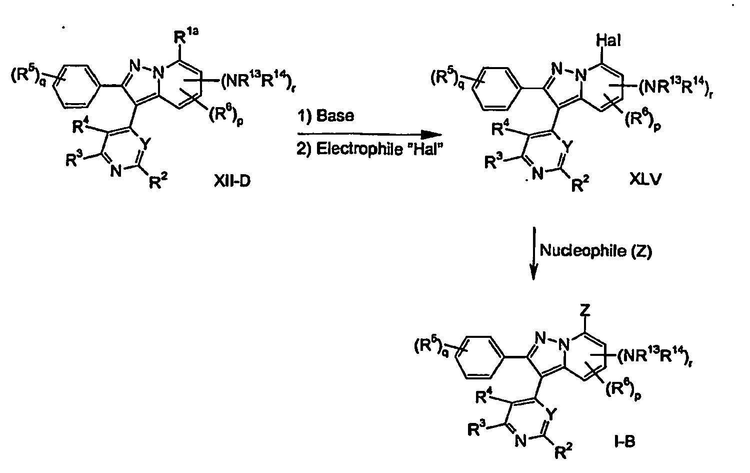 wherein Hal is halo, Z is selected from the group consisting of -OR⁷, -OAy, -OHet, -SR⁷, -SAy and -SHet and all other variables are as defined above. Electrophilic halogenating reagents include, but are not limited to: N-bromosuccinimide (Hal = bromine); N-chlorosuccinimide (Hal = chlorine); carbon tetrachloride (Hal = chlorine); N-iodosuccinimide (Hal = iodine) and iodine.

Suitable nucleophiles for use in the foregoing reaction include, but are not limited to HOR⁷, HOAy, HO-Het, HSR⁷, HSAy and HS-Het Solvents for use in this reaction include but are not limited to tetrahydrofuran, diethylether, and 1-methyl-2-pyrrolidinone. The base may be sodium hydride, sodium-tert-butoxide and the like.

Each of the foregoing processes may further comprise the step of converting a compound of formula (XII-E) or (I) to a pharmaceutically acceptable salt, solvate or physiologically functional derivative thereof, using techniques well known to those skilled in the art.

As will be apparent to those skilled in the art, a particular compound of formula (I), may be converted to another compound of formula (I) using conventional techniques. For example, one method of converting a compound of formula (I) to another compound of formula (I) comprises a) oxidizing the compound of formula (I-C, where R¹ is -SR¹⁵ and R¹⁵ is alkyl) to prepare a compound of formula (I-D) and then b) optionally reacting a compound of formula (I-D) with an oxygen nucleophile selected from the group consisting of HOR¹⁰, HOAy and HOHet, where R¹⁰, Ay and Het are as defined above, to prepare a compound of formula (I-E). 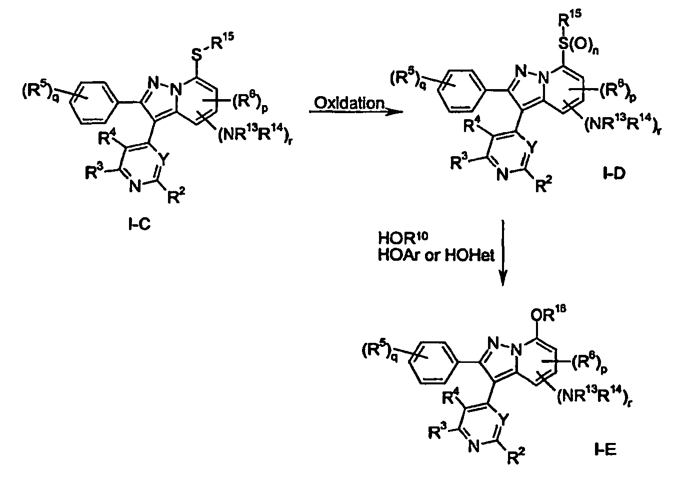 wherein R¹⁵ is alkyl, R¹⁶ is selected from the group consisting of R¹⁰, Ay and Het, and all other variables are as defined above.

Typically the oxidizing agent used in the preparation of compounds of formula (I-D) is a peracid, such as m-chloroperoxybenzoic acid or the like optionally with a base such as sodium bicarbonate. Suitable solvents for the oxidation include, but are not limited to, dichloromethane, chloroform and the like.

Treatment of the sulfoxide with an alkoxide (e.g. sodium ethoxide) gives a compound of formula (I-E). Typical solvents for the nucleophilic displacement include, but are not limited to, alcohols.

In another example, certain compounds of formula (I) may be converted to other compounds of formula (I) by a) oxidizing the compound of formula (I-F) to prepare a compound of formula (I-G) and then b) optionally reacting a compound of formula (I-G) with an oxygen or amine nucleophile of formula R², wherein R² is selected from the group consisting of -NR⁷R⁸, -OR⁷, Het attached through N, -NHHet, and NHR¹⁰Het, to prepare a compound of formula (I) wherein R² is selected from the group consisting of -NR⁷R⁸, -OR⁷, Het attached through N, -NHHet, and -NHR¹⁰Het, and all other variables are as defined above

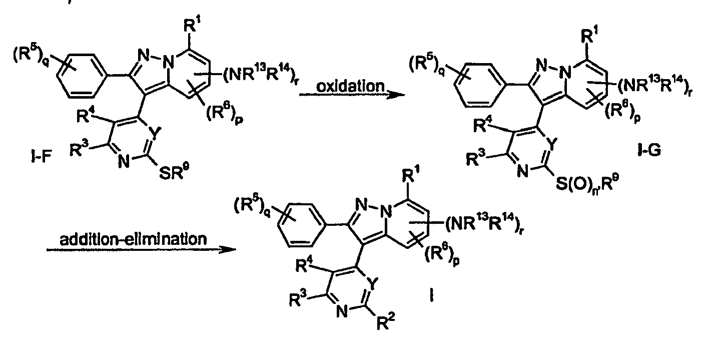

More specifically, a compound of formula (I-G) may be conveniently prepared by reacting a compound of formula (I-F) (i.e., a compound of formula (I) wherein R² is -S(O)nR⁹ where n is 0), with an oxidizing agent in an inert solvent, optionally in the presence of a base. Typically the oxidizing agent is a peracid such as m-chloroperbenzoic acid or the like optionally with a base such as sodium bicarbonate. Careful monitoring of the stoichiometry between the oxidizing agent and the substrate allows the product distribution between sulfoxide (n=1), and sulfone (n=2) to be controlled. Suitable solvents include but are not limited to, dichloromethane, chloroform and the like. Compounds of formula (I-F) are prepared by methods described above wherein R²=SR⁹.

A compound of formula (I-G) may be converted to a compound of formula (I) wherein R² is selected from the group consisting of -NR⁷R⁸, -OR⁷. Het attached through N, -NHHet, and NHR¹⁰Het, by reacting a compound of formula (I-G) with an oxygen or amine nucleophile of formula R², wherein R² is selected from the group consisting of -NR⁷R⁸, -OR⁷, Het linked through N, -NHHet and -NHR¹⁰Het. The reaction may be carried out neat or in a suitable solvent and may be heated to 50-150°C. Typically the solvent is a lower alcohol such as methanol, ethanol, isopropanol and the like or solvent such as *N,N*-dimethylformamide or tetrahydrofuran, and the like. Optionally a base may be used to facilitate the reaction. Typically the base can be potassium carbonate, or an amine base such as triethylamine.

As will be apparent to one skilled in the art, the foregoing conversion method is applicable to compounds of formula (XII-E) (e.g. compounds where R^{1a} is H) wherein R² is -SR⁹ to form other compounds of the formula (XII-E) wherein R² is -NR⁷R⁸, -OR⁷, Het linked through N, -NHHet and -NHR¹⁰Het. Such a compound of formula (XII-E) may be further converted to a compound of formula (I) using methods described above.

Another particularly useful method for converting a compound of formula (I) to another compound of formula (I) comprises reacting a compound of formula (I-H) (i.e., a compound of formula (I) wherein R² is fluoro) with an amine, and optionally heating the mixture to 50-150°C to prepare a compound of formula (I-I) (i.e., a compound of formula (I) wherein R² is -NR⁷R⁸). 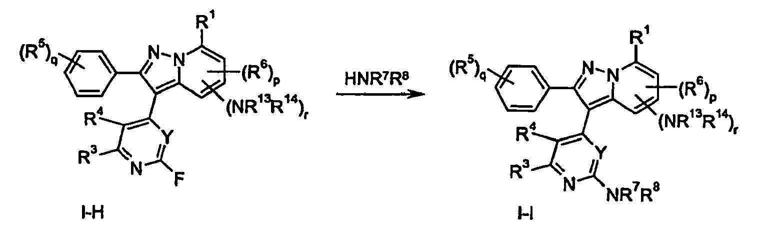 wherein all other variables are as defined above in connection with any of the processes described above.

This procedure may be carried out by mixing a compound of formula (I-H) in an amine neat, or in a suitable solvent with an excess of amine to produce a compound of formula (I-I). Typically the solvent is a lower alcohol such as methanol, ethanol, isopropanol and the like. Other suitable solvents may include *N,N-*dimethylformamide, 1-methyl-2-pyrrolidine and the like.

As will be apparent to one skilled in the art, this method for replacement of R² = fluoro with an amine in compounds of formula (I) to form other compounds of formula (I) is applicable to analagous sequences for compounds of formula (XII-E) (e.g. compounds where R^{1a} is H). The newly formed compounds of formula (XII-E) can be ultimately converted to compounds of formula (I) using methods described herein.

As a further example, a compound of formula (I-J) (i.e., a compound of formula (I) wherein q is 1 or more and at least one R⁵ is O-methyl) may be converted to a compound of formula (I-K) (i.e., a compound of formula (I) wherein q is 1 or more and at least one R⁵ is OH) using conventional demethylation techniques. Additionally, a compound of formula (I-K) may optionally be converted to a compound of formula (I-L) (i.e., a compound of formula (I) wherein q is 1 or more and at least one R⁵ is OR¹⁰). For example, the foregoing conversions are represented schematically as follows: 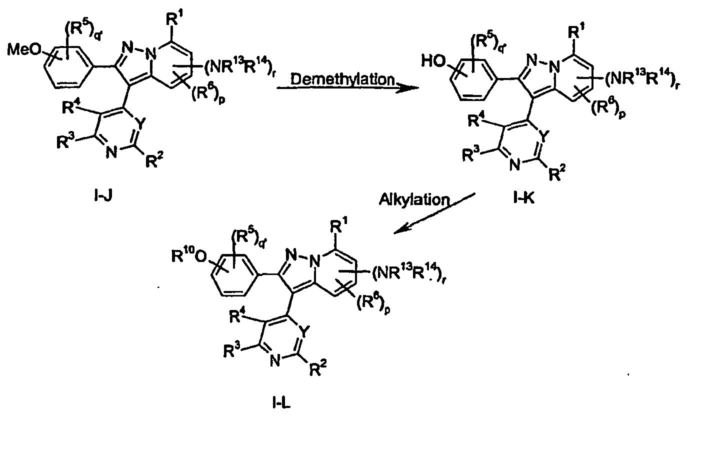 wherein q' is 1, 2, 3 or 4; Me is methyl and all other variables are as defined above.

The demethylation reaction may be carried out by treating a compound of formula (I-J) in a suitable solvent with a Lewis acid at a temperature of -78°C to room temperature, to produce a compound of formula (I-K). Typically the solvent is an inert solvent such as dichloromethane, chloroform, acetonitrile, toluene and the like. The Lewis acid may be boron tribromide, trimethylsilyl iodide or the like.

Optionally, the compound of formula (I-K) may be further converted to a compound of formula (I-L) by an alkylation reaction. The alkylation reaction may be carried out by treating a compound of formula (I-K) in suitable solvent with an alkyl halide of formula R¹⁰-halo where R¹⁰ is as defined above, to form another compound of formula (I-L). The reaction is typically carried out in the presence of a base and with optional heating to 50-200°C. The reaction may be carried out in a solvent such as N,N-dimethylformamide, dimethylsulfoxide and the like. Typically the base is potassium carbonate, cesium carbonate, sodium hydride or the like. Additionally, as will be apparent to one skilled in the art, the alkylation reaction can be carried out under Mitsunobu conditions.

Based upon this disclosure and the examples contained herein one skilled in the art can readily convert a compound of formula (I) or a pharmaceutically acceptable salt, solvate or physiologically functional derivative thereof into another compound of formula (I), or a pharmaceutically acceptable salt, solvate or physiologically functional derivative thereof.

The present invention also provides radiolabeled compounds of formula (I) and biotinylated compounds of formula (I). Radiolabeled compounds of formula (I) and biotinylated compounds of formula (I) can be prepared using conventional techniques. For example, radiolabeled compounds of formula (I) can be prepared by reacting the compound of formula (I) with tritium gas in the presence of an appropriate catalyst to produce radiolabeled compounds of formula (I). In one particular embodiment, the compound of formula (I) is tritiated.

The radiolabeled compounds of formula (I) and the biotinylated compounds of formula (I) are useful in assays for the identification of compounds for the treatment or prophylaxis of viral infections such as herpes viral infections. Accordingly, the present invention provides an assay method for identifying compounds which have activity for the treatment or prophylaxis of viral infections such as herpes viral infections, which method comprises the step of specifically binding the radiolabeled compound of formula (I) or the biotinylated compound of formula (I) to the target protein. More specifically, suitable assay methods will include competition binding assays. The radiolabeled compounds of formula (I) and the biotinylated compounds of formula (I) can be employed in assays according to the methods conventional in the art.

The following examples are illustrative embodiments of the invention, not limiting the scope of the invention in any way. Reagents are commercially available or are prepared according to procedures in the literature. Example numbers refer to those compounds listed in the tables above. ¹H and ¹³C NMR spectra were obtained on Varian Unity Plus NMR spectrophotometers at 300 or 400 MHz, and 75 or 100 MHz respectively. ¹⁹F NMR were recorded at 282 MHz. Mass spectra were obtained on Micromass Platform, or ZMD mass spectrometers from Micromass Ltd. Altrincham, UK, using either Atmospheric Chemical Ionization (APCI) or Electrospray Ionization (ESI). Analytical thin layer chromatography was used to verify the purity of some intermediates which could not be isolated or which were too unstable for full characterization, and to follow the progress of reactions. Unless otherwise stated, this was done using silica gel (Merck Silica Gel 60 F254). Unless otherwise stated, column chromatography for the purification of some compounds, used Merck Silica gel 60 (230-400 mesh), and the stated solvent system under pressure. All compounds were characterized as their free-base form unless otherwise stated. On occasion the corresponding hydrochloride salts were formed to generate solids where noted.

### Example 1: N-Cyclopentyl-3-[2-(cyclopentylamino)-4-pyrimidinyl]-2-(4-fluorophenyl)pyrazolo[1,5-a]pyridin-5-amine.

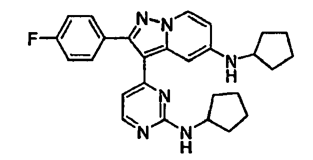

### a) 2-(4-Chloro-2-pyridinyl)-1-(4-fluorophenyl)ethanone.

To a cold (0°C) solution of 4-chloro-2-picoline (5.0 g, 39 mmol) and ethyl 4-fluorobenzoate (6.6 g, 39 mmol) in tetrahydrofuran (100 mL) was added lithium bis(trimethylsilyl)amide (80 mL, 1.0 M in tetrahydrofuran, 80 mmol) dropwise *via* a pressure equalizing funnel over 30 minutes. Upon complete addition, the cold bath was removed and the resulting solution was stirred at room temperature for 15 hours. The reaction mixture was concentrated under reduced pressure and methanol was added to the reaction, resulting in the formation of a white precipitate. The precipitate was collected by filtration and dried to give 2-(4-chloro-2-pyridinyl)-1-(4-fluorophenyl)ethanone (9.6 g, 99%) as a white solid. ¹H-NMR (DMSO-*d₆*): δ 7.90 (m, 3H), 7.11 (t, 2H), 6.56 (s, 1H), 5.67 (s, 1H), 4.14 (m, 2H); ¹⁹F-NMR (DMSO-*d₆*): δ -115.67; MS m/z 250 (M+1).

### b) 2-(4-Chloro-2-pyridinyl)-1-(4-fluorophenyl)ethanone oxime.

To a solution of 2-(4-chloro-2-pyridinyl)-1-(4-fluorophenyl)ethanone (9.6 g, 38 mmol) in methanol (200 mL) was added hydroxylamine hydrochloride (13.5 g, 190 mmol) followed by the addition of a sodium hydroxide solution (7.8 g, 190 mmol in 50 mL of water). The resulting suspension was heated at reflux for 2 hours and then allowed to cool to room temperature. The mixture was concentrated and water was added to the resulting slurry. A white precipitate formed, which was collected by filtration, washed with water and dried (magnesium sulfate) to give 2-(4-chloro-2-pyridinyl)-1-(4-fluorophenyl)ethanone oxime (8.45 g, 84%) as a white solid. ¹H-NMR (DMSO-*d*₆): δ 11.56 (s, 1H). 8.44 (d, 1H). 7.80 (m, 2H), 7.40 (m, 2H), 7.22 (m, 2H), 4.29 (s, 2H); ¹⁹F-NMR (DMSO-*d*₆): δ -113.44; MS *m*/*z* 265 (M+1).

### c) 5-Chloro-2-(4-fluorophenyl)pyrazolo[1,5-a]pyridine.

To a solution of 2-(4-chloro-2-pyridinyl)-1-(4-fluorophenyl)ethanone oxime (8.0 g, 30 mmol) in 1,2-dimethoxyethane (50 mL) at 0°C was added trifluoroacetic anhydride (6.3 g, 30 mmol), keeping the temperature below 10°C during the addition. After the addition was complete, the reaction was warmed to room temperature. The solution was then cooled to 4°C and a solution of triethylamine (8.4 mL, 60 mmol) in 1,2-dimethoxyethane (20 mL) was added over a period of 0.5 hours. The mixture was allowed to warm to room temperature and was stirred for 1.5 hours. To this mixture was added iron(II) chloride (40 mg) and the reaction was heated at 75°C for 15 hours. The reaction mixture was poured into water (300 mL). The resulting suspension was extracted with ethyl acetate. The combined organics were dried (magnesium sulfate), filtered and concentrated to a solid residue. This residue was purified by flash chromatography (1:1 ethyl acetate-hexane) to give 5-chloro-2-(4-fluorophenyl)pyrazolo[1,5-*a*]pyridine (4.2 g, 57 %) as a white solid. ¹H-NMR (CDCl₃): δ 8.36 (d, 1H), 7.93 (q, 2H), 7.49 (d, 1H), 7.15 (t, 2H), 6.70 (dd, 1H), 6.69 (s, 1H); ¹⁹F-NMR (CDCl₃): δ -113.30; MS *m*/*z* 247 (M+1).

### d) 5-Chloro-2-(4-fluorophenyl)pyrazolo[1,5-a]pyridine-3-carbaldehyde.

Phosphorous oxychloride (0.6 mL, 6.4 mmol) was added to N,N-dimethylformamide (10 mL) and the resulting mixture stirred at room temperature for 10 minutes. 5-Chloro-2-(4-fluorophenyl)pyrazolo[1,5-*a*]pyridine (1.0 g, 4.1 mmol) was added and the reaction mixture was stirred at room temperature for 12 hours. The reaction mixture was poured into ice-water and neutralized to pH 7 with aquous ammonium hydroxide. The resulting slurry was extracted with dichloromethane (3 x 40 mL). The combined organics were washed with brine, dried (magnesium sulfate), filtered and concentrated to give, after recrystallization from acetonitrile, 5-chloro-2-(4-fluorophenyl)pyrazolo [1,5-*a*]pyridine-3-carbaldehyde (0.95 g, 85 %) as a white solid. ¹H-NMR (CDCl₃): δ10.07 (s, 1H), 8.49 (d, 1H), 8.44 (d, 1H), 7.78 (q, 2H), 7.22 (t, 2H), 7.07 (dd, 1H); MS *m*/*z* 275 (M+1).

### e) 1-[5-Chloro-2-(4-fluorophenyl)pyrazolo[1,5-a]pyridin-3-yl]-2-butyn-1-one.

To a solution of 5-chloro-2-(4-fluorophenyl)pyrazolo[1,5-*a*]pyridine-3-carbaldehyde (0.93 g, 3.4 mmol) in tetrahydrofuran (20 mL) at -78°C was added ethynylmagnesium bromide (16 mL, 0.5 M in tetrahydrofuran, 8.0 mmol). The mixture was allowed to warm to room temperature and stirred for 1 hour. Water was added to the reaction and the resulting mixture was extracted with ethyl acetate. The ethyl acetate phase was dried (magnesium sulfate), filtered and concentrated to a solid residue. This residue was dissolved in dichloromethane (50 mL) and manganese dioxide (5 g) was added. This slurry was stirred at room temperature for 2 hours. The manganese dioxide was removed by filtration and the filtrate was concentrated to a solid. This solid was purified by flash chromatography (dichloromethane) to give 1-[5-chloro-2-(4-fluorophenyl)pyrazolo[1,5-*a*]pyridin-3-yl]-2-butyn-1-one (0.63 g, 62 % for two steps) as a white solid. ¹H-NMR (CDCl₃): δ 8.52 (d, 1H), 8.47 (d, 1H), 7.69 (q, 2H), 7.18 (t, 2H), 7.07 (dd, 1 H), 3.00 (s, 1H); ¹⁹F-NMR (CDCl₃): δ -111.69; MS m/z 299 (M+1).

### f) 4-[5-Chloro-2-(4-fluorophenyl)pyrazolo[1,5-a]pyridin-3-yl]-N-cyclopentyl-2-pyrimidinamine.

To a solution of 1-[5-chloro-2-(4-fluorophenyl)pyrazolo[1,5-a]pyridin-3-yl]-2-butyn-1-one (0.61 g, 2.0 mmol) in N,N-dimethylformamide was added cyclopentyl guanidine hydrochloride (0.67 g, 4.1 mmol) followed by anhydrous potassium carbonate (0.57 g, 4.1 mmol). The resulting mixture was heated at 80°C for 12 hours. Upon cooling to room temperature, water was added. The mixture was extracted with ethyl acetate. The ethyl acetate phase was washed with brine, dried (magnesium sulfate), filtered and concentrated *in vacuo.* The resulting residue was purified by flash chromatography (1:1 ethyl acetate-hexane) to give, after recrystallization from acetonitrile, 4-[5-chloro-2-(4-fluorophenyl)pyrazolo[1,5-*a*]pyridin-3-yl]-*N-*cyclopentyl-2-pyrimidinamine (0.6 g, 74 %) as a white solid. ¹H-NMR (CDCl₃): δ8.54 (broad s, 1H), 8.40 (d, 1H), 8.04 (d, 1H), 7.60 (q, 2H), 7.16 (t, 2H), 6.88 (dd, 1H), 6.28 (d, 1H), 5.22 (d, 1H), 4.40 (m, 1H), 1.4-2.2 (m, 8H); ¹⁹F-NMR (CDCl₃): δ -112.5; MS *m*/*z* 408 (M+1).

### g) N-Cyclopentyl-3-[2-(cyclopentylamino)-4-pyrimidinyl]-2-(4-fluorophenyl)pyrazolo[1,5-a]pyridin-5-amine.

To a solution of 4-[5-chloro-2-(4-fluorophenyl)pyrazolo[1,5-*a*]pyridin-3-yl]-*N-*cyclopentyl-2-pyrimidinamine (0.1 g, 0.25 mmol) in cyclopentylamine (5 mL) was added racemic-2,2'-bis(diphenylphosphino)-1,1'-binaphthyl (46 mg, 0.08 mmol), cesium carbonate (120 mg, 0.38 mmol) and palladium (II) acetate (11 mg, 0.05 mmol). The resulting mixture was stirred at 80°C for 24 hours, at which time the reaction was judged complete by thin layer chromatography. The solution was cooled to room temperature and ethyl acetate and water were added to the reaction mixture. The phases were separated, and the aquous phase again extracted with ethyl acetate. The combined organics were dried (magnesium sulfate), filtered and concentrated. The resulting residue was purified by flash chromatography (1:1 hexanes-ethyl acetate) to give *N*-cyclopentyl-3-[2-(cyclopentylamino)-4-pyrimidinyl]-2-(4-fluorophenyl)pyrazolo[1,5-*a*]pyridin-5-amine (78 mg, 70 %) as a white solid. ¹H-NMR (CDCl₃): δ 8.16 (d, 1H), 7.95 (d, 1H), 7.58 (q, 2H), 7.38 (d, 1H), 7.12 (t, 2H), 6.24 (dd, 1H), 6.20 (d, 1H), 5.05 (d, 1H), 4.40 (m, 1H), 4.13 (m, 1H), 3.89 (m, 1H), 1.5-2.2 (m, 16H); ¹⁹F-NMR (CDCl₃): δ -113.7; MS *m*/*z* 457 (M+1).

### Example 2: N-Cyclopentyl-3-[2-(cyclopentylamino)-4-pyrimidinyl]-7-(ethylsulfanyl)-2-(4-fluorophenyl)pyrazolo[1,5-a]pyridin-5-amine.

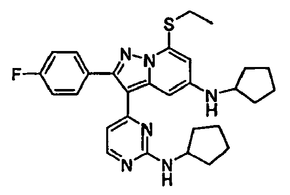

To a solution of *N*-cyclopentyl-3-[2-(cyclopentylamino)-4-pyrimidinyl]-2-(4-fluorophenyl)pyrazolo[1,5-*a*]pyridin-5-amine (0.11 g, 0.24 mmol) in anhydrous tetrahydrofuran (5 mL) at -78 °C was added n-butyllithium (0.75 mL of 1.6 M solution in hexanes, 1.2 mmol). The resulting solution was stirred at -78°C for 10 minutes, then ethyl disulfide (0.15 mL, 0.24 mmol) was added. The reaction mixture was allowed to warm to room temperature and then quenched by the addition of water. Ethyl acetate and water were added to the reaction mixture. The phases were separated, and the aquous phase again extracted with ethyl acetate. The combined organics were dried (magnesium sulfate), filtered and concentrated. The resulting residue was purified by flash chromatography (1:1 hexanes-ethyl acetate) to give *N*-cyclopentyl-3-[2-(cyclopentylamino)-4-pyrimidinyl]-7-(ethylsulfanyl)-2-(4-fluorophenyl)pyrazolo[1,5-*a*]pyridin-5-amine (50 mg, 40%) as a solid. ¹H-NMR (CDCl₃): δ 7.99 (d, 1H), 7.64 (q, 2H), 7.34 (d, 1H), 7.13 (t, 2H), 6.24 (d, 1H), 6.17 (d, 1H), 5.06 (d, 1H), 4.44 (m, 1H), 4.10 (m, 1H), 3.94 (m, 1H), 3.12 (q, 2H), 2.0-2.1 (m, 4H), 1.5-2.0 (m, 12H), 1.46 (t, 3H); ¹⁹F-NMR (CDCl₃): δ-113.9; MS *m*/*z* 518 (M+1).

### Example 3: N-Cyclopentyl-3-[2-(cyclopentylamino)-4-pyrimidinyl]-2-(4-fluorophenyl)-7-methylpyrazolo[1,5-a]pyridin-5-amine.

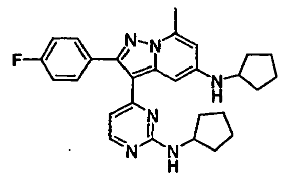

To a solution of *N*-cyclopentyl-3-[2-(cyclopentylamino)-4-pyrimidinyl]-2-(4-fluorophenyl)pyrazolo[1,5-α]pyridin-5-amine (0.08 g, 0.18 mmol) in anhydrous tetrahydrofuran (5 mL) at -78 °C was added n-butyllithium (0.55 mL of 1.6 M solution in hexanes, 0.9 mmol). The resulting solution was stirred at -78°C for 10 minutes, then iodomethane (0.06 mL, 0.96 mmol) was added. The reaction mixture was allowed to warm to room temperature and then quenched by the addition of water. Ethyl acetate and water were added to the reaction mixture. The phases were separated, and the aqueous phase again extracted with ethyl acetate. The combined organics were dried (magnesium sulfate), filtered and concentrated. The resulting residue was purified by flash chromatography (1:1 hexanes-ethyl acetate) to give *N*-cyclopentyl-3-[2-(cyclopentylamino)-4-pyrimidinyl]-2-(4-fluorophenyl)-7-methylpyrazolo[1,5-*a*]pyridin-5-amine (30 mg, 40%) as a solid. ¹H-NMR (CDCl₃): δ 7.98 (d, 1H), 7.62 (q, 2H), 7.38 (d, 1H), 7.15 (t, 2H), 6.22 (d, 1H), 6.16 (d, 1H), 5.04 (d, 1H), 4.44 (m, 1H), 4.04 (d, 1H), 3.94 (m, 1H), 2.71 (s, 3H), 2.0-2.1 (m, 4H), 1.4-1.9 (m. 12H); ¹⁹F-NMR (CDCl₃): δ -114.0; MS *m*/*z* 472 (M±1).

### Example 4: N-Cyclopentyl-3-[2-(cyclopentylamino)-4-pyrimidinyl]-7-ethoxy-2-(4-fluorophenyl)pyrazolo[1,5-a]pyridin-5-amine.

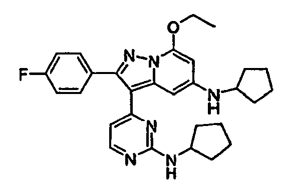

To a solution of *N*-cyclopentyl-3-[2-(cyclopentylamino)-4-pyrimidinyl]-7-(ethylsulfanyl)-2-(4-fluorophenyl)pyrazolo[1,5-*a*]pyridin-5-amine (0.05 g, 0.1 mmol) in dichloromethane (5 mL) was added m-chloroperoxybenzoic acid (57-86 %, 70 mg, 0.4 mmol) in dichloromethane. The resulting solution was stirred at room temperature for 30 minutes. The organic phase was extracted with aqueous potassium carbonate, dried (magnesium sulfate), filtered and concentrated to a solid. To this solid was added a solution of sodium ethoxide (5 mL, 3 M in ethanol) and the resulting solution heated at reflux for 2 hours. The resulting mixture was concentrated to dryness and purified by flash chromatography (1:1 hexanes-ethyl acetate) to give *N*-cyclopentyl-3-[2-(cyclopentylamino)-4-pyrimidinyl]-7-ethoxy-2-(4-fluorophenyl)pyrazolo[1,5-*a*]pyridin-5-amine (20 mg, 40%) as a solid. ¹H-NMR (CDCl₃): δ 7.96 (d, 1H), 7.60 (q, 2H), 7.20 (d, 1H), 7.13 (t, 2H), 6.20 (d, 1H), 5.64 (d, 1H), 5.04 (m, 1H), 4.40 (q, 2H), 4.08 (m, 1H), 3.95 (m, 1H), 2.0-2.1 (m, 4H), 1.5-1.9 (m, 12H), 1.30 (t, 3H); ¹⁹F-NMR (CDCl₃): δ -113.92; MS *m*/*z* 502 (M+1).

### Example 5: N-Cyclopentyl-3-[2-(cyclopentylamino)-4-pyrimidinyl]-2-(4-fluorophenyl)-7-(methylsulfanyl)pyrazolo[1,5-a]pyridin-5-amine.

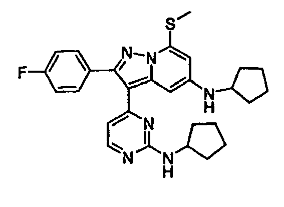

In a similar manner as described in Example 2 from *N*-Cyclopentyl-3-[2-(cyclopentylamino)-4-pyrimidinyl]-2-(4-fluorophenyl)pyrazolo[1,5-a]pyridin-5-amine (97 mg, 0.22 mmol) and methyl disulfide was obtained *N*-cyclopentyl-3-[2-(cyclopentylamino)-4-pyrimidinyl]-2-(4-fluorophenyl)-7-(methylsulfanyl)pyrazolo[1,5-*a*]pyridin-5-amine (100 mg, 90 %) as a solid. ¹H-NMR (CDCl₃): δ 7.98 (d, 1 H), 7.65 (q, 2H), 7.32 (d, 1H), 7.15 (t, 2H), 6.23 (d, 1H), 6.05 (d, 1H), 5.00 (d, 1H), 4.44 (m, 1 H), 4.15 (m, 1H), 3.95 (m, 1H), 2.59 (s, 3H), 2.0-2.1 (m, 4H), 1.5-1.9 (m, 12H); ¹⁹F-NMR (CDCl₃):δ -113.8; MS *m*/*z* 504 (M+1).

### Example 6: N-Cyclopentyl-3-[2-(cyclopentylamino)-4-pyrimidinyl]-2-(4-fluorophenyl)-7-(2-methoxyethoxy)pyrazolo[1,5-a]pyridin-5-amine.

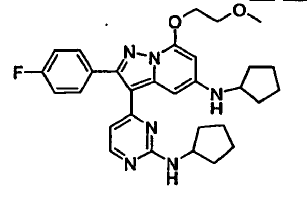

In a similar manner as described in Example 4 from *N*-cyclopentyl-3-[2-(cyclopentylamino)-4-pyrimidinyl]-2-(4-fluorophenyl)-7-(methylsulfanyl)pyrazolo[1,5-a]pyridin-5-amine (90 mg, 0.18 mmol) and methoxyethanol was obtained *N-*cyclopentyl-3-[2-(cyclopentylamino)-4-pyrimidinyl]-2-(4-fluorophenyl)-7-(2-methoxyethoxy)pyrazolo[1,5-a]pyridin-5-amine (20 mg, 21% for 2 steps) as a solid. ¹H-NMR (CDCl₃): δ 7.95 (d,1H), 7.62 (q, 2H), 7.20 (d, 1H), 7.13 (t, 2H), 6.20 (d,1H), 5.75 (d, 1 H), 5.05 (m, 1 H), 4.46 (t, 2H), 4.10 (d, 1H), 3.93 (t, 2H), 3.49 (s, 3H), 2.0-2.1 (m, 4H), 1.5-1.9 (m, 12H); ¹⁹F-NMR (CDCl₃): δ -113.85; MS *m*/*z* 532 (M+1).

### Example 7: N-Cyclopentyl-3-[2-(cyclopentylamino)-4-pyrimidinyl]-2-(4-methoxyphenyl)-pyrazolo[1,5-a]pyridin-5-amine.

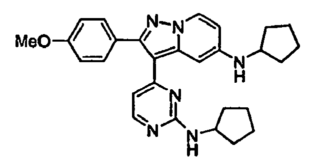

### a) 2-(4-Chloro-2-pyridinyl)-1-(4-methoxyphenyl)ethanone.

To a cold (0°C) solution of 4-chloro-2-picoline (10 g, 78.4 mmol) and ethyl 4-methoxybenzoate (14.1 g, 78.4 mmol) in tetrahydrofuran (100 mL) was added lithium bis(trimethylsilyl)amide (157 mL, 1.0 M in tetrahydrofuran, 157 mmol) dropwise *via* a pressure equalizing funnel over half an hour. Upon complete addition, the ice bath was removed and the resulting solution was heated at 45°C for 15 hours. The mixture was cooled to room temperature, and the solution was concentrated. Methanol was added to quench the reaction, resulting in the formation of a yellow precipitate. The precipitate was collected by filtration and dried to give the product as a mixture of enol and ketone tautomers. MS *m*/*z* 262 (M+1).

### b) 2-(4-Chloro-2-pyridinyl)-1-(4-methoxyphenyl)ethanone oxime.

To a solution of 2-(4-chloro-2-pyridinyl)-1-(4-methoxyphenyl)ethanone in methanol (200 mL) was added hydroxylamine hydrochloride (27.2 g, 392 mmol) followed by the addition of a sodium hydroxide solution (15.7 g, 392 mmol in 50 mL of water). The resulting suspension was heated at reflux for 1 hour and then allowed to cool to room temperature. The mixture was concentrated and water was added to the resulting slurry. A white precipitate formed, which was collected by filtration, washed with water and dried to give 2-(4-chloro-2-pyridinyl)-1-(4-methoxyphenyl)ethanone oxime (11.8 g) as a white solid. ¹H NMR (CDCl₃): δ 8.47 (d, 1H), 7.72 (d, 2H), 7.36 (d, 1H), 7.19 (dd, 1H), 6.91 (d, 2H), 4.43 (s, 2H), 3.84 (s, 3H); MS *m*/*z* 277 (M+1).

### c) 5-Chloro-2-(4-methoxyphenyl)pyrazolo[1,5-a]pyridine.

To a solution of 2-(4-chloro-2-pyridinyl)-1-(4-methoxyphenyl)ethanone oxime (11.8 g, 42.6 mmol) in 1,2-dimethoxyethane (200 mL) at 0°C was added trifluoroacetic anhydride (6.3 mL, 44.8 mmol), keeping the temperature below 10°C during the addition. After the addition was complete, the reaction was warmed to 15°C. The solution was then cooled to 4°C and a solution of triethylamine (12.5 mL, 89.5 mmol) in 1,2-dimethoxyethane (15 mL) was added over a period of 0.5 hours. The mixture was allowed to warm to room temperature and was stirred at room temperature for 5 hours. To this mixture was added iron(II)chloride (0.11 g, 0.85 mmol) and the reaction was heated at 75°C for 15 hours. The reaction mixture was poured into water (300 mL). The resulting suspension was extracted with ethyl acetate. The organic phase was dried (magnesium sulfate), filtered and concentrated to a solid. This solid was recrystallized from methanol to give 5-chloro-2-(4-methoxyphenyl)pyrazolo[1,5-*a*]pyridine (6.64 g, 60%) as white needles. ¹H NMR (CDCl₃): δ 8.35 (d, 1H). 7.86 (d, 2H), 7.46 (d, 1H). 6.97 (d, 2H), 6.67 (d, 1H), 6.65 (s, 1H). 3.85 (s, 3H); MS *m*/*z* 259 (M+1).

### d) 1-[5-(Chloro)-2-(4-methoxyphenyl)pyrazolo[1,5-a]pyridin-3-yl]ethanone.

To a solution of 5-chloro-2-(4-methoxyphenyl)pyrazolo[1,5-*a*]pyridine (3.0 g, 11.6 mmol) in toluene (100 mL) at room temperature was added acetic anhydride (1.6 mL, 17.4 mmol). Boron trifluoride diethyletherate (1.8 mL, 13.9 mmol) was then added dropwise and the resulting solution was heated at reflux for 4 hours. The reaction mixture was cooled to room temperature and quenched by the dropwise addition of saturated aqueous sodium bicarbonate. The reaction was extracted with ethyl acetate, and the ethyl acetate phase washed with brine, dried (magnesium sulfate), filtered and concentrated. The residue was purified by recrystallization from ethyl acetate-hexanes to give 1-[5-(chloro)-2-(4-methoxyphenyl)pyrazolo[1,5-*a*]pyridin-3-yl]ethanone (2.31 g, 66%). ¹H NMR (CDCl₃): δ 8.44 (d, 1H), 8.40 (d, 1H), 7.49 (d, 2H), 7.02(d, 2H), 6.97 (dd, 1 H), 3.85 (s, 3H), 2.15 (s, 3H); MS *m*/*z* 301 (M+1).

### e). 1-[5-(Cyclopentylamino)-2-(4-methoxyphenyl)pyrazolo[1,5-a]pyridin-3-yl]ethanone.

To a solution of 1-[5-(chforo)-2-(4-methoxyphenyl)pyrazolo[1,5-a]pyridin-3-yl]ethanone (1.77 g, 5.88 mmol) in toluene (60 mL) was added successively *racemic-*2,2'-bis(diphenylphosphino)-1,1'-binaphthyl (220 mg, 0.35 mmol), cesium carbonate (2.88 g, 8.83 mmol), cyclopentylamine (2.9 mL, 29.4 mmol), and palladium (II) acetate (53 mg, 0.24 mmol). The resulting mixture was stirred at 95°C for 3 days, at which time the reaction was judged complete by thin layer chromatography. The solution was cooled to room temperature and diethyl ether and water were added to the reaction mixture. The phases were separated, and the aqueous phase again extracted with diethyl ether. The combined organic phases were dried (magnesium sulfate), filtered and concentrated. The resulting residue was purified by flash chromatography (3:2 hexanes:ethyl acetate) to give 1-[5-(cyclopentylamino)-2-(4-methoxyphenyl)pyrazolo[1,5-*a*]pyridin-3-yl]ethanone (1.14 g, 56%) as a yellow solid. ¹H NMR (CDCl₃): δ 8.19 (d, 1H), 7.52 (d, 2H), 7.45 (d, 1 H), 7.03 (d, 2H), 6.35 (dd, 1H), 4.15 (broad s, 1H), 3.98 (m, 1H), 3.91 (s, 3H), 2.21-2.15 (m, 2H), 2.11 (s, 3H), 1.79-1.54 (m, 6H); MS *m*/*z* 350 (M+1).

### f) 1-[5-(Cyclopentylamino)-2-(4-methoxyphenyl)pyrazolo[1,5-a]pyridin-3-yl]-3-(dimethylamino)-2-propen-1-one.

A solution of 1-[5-(cyclopentylamino)-2-(4-methoxyphenyl)pyrazolo[1,5-a]pyridin-3-yl]ethanone (1.14 g, 3.26 mmol) in *N,N*-dimethylformamide dimethyl acetal (25 mL) was heated at reflux for 5 days. The mixture was allowed to cool to room temperature. Water was added and the resulting mixture was extracted with ethyl acetate. The organic phase was dried (magnesium sulfate), filtered and concentrated. The resulting residue was crystallized from ethyl acetate to give 1-[5-(cyclopentylamino)-2-(4-methoxyphenyl)pyrazolo[1,5-*a*]pyridin-3-yl]-3-(dimethylamino)-2-propen-1-one (1.05 g, 80%) as a yellow solid. ¹H NMR (CDCl₃): δ 8.11 (d, 1 H), 7.56 (m, 3H), 7.41 (d, 1 H), 6.95 (d, 2H), 6.22 (dd, 1 H), 5.07 (d, 1 H), 4.11 (d, 1H), 3.95 (m, 1H), 3.84 (s, 3H), 3.0-2.3 (broad, 6H), 2.12(m, 2H), 1.74-1.48 (m, 6H); MS *m*/*z* 405 (M+1).

### g) N-Cyclopentyl-3-[2-(cyclopentylamino)-4-pyrimidinyl]-2-(4-methoxyphenyl)-pyrazolo[1,5-o]pyridin-5-amine.

To a solution of 1-[5-(cyclopentylamino)-2-(4-methoxyphenyl)pyrazolo[1,5-*a*]pyridin-3-yl]-3-(dimethylamino)-2-propen-1-one (1.05 g, 2.60 mmol) in N,N-dimethylformamide (20 mL) was added *N*-cyclopentyl guanidine hydrochloride (1.27 g, 7.79 mmol; Prepared by modification of a procedure from Bannard, R. A. B. et al., *Can. J.* Chem. 1958. *36,* 1541-1549), followed by potassium carbonate (0.54 g, 3.89 mmol). The resulting solution was heated at reflux for 15 hours. Upon cooling to room temperature, water was added. The mixture was extracted with ethyl acetate. The ethyl acetate phase was washed with brine, dried (magnesium sulfate), filtered and concentrated *in vacuo.* The resulting residue was purified by flash chromatography (4:6 ethyl acetate:hexane) to give *N*-cyclopentyl-3-[2-(cyclopentylamino)-4-pyrimidinyl]-2-(4-methoxyphenyl)-pyrazolo[1,5-a]pyridin-5-amine (1.06 g, 87%) as a yellow solid. ¹H.NMR (CDCl₃): δ 8.15 (d, 1H), 7.91 (d, 1H), 7.51 (d, 2H), 7.41 (d, 1H), 6.94 (d, 2H), 6.26 (d, 1H), 6.22 (dd, 1H), 5.11 (d, 1H), 4.42 (m, 1H). 4.09 (d, 1H), 3.88 (m, 1H). 3.85 (s, 3H), 2.10-2.01 (m, 4H), 1.76-1.52 (m, 12H); MS *m*/*z* 469 (M+1).

### Example 8: N-Cyclopentyl-3-[2-(cyclopentylamino)-4-pyrimidinyl]-2-(4-methoxyphenyl)-pyrazolo[1,5-o]pyridin-5-amine (An alternative synthesis).

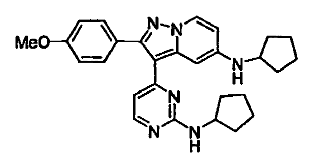

### a) 5-Chloro-2-(4-methoxyphenyl)pyrazolo[1,5-α]pyridine-3-carbaldehyde.

To *N,N-*dimethylformamide (20 mL) at 0°C was added phosphorous oxychloride (0.54 mL, 7.8 mmol). After the addition was complete, the mixture was warmed to room temperature and stirred for 1 hour. To this was added 5-chloro-2-(4-methoxyphenyl)pyrazolo[1,5-*a*]pyridine (1.0 g, 3.86 mmol) and the resultant solution was stirred 2 hours. Water was added, followed by dichloromethane. The aqueous layer was extracted with dichloromethane. The combined organics were washed with brine, dried over magnesium sulfate, filtered and concentrated. A white crystalline compound, 5-chloro-2-(4-methoxyphenyl)pyrazolo[1,5-*a*]pyridine-3-carbaldehyde (0.9 g, 81%), was obtained. ¹H NMR (CDCl₃): δ 10.12 (s, 1 H), 8.52 (d,1H), 8.47 (d, 1 H), 7.76 (d, 2H), 7.11-7.06 (m, 3H), 3.93(s, 3H); MS *m*/*z* 287 (M+1).

### b) 1-[5-Chloro-2-(4-methoxyphenyl)pyrazolo[1,5-a]pyridin-3-yl]-2-propyn-1-ol.

To a cold (-78°C) suspension of 5-chloro-2-(4-methoxyphenyl)pyrazolo[1,5-*a*]pyridine-3-carbaldehyde (0.90 g, 3.14 mmol) in tetrahydrofuran (50 mL) was added ethynylmagnesium bromide (7.5 mL, 0.5 M in tetrahydrofuran, 3.77 mmol) dropwise. The reaction mixture was stirred at -78°C for 1 hour, then at room temperature for 4 hours. The resultant solution was poured into saturated aqueous sodium bicarbonate and extracted with ethyl acetate. The organic layer was washed with water and brine and the combined organics were dried over magnesium sulfate. Filtration and concentration provided 1-[5-chloro-2-(4-methoxyphenyl)pyrazolo[1,5-*a*]pyridin-3-yl]-2-propyn-1-ol (1.05 g, 100%) as a white solid. ¹H NMR (CDCl₃) δ 8.40 (d, 1H), 8.05 (s, 1 H), 7.72 (d, 2H), 7.05 (d, 2H), 6.80 (dd, 1 H), 5.78 (s, 1 H), 3.91 (s, 3H), 2.74 (s, 1 H), 2.53 (s, 1H); MS *m*/*z* 313 (M+1).

### c) 1-[5-Chloro-2-(4-methoxyphenyl)pyrazolo[1,5-a]pyridin-3-yl]-2-propyn-1-one.

To a solution of 1-[5-chloro-2-(4-methoxyphenyl)pyrazolo[1,5-*a*]pyridin-3-yl]-2-propyn-1-ol (1.05 g, 3.14 mmol) in chloroform (100 mL) was added manganese dioxide (6.82 g, 78.5 mmol). The reaction mixture was stirred at room temperature for 3.5 hours. The suspension was filtered through a pad of Celite and the filtrate was concentrated to give 1-[5-chloro-2-(4-methoxyphenyl)pyrazolo[1,5-*a*]pyridin-3-yl)-2-propyn-1-one (0.99 g, 100%) as a pale yellow solid. ¹H NMR (CDCl₃) δ 8.50 (d, 1 H), 8.46 (d, 1H), 7.64 (d, 2H), 7.04 (dd, 1H), 6.98 (d, 2H), 3.87 (s, 3H), 2.99 (s, 1H); MS *m*/*z* 295 (M+1).

### d) 4-[5-Chforo-2-(4-methoxyphenyl)pyrazolo[1,5-a]pyridin-3-yl]-N-cyclopentyl-2-pyrimidinamine.

Sodium ethylate (0.7 mL (2.09 mmol), 21% in ethanol) and cyclopentyl guanidine hydrochloride (0.47 g, 2.88 mmol) were added sequentially to ethanol (30 mL). The resulting solution was stirred at room temperature for 30 minutes. 1-[5-chloro-2-(4-methoxyphenyl)pyrazolo[1,5-*a*]pyridin-3-yl]-2-propyn-1-one (0.5 g, 1.61 mmol) was added, and the suspension was stirred at room temperature for 2 days. The reaction was quenched by the addition of water. The aqueous phase was extracted by ethyl acetate. The organics were combined, washed with brine and dried over magnesium sulfate. Filtration and concentration gave a solid. This solid was recrystallized from methanol to give 4-[5-chloro-2-(4-methoxyphenyl)pyrazolo[1,5-*a*]pyridin-3-yl]-*N-*cyclopentyl-2-pyrimidinamine (0.45 g, 66%) as a pale yellow solid. ¹H NMR (CDCl₃) δ 8.59 (b, 1H), 8.42 (d, 1 H), 8.05 (d, 1H), 7.59 (d, 2H), 7.03 (d, 2H), 6.91 (dd, 1H), 6.39 (d, 1H), 5.34 (broad s, 1H). 4.42 (m, 1H), 3.92 (s, 3H), 2.17 (m, 2H), 1.86-1.60 (m, 6H); MS m/z 420 (M+1).

### e) N-Cyclopentyl-3-[2-(cyclopentylamino)-4-pyrimidinyl]-2-(4-methoxyphenyl)-pyrazolo[1,5-a]pyridin-5-amine.

Treatment of 4-[5-chloro-2-(4-methoxyphenyl)pyrazolo[1,5-*a*]pyridin-3-yl]-*N-*cyclopentyl-2-pyrimidinamine with cyclopentylamine under similar conditions as described in Example 7 gives the desired *N*-Cyclopentyl-3-[2-(cyclopentylamino)-4-pyrimidinyl]-2-(4-methoxyphenyl)-pyrazolo[1,5-a]pyridin-5-amine.

### Example 9: N-Cyclopentyl-3-[2-(cyclopentylamino)-4-pyrimidinyl]-2-(4-methoxyphenyl)-7-(methylsulfanyl)pyrazolo[1,5-o]pyridin-5-amine.

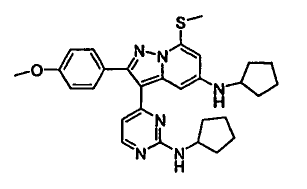

A solution of *N*-cyelopentyl-3-[2-(cyclopentylamino)-4-pyrimidinyl]-2-(4-methoxyphenyl)-pyrazolo[1,5-*a*]pyridin-5-amine (0.05 g, 0.11 mmol) was treated with n-butyllithium and methyl disulfide as described in Example 2 to give *N*-cyclopentyl-3-[2-(cyclopentylamino)-4-pyrimidinyl]-2-(4-methoxyphenyl)-7-(methylsulfanyl)pyrazolo[1,5-*a*]pyridin-5-amine (40 mg, 71 %) as a yellow foam. ¹H NMR (CDCl₃): δ 7.95 (d, 1H). 7.58 (d, 2H), 7.36 (d, 1H), 6.97, (d, 2H), 6.28 (d, 1H), 6.04 (d, 1H), 5.20 (d, 1 H), 4.47 (m, 1H), 4.15 (m, 1H) 3.93 (m, 1H), 3.88 (s, 3H), 2.58 (s, 3H), 2.0-2.1 (4H), 1.5-1.9 (m.12H): MS *m*/*z* 516 (M+1).

### Example 10: N-Cyclopentyl-3-[2-(cyclopentylamino)-4-pyrimidinyl]-7-(ethylsulfanyl)-2-(4-methoxyphenyl)pyrazolo[1,5-a]pyridin-5-amine.

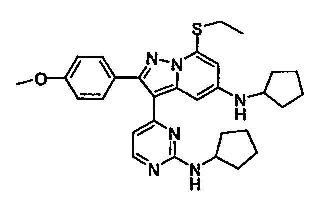

A solution of *N*-cyclopentyl-3-[2-(cyclopentylamino)-4-pyrimidinyl]-2-(4-methoxyphenyl)-pyrazolo[1,5-*a*]pyridin-5-amine (0.05 g, 0.11 mmol) was treated with n-butyllithium and ethyl disulfide as described in Example 2 to give *N*-cyclopentyl-3-[2-(cyclopentylamino)-4-pyrimidinyl]-7-(ethylsulfanyl)-2-(4-methoxyphenyl)pyrazolo[1,5-*a*]pyridin-5-amine (40 mg) as a yellow foam. ¹H NMR (CDCl₃): δ 7.97 (d, 1H), 7.58 (d, 2H), 7.37 (d, 1H), 6.96 (d, 2H), 6.29 (d, 1H), 6.16 (d, 1H), 5.04 (m, 1H), 4.46 (m, 1H). 4.07 (d, 1H), 3.94 (m, 1H), 3.89 (s, 3H), 3.14 (q. 2H), 2.0-2.1 (m, 4H), 1.5-1.9 (m, 12H), 1.46 (t, 3H); MS *m*/*z* 530 (M+1).

### Example 11: N-Cyclopentyl-3-[2-(cyclopentylamino)-4-pyrimidinyl]-7-(isopropylsulfanyl)-2-(4-methoxyphenyl)pyrazolo[1,5-a]pyridin-5-amine.

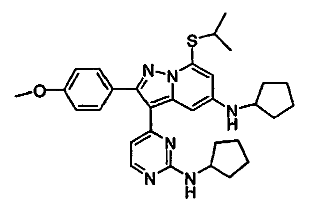

A solution of *N*-cyclopentyl-3-[2-(cyclopentylamino)-4-pyrimidinyl]-2-(4-methoxyphenyl)-pyrazolo[1,5-o]pyridin-5-amine (0.05 g, 0.11 mmol) was treated with n-butyllithium and isopropyl disulfide as described in Example 2 to give N-cyclopentyl-3-[2-(cyclopentylamino)-4-pyrimidinyl]-7-(isopropylsulfanyl)-2-(4-methoxyphenyl)pyrazolo[1,5-*a*]pyridin-5-amine (35 mg, 60%) as a yellow foam. ¹H NMR (CDCl₃): δ 7.97 (d, 1H), 7.58 (d, 2H), 7.40 (m, 1H), 6.97 (d, 2H), 6.30 (m, 2H), 5.10 (d, 1H), 4.46 (m. 1H), 4.08 (d, 1H), 3.93 (m, 1H), 3.89 (s, 3H), 2.0-2.1 (m, 5H), 1.5-1.9 (m,12H), 1.43 (d, 6H); MS *m*/*z* 544 (M+1).

### Example 12: N-Cyclopentyl-3-[2-(cyclopentylamino)-4-pyrimidinyl]-2-(4-methoxyphenyl)-7-(phenylsulfanyl)pyrazolo[1,5-a]pyridin-5-amine.

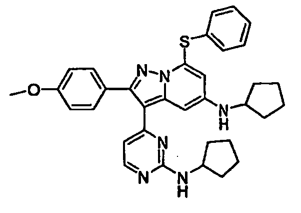

A solution of *N*-cyclopentyl-3-[2-(cyclopentylamino)-4-pyrimidinyl]-2-(4-methoxyphenyl)-pyrazolo[1,5-*a*]pyridin-5-amine (0.05 g, 0.11 mmol) was treated with n-butyllithium and phenyl disulfide as described in Example 2 to give *N*-cyclopentyl-3-[2-(cyclopentylamino)-4-pyrimidinyl]-2-(4-methoxyphenyl)-7-(phenylsulfanyl)pyrazolo[1,5-*a*]pyridin-5-amine (30 mg, 49%) as a yellow foam. ¹H NMR (CDCl₃): δ 7.97 (d, 1H), 7.73 (m, 2H), 7.61 (d, 2H), 7.54 (m, 3H), 7.32 (d, 1H), 6.98 (d, 2H), 6.31 (d, 1H), 5.53 (d, 1H), 5.05 (d, 1H), 4.45 (m, 1H), 3.90 (s, 3 H), 3.80 (m, 1H), 2.0-2.1 (m, 4H), 1.5-1.9 (m, 12H); MS *m*/*z* 578 (M+1).

### Example 13: N-Cydopentyl-3-[2-(cydopentylamino)-4-pyrimidinyl]-2-(4-fluorophenyl)-7-(isopropylsulfanyl)pyrazolo[1,5-a]pyridin-5-amine.

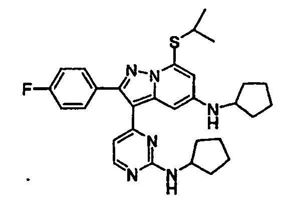

*N*-Cyclopentyl-3-[2-(cyclopentylamino)-4-pyrimidinyl]-2-(4-fluorophenyl)pyrazolo[1,5-a]pyridin-5-amine (60 mg, 0.13 mmol) was treated with n-butyllithium and isopropyl disulfide as described in Example 2 to give *N*-cyclopentyl-3-[2-(cyclopentylamino)-4-pyrimidinyl]-2-(4-fluorophenyl)-7-(isopropylsulfanyl)pyrazolo[1,5-*a*]pyridin-5-amine (50 mg, 72 %) as a yellow solid. ¹H-NMR (CDCl₃): δ 7.99 (d, 1H), 7.62 (q, 2H), 7.36 (d, 1H), 7.11 (t, 2H), 6.30 (d, 1H), 6.24 (d, 1H). 5.10 (d, 1H). 4.45 (m, 1H), 4.10 (d, 1H). 3.90 (m, 2H), 2.0-2.1 (m, 4H), 1.5-1.9 (m, 12H), 1.33 (d, 6H); ¹⁹F-NMR (CDCl₃): δ -113.90; MS *m*/*z* 532 (M+1).

### Example 14: Biological Activity

In the following example, "MEM" means Minimal Essential Media; "FBS" means Fetal Bovine Serum; "NP40" and "Igepal" are detergents; "MOI" means Multiplicity of Infection; "NaOH" means sodium hydroxide; "MgCl₂" means magnesium chloride; "dATP" means deoxyadenosine 5' triphosphate; "dUTP" means deoxyuridine 5' triphosphate; "dCTP" means dexoxycytidine 5' triphosphate; "dGTP" means deoxyguanosine 5' triphosphate; "GuSCN" means Guanidinium thiocyanate; "EDTA" means ethylenediamine tetraacetic acid; "TE" means Tris-EDTA; "SCC" means sodium chloride/sodium citrate; "APE" means a solution of ammonia acetate, ammonia phosphate, EDTA; "PBS" means phosphate buffered saline; and "HRP" means horseradish peroxidase.

### a) Tissue Culture and HSV infection.

Vero 76 cells were maintained in MEM with Earle's salts, L-glutamine, 8% FBS (Hyclone, A-1111-L) and 100 units/mL Penicillin-100 µg/mL Streptomycin. For assay conditions, FBS was reduced to 2%. Cells are seeded into 96-well tissue culture plates at a density of 5 x 10⁴ cells/well after being incubated for 45 min at 37°C in the presence of HSV-1 or HSV-2 (MOI =0.001). Test compounds are added to the wells and the plates are incubated at 37°C for 40- 48 hours. Cell lysates are prepared as follows: media was removed and replaced with 150 µL/well 0.2 N NaOH with 1% Igepal CA 630 or NP-40. Plates were incubated up to 14 days at room temperature in a humidified chamber to prevent evaporation.

### (b) Preparation of detection DNA.

For the detection probe, a gel-purified, digoxigenin-labeled, 710-bp PCR fragment of the HSV UL-15 sequence was utilized. PCR conditions included 0.5 µM primers, 180 µM dTTP, 20 µM dUTP-digoxigenin (Boehringer Mannheim 1558706), 200 µM each of dATP, dCTP, and dGTP, 1X PCR Buffer II (Perkin Elmer), 2.5 mM MgCl₂, 0.025 units/µL of AmpliTaq Gold polymerase (Perkin Elmer), and 5 ng of gel-purified HSV DNA per 100 µL Extension conditions were 10 min at 95°C, followed by 30 cycles of 95°C for 1 min, 55°C for 30 sec, and 72°C for 2 min. The amplification was completed with a 10-min incubation at 72°C. Primers were selected to amplify a 278 bp prove spanning a section of the HSV1 UL15 open reading frame (nucleotides 249-977). Single-stranded transcripts were purified with Promega M13 Wizard kits. The final product was mixed 1:1 with a mixture of 6 M GuSCN, 100 mM EDTA and 200 µg/mL herring sperm DNA and stored at 4°C.

### (c) Preparation of capture plates.

The capture DNA plasmid (HSV UL13 region in pUC) was linearized by cutting with Xba I, denatured for 15 min at 95°C and diluted immediately into Reacti-Bind DNA Coating Solution (Pierce, 17250, diluted 1 :1 with TE buffer, pH 8) at 1 ng/µL 75 µL/well were added to Corning (#3922 or 9690) white 96-well plates and incubated at room temperature for at least 4 hrs before washing twice with 300 µL/well 0.2X SSC/0.05% Tween-20 (SSC/T buffer). The plates were then incubated overnight at room temperature with 150 µL/well 0.2 N NaOH, 1% IGEPAL and 10 µg/mL herring sperm DNA.

### (d) Hybridization.

Twenty-seven (27) µL of cell lysate was combined with 45 µL of hybridization solution (final concentration: 3M GuSCN, 50 mM EDTA, 100 µg/ml salmon sperm DNA, 5X Denhardt's solution, 0.25X APE, and 5 ng of the digoxigenin-labeled detection probe). APE is 1.5 M NH₄-acetate, 0.15 M ammonium phosphate monobasic, and 5 mM EDTA adjusted to pH 6.0. Mineral oil (50 µL) was added to prevent evaporation. The hybridization plates were incubated at 95°C for 10 minutes to denature the DNA, then incubated at 42°C overnight The wells were washed 6X with 300 µL/well SSC/T buffer then incubated with 75 µL/well anti-digoxigenin- HRP-conjugated antibody (Boehringer Mannheim 1207733, 1:5000 in TE) for 30 min at room temperature. The wells were washed 6X with 300 µL/well with PBS/0.05% Tween-20 before 75 µL/well SuperSignal LBA substrate (Pierce) was added. The plates were incubated at room temperature for 30 minutes and chemiluminescence was measured in a Wallac Victor reader.

### e) Results.

The following results were obtained for HSV-1.

| Example No. | IC₅₀ (µM) |
|---|---|
| 2 | 0.074 |
| 3 | 0.322 |
| 4 | 0.160 |
| 5 | 0.13 |
| 6 | 0.082 |
| 9 | 0.135 |
| 10 | 0.046 |
| 11 | 0.175 |
| 12 | 2.0 |
| 13 | 0.26 |

The results demonstrate that the compounds of the present invention are useful for the treatment and prophylaxis of herpes viral infections.

## Claims

1. A compound of formula (I): 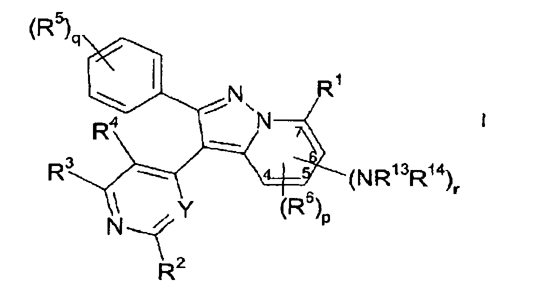 wherein:
R¹ is selected from the group consisting of alkyl, cycloalkyl, -OR⁷, -OAy, - C(O)R⁹, -CO₂R⁹, -C(O)NR⁷R⁸, -S(O)ₙR⁹, -S(O)ₙAy, -S(O)ₙHet, -S(O)₂NR⁷R⁸, - R¹⁰cycloalkyl, -R¹⁰OR⁹, -R¹⁰NR⁷R⁸, cyano, nitro and azido;
each R⁷ and R⁸ are the same or different and are each independently selected from the group consisting of H, alkyl, alkenyl, cycloalkyl, cycloalkenyl, -OR⁹, -C(O)R⁹, -CO₂R⁹, -C(O)NR⁹R¹¹, -C(S)NR⁹R¹¹, - C(NH)NR⁹R¹¹, -SO₂R¹⁰, -SO₂NR⁹R¹¹, -R¹⁰cycloalkyl, -R¹⁰OR⁹, - R¹⁰C(O)R⁹, -R¹⁰CO₂R⁹, -R¹⁰C(O)NR⁹R¹¹, -R¹⁰C(S)NR⁹R¹¹, -R¹⁰C(NH)NR⁹R¹¹, -R¹⁰SO₂R¹⁰, -R¹⁰SO₂NR⁹R¹¹, -R¹⁰NR⁹R¹¹, -R¹⁰NHCOR⁹, -R¹⁰NHC(NH)NR⁹R¹¹, -R¹⁰NHSO₂R⁹ and -R¹⁰SO₂NHCOR⁹;
each R⁹ and R¹¹ are the same or different and are each independently selected from the group consisting of H, alkyl, cycloalkyl, - R¹⁰cycloalkyl, -R¹⁰OH, -R¹⁰(OR¹⁰)_{w} where w is 1-10, and -R¹⁰NR¹⁰R¹⁰;
each R¹⁰ is the same or different and is independently selected from the group consisting of alkyl; alkenyl, alkynyl, cycloalkyl and cycloalkenyl;
Ay is aryl;
Het is a 5- or 6-membered heterocyclic or heteroaryl group;
n is 0, 1 or 2;
R² is selected from the group consisting of Het, -OR⁷, -S(O)ₙR⁹, -NR⁷R⁸, -NHHet and -NHR¹⁰Het;
Y is N or CH;
R³ and R⁴ are the same or different and are each independently selected from the group consisting of H, halo, alkyl, alkenyl, cycloalkyl, Ay, Het, -OR⁷, -OAy, -C(O)R⁷, C(O)Ay, -CO₂R⁷, -CO₂Ay, -SO₂NHR⁹, -NR⁷R⁸, -NR⁷Ay, -NHHet, -NHR¹⁰Het, -R¹⁰OR⁷, -R¹⁰OAy, -R¹⁰NR⁷R⁸ and -R¹⁰NR⁷ Ay;
q is 0, 1, 2, 3, 4 or 5;
each R⁵ is the same or different and is independently selected from the group consisting of halo, alkyl, alkenyl, alkynyl, cycloalkyl, cycloalkenyl, Ay, Het, -OR⁷, -OAy, -OHet, -C(O)R⁹, -C(O)Ay, -C(O)Het, -C(O)NR⁷R⁸, - C(S)NR⁹R¹¹, -C(O)NR⁷Ay, -C(O)NHR¹⁰Het, -CO₂R⁹, -C(NH)NR⁷R⁸, -C(NH)NR⁷Ay, -S(O)ₙR⁹, -S(O)ₙAy, -S(O)ₙHet, -S(O)₂NR⁷R⁸, -S(O)₂NR⁷Ay, -NR⁷R⁸, -NR⁷Ay, -NHHet, -NHR¹⁰Ay, -NHR¹⁰Het, -R¹⁰cycloalkyl, -R¹⁰OR⁹, -R¹⁰C(O)R⁹, -R¹⁰CO₂R⁹, -R¹⁰C(O)NR⁹R¹¹, -R¹⁰C(S)NR⁹R¹¹, - R¹⁰C(NH)NR⁹R¹¹, -R¹⁰SO₂R⁹, -R¹⁰SO₂NR⁹R¹¹, -R¹⁰SO₂NHCOR⁹, -R¹⁰NR⁷R⁸, -R¹⁰NR⁷Ay, -R¹⁰NHC(NH)NR⁹R¹¹, cyano, azido and nitro; or
two adjacent R⁵ groups together with the atoms to which they are bonded form a C₅₋₆cycloalkyl or aryl;
r is 1, 2 or 3;
each R¹³ and R¹⁴ are the same or different and are each independently selected from the group consisting of H, alkyl, alkenyl, cycloalkyl, cycloalkenyl, Ay, Het, -C(O)R⁹, -CO₂R⁹, -C(O)NR⁹R¹¹, -C(S)NR⁹R¹¹, -C(NH)NR⁹R¹¹, -SO₂R¹⁰, - SO₂NR⁹R¹¹, -R¹⁰cycloalkyl, -R¹⁰OR⁹, -R¹⁰C(O)R⁹, -R¹⁰CO₂R⁹, -R¹⁰C(O)NR⁹R¹¹,-R¹⁰C(S)NR⁹R¹¹, -R¹⁰C(NH)NR⁹R¹¹, -R¹⁰SO₂R¹⁰, -R¹⁰SO₂NR⁹R¹¹, -R¹⁰NR⁹R¹¹, -R¹⁰NHCOR⁹, -R¹⁰NHC(NH)NR⁹R¹¹, -R¹⁰NHSO₂R⁹ and -R¹⁰SO₂NHCOR⁹;
p is 0, 1 or 2, wherein p + r ≤ 3; and
each R⁶ is the same or different and is independently selected from the group consisting of halo, alkyl, Het, -OR⁷, -C(O)NR⁷R⁸, -S(O)ₙR⁹ -S(O)₂NR⁷R⁸, and cyano; or two adjacent R⁶ groups together with the atoms to which they are bonded form a C₅₋₆cycloalkyl or 5- or 6-membered heterocyclic group containing 1 or 2 heteroatoms;
wherein when Y is CH, R³ is not -NR⁷Ay;
and pharmaceutically acceptable salts and solvates thereof.

2. The compound according to claims 1 wherein R¹ is selected from the group consisting of alkyl, -OR⁷, -C(O)NR⁷R⁸ and S(O)ₙR⁹.

3. The compound according to any of claims 1-2 wherein R² is selected from the group consisting of Het, -NR⁷R⁸ and NHHet.

4. The compound according to any of claims 1-3 wherein Y is N.

5. The compound according to any of claims 1-3 wherein Y is CH.

6. The compound according to any of claims 1-5 wherein R³ and R⁴ are the same or different and are each independently selected from the group consisting of H, halo, alkyl, Ay, -OR⁷, -CO₂R⁷ -NR⁷R⁸, -R¹⁰OR⁷ and -R¹⁰NR⁷R⁸.

7. The compound according to any of claims 1-5 wherein R³ and R⁴ are each H.

8. The compound according to any of claims 1-7 wherein q is 0, 1 or 2.

9. The compound according to any of claims 1-8 wherein each R⁵ is the same or different and is independently selected from the group consisting of halo, alkyl, alkenyl, Ay, Het, -OR⁷, -OAy, -CO₂R⁹, -C(O)NR⁷R⁸, -C(O)NR⁷Ay, - S(O)₂NR⁷R⁸, -NR⁷R⁸, -NR⁷Ay, -NHR¹⁰Ay, cyano, nitro and azido.

10. The compound according to any of claims 1-8, wherein each R⁵ is the same or different and is independently selected from the group consisting of halo, alkyl, -OR⁷, -NR⁷R⁸ and cyano.

11. The compound according to any of claims 1-10, wherein p is 0 or 1.

12. The compound according to any of claims 1-11 wherein r is 1.

13. The compound according to any of claims 1-12, wherein each R¹³ and R¹⁴ are the same or different and are each independently selected from the group consisting of H, alkyl, cycloalkyl, -C(O)R⁹, -CO₂R⁹, -R¹⁰cycloalkyl, - R¹⁰OR⁹ and -R¹⁰CO₂R⁹, -R¹⁰NR⁹R¹¹.

14. The compound according to any of claims 1-12, wherein each R¹³ and R¹⁴ are the same or different and are each independently selected from the group consisting of H, alkyl and cycloalkyl.

15. A compound selected from the group consisting of:
*N*-Cyclopentyl-3-[2-(cyclopentylamino)-4-pyrimidinyl]-7-(ethylsulfanyl)-2-(4-fluorophenyl)pyrazolo[1,5-*a*]pyridin-5-amine;
*N*-Cyclopentyl-3-[2-(cyclopentylamino)-4-pyrimidinyl]-2-(4-fluorophenyl)-7-methylpyrazolo[1,5-*a*]pyridin-5-amine;
*N*-Cyclopentyl-3-[2-(cyclopentylamino)-4-pyrimidinyl]-7-ethoxy-2-(4-fluorophenyl)pyrazolo[1,5-*a*]pyridin-5-amine;
*N*-Cyclopentyl-3-[2-(cyclopentylamino)-4-pyrimidinyl]-2-(4-fluorophenyl)-7-(methylsulfanyl)pyrazolo[1,5-*a*]pyridin-5-amine;
*N*-cyclopentyl-3-[2-(cydopentylamino)-4-pyrimidinyl]-2-(4-fluorophenyl)-7-(2-methoxyethoxy)pyrazolo[1,5-a]pyridin-5-amine;
*N*-Cyclopentyl-3-[2-(cyclopentylamino)-4-pyrimidinyl]-2-(4-methoxyphenyl)-7-(methylsulfanyl)pyrazolo[1,5-*a*]pyridin-5-amine;
*N*-cyclopentyl-3-[2-(cyclopentylamino)-4-pyrimidinyl]-7-(ethylsulfanyl)-2-(4-methoxyphenyl)pyrazolo[1,5-*a*]pyridin-5-amine;
*N*-Cyclopentyl-3-[2-(cyclopentylamino)-4-pyrimidinyl]-7-(isopropylsulfanyl)-2-(4-methoxyphenyl)pyrazolo[1,5-*a*]pyridin-5-amine;
*N*-Cyclopentyl-3-[2-(cyclopentylamino)-4-pyrimidinyl]-2-(4-methoxyphenyl)-7-(phenylsulfanyl)pyrazolo[1,5-*a*]pyridin-5-amine; and
*N*-Cyclopentyl-3-[2-(cyclopentylamino)-4-pyrimidinyl]-2-(4-fluorophenyl)-7-(isopropylsulfanyl)pyrazolo[1,5-*a*]pyridin-5-amine; and
pharmaceutically acceptable salts and solvates thereof.

16. A pharmaceutical composition comprising a compound according to any of claims 1-15.

17. The pharmaceutical composition according to claim 16 further comprising a pharmaceutically acceptable carrier or diluent.

18. The pharmaceutical composition according to claim 16 further comprising an antiviral agent selected from the group consisting of acyclovir, valacyclovir and pharmaceutically acceptable salts thereof.

19. A compound according to any of claims 1-15 for use in therapy.

20. A compound according to any of claims 1-15, for use in the prophylaxis or treatment of a herpes viral infection in an animal.

21. A compound according to any of claims 1-15, for use in the prophylaxis or treatment of a condition or disease associated with a herpes viral infection in an animal.

22. The use of a compound according to any of claims 1-15 for the preparation of a medicament for the prophylaxis or treatment of a herpes viral infection in an animal.

23. The use according to claim 22 wherein said herpes viral infection is selected from the group consisting of herpes simplex virus 1, herpes simplex virus 2, cytomegalovirus, Epstein Barr virus, herpes zoster virus, human herpes virus 6, human herpes virus 7, and human herpes virus 8.

24. The use of a compound according to any of claims 1-15 for the preparation of a medicament for the prophylaxis or treatment of a condition or disease associated with a herpes viral infection in an animal.

25. A pharmaceutical composition comprising a compound according to any of claims 1-15 for use in the preparation of a medicament for the prophylaxis or treatment of a herpes viral infection in an animal.

26. A process for preparing a compound according to any of claims 1-15,
wherein Y is N; R² is selected from the group consisting of Het, -OR⁷,-S(O)ₙR⁹, -NR⁷R⁸, -NHHet and -NHR¹⁰Het, comprising the steps of:
a) reacting a compound of formula (XI): 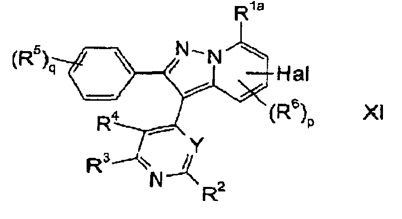 wherein Hal is halo; and
R^{1a} is selected from the group consisting of H, alkyl, cycloalkyl, -OR⁷,-OAy, -C(O)R⁹, -CO₂R⁹, -C(O)NR⁷R⁸, -S(O)ₙR⁹, -S(O)ₙAy, -S(O)ₙHet, - S(O)₂NR⁷R⁸, -R¹⁰cycloalkyl, -R¹⁰OR⁹, -R¹⁰NR⁷R⁸, cyano, nitro and azido;
with an amine or imine to prepare a compound of formula (XII): 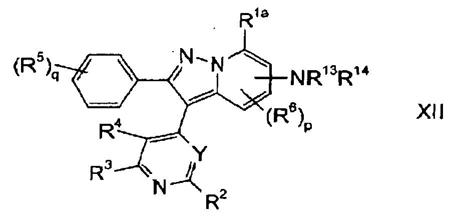 and
b) in the embodiment wherein R^{1a} is H, converting the compound of formula (XII) to a compound of formula (I).

27. A process for preparing a compound according to any of claims 1-15, wherein Y is N; R² is selected from the group consisting of Het, -OR⁷,-S(O)ₙR⁹, -NR⁷R⁸, -NHHet and -NHR¹⁰Het; R³ is H and R⁴ is H,
said process comprising the steps of:
a) reacting a compound of formula (IX): 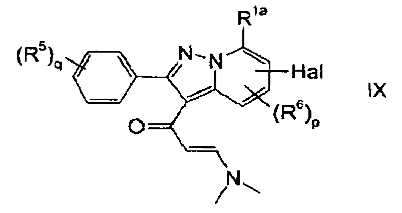 wherein Hal is halo; and
R^{1a} is selected from the group consisting of H, alkyl, cycloalkyl, -OR⁷, - OAy, -C(O)R⁹, -CO₂R⁹, -C(O)NR⁷R⁸, -S(O)ₙR⁹, -S(O)ₙAy, -S(O)ₙHet, - S(O)₂NR⁷R⁸, -R¹⁰cycloalkyl, -R¹⁰OR⁹, -R¹⁰NR⁷R⁸, cyano, nitro and azido;
with a compound of formula (X): 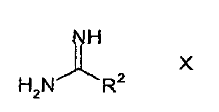 to prepare a compound of formula (XI): 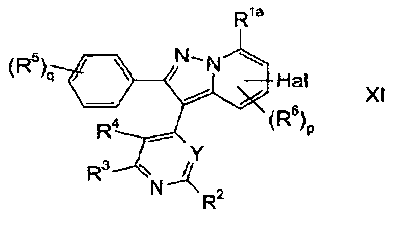
b) reacting the compound of formula (XI) with an amine or imine to prepare a compound of formula (XII): 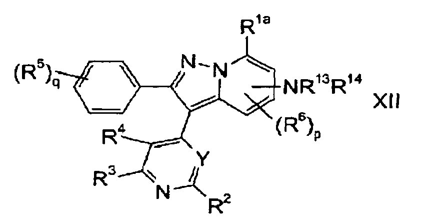 and
c) in the embodiment wherein R^{1a} is H, converting the compound of formula (XII) to a compound of formula (I).

28. A process for preparing a compound according to any of claims 1-15 wherein Y is N; R² is selected from the group consisting of Het, -OR⁷, - S(O)ₙR⁹, -NR⁷R⁸, -NHHet and -NHR¹⁰Het; R³ is selected from the group consisting of H, alkyl, alkenyl, cycloalkyl, Ay, Het, -C(O)R⁷, -C(O)Ay, -CO₂R⁷, - CO₂Ay, -SO₂NHR⁹ -NR⁷R⁸ where R⁷ and R⁸ are not H, -NR⁷Ay where R⁷ is not H, - R¹⁰OR⁷, -R¹⁰OAy, -R¹⁰NR⁷R⁸ and -R¹⁰NR⁷Ay; and R⁴ is H;
said process comprising the steps of:
a) reacting a compound of formula (XXIX): 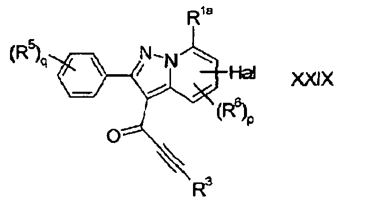 wherein Hal is halo; and
R^{1a} is selected from the group consisting of H, alkyl, cycloalkyl, -OR⁷, - OAy, -C(O)R⁹, -CO₂R⁹, -C(O)NR⁷R⁸, -S(O)ₙR⁹, -S(O)ₙAy, -S(O)ₙHet, - S(O)₂NR⁷R⁸, -R¹⁰cycloalkyl, -R¹⁰OR⁹, -R¹⁰NR⁷R⁸, cyano, nitro and azido;
with a compound of formula (X): 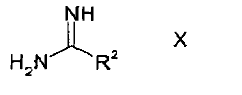 to prepare a compound of formula (XI): 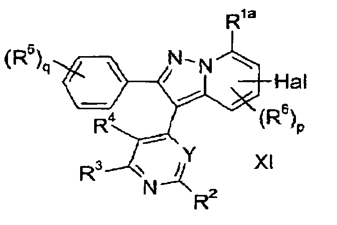
b) reacting the compound of formula (XI) with an amine or imine to prepare a compound of formula (XII): 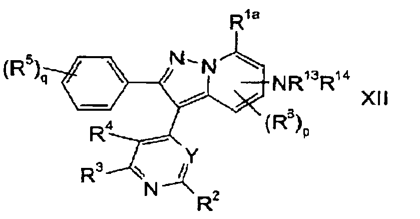 and
c) in the embodiment wherein R^{1a} is H, converting the compound of formula (XII) to a compound of formula (I).

29. A process for preparing a compound according to any of claims 1-15, wherein Y is N and R² is selected from the group consisting of Het, -OR⁷, - S(O)ₙR⁹, -NR⁷R⁸, -NHHet and -NHR¹⁰Het, said process comprising the steps of:
a) reacting a compound of formula (XXXV): 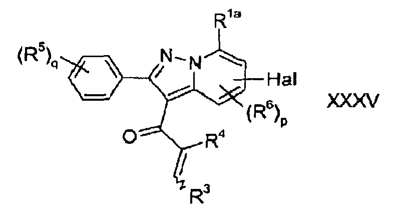 wherein Hal is halo; and
R^{1a} is selected from the group consisting of H, alkyl, cycloalkyl, -OR⁷, - OAy, -C(O)R⁹, -CO₂R⁹, -C(O)NR⁷R⁸, -S(O)ₙR⁹, -S(O)ₙAy, -S(O)ₙHet, - S(O)₂NR⁷R⁸, -R¹⁰cycloalkyl, -R¹⁰OR⁹, -R¹⁰NR⁷R⁸, cyano, nitro and azido;
with a compound of formula (X): 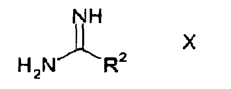 followed by oxidative aromatization, to prepare a compound of formula (XI): 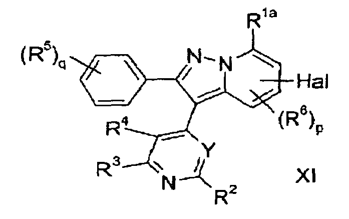
b) reacting the compound of formula (XI) with an amine or imine to prepare a compound of formula (XII): 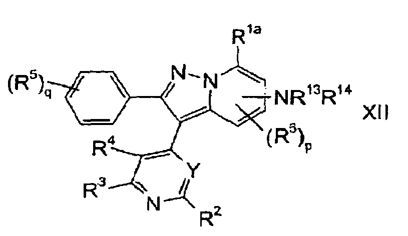 and
c) in the embodiment wherein R^{1a} is H, converting the compound of formula (XII) to a compound of formula (I).

30. A process for preparing a compound according to any of claims 1-15, said process comprising the steps of:
a) reacting a compound of formula (XL): 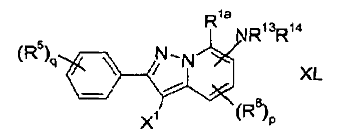 wherein X¹ is halo; and
wherein R^{1a} is selected from the group consisting of H, alkyl, cycloalkyl, -OR⁷, -OAy, -C(O)R⁹, -CO₂R⁹, -C(O)NR⁷R⁸, -S(O)ₙR⁹, -S(O)ₙAy, - S(O)ₙHet, -S(O)₂NR⁷R⁸, -R¹⁰cycloalkyl, -R¹⁰OR⁹, -R¹⁰NR⁷R⁸, cyano, nitro and azido;
with a compound of formula (XLI) 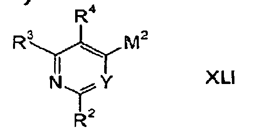 wherein M² is selected from the group consisting of -B(OH)₂, - B(ORa)₂, -B(Ra)₂, -Sn(Ra)₃, Zn-halide, ZnRa, Mg-halide where Ra is alkyl or cycloalkyl and halide is halo;
to prepare a compound of formula (XII): 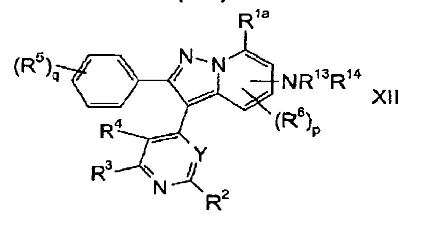 and
c) in the embodiment wherein R^{1a} is H, converting the compound of formula (XII) to a compound of formula (I).

31. The process according to any of claims 26-30 further comprising the step of converting a compound of formula (XII) to a compound of formula (XII-D): 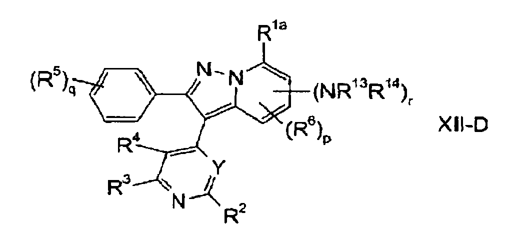 wherein r is 2 or 3.

32. The process according to any of claims 26-31 further comprising the step of converting a compound of formula (I) to a pharmaceutically acceptable salt or solvate thereof.

33. The process according to any of claims 26-32 further comprising the step of converting a compound of formula (I) or a pharmaceutically acceptable salt or solvate or physiologically thereof to another compound of formula (I) or a pharmaceutically acceptable salt or solvate thereof.

## Patentansprüche

1. Verbindung der Formel (I): 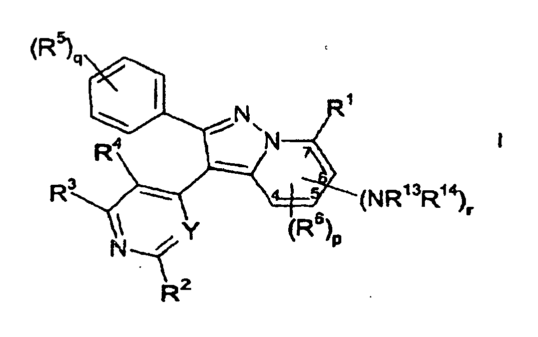 worin:
R¹ ausgewählt ist aus der Gruppe, bestehend aus Alkyl, Cycloalkyl, -OR⁷, -OAy, -C(O)R⁹, -CO₂R⁹, -C(O)NR⁷R⁸, -S(O)ₙR⁹, S(O)ₙAy, S(O)ₙHet, -S(O)₂NR⁷R⁸, -R¹⁰-Cycloalkyl, R¹⁰OR⁹, -R¹⁰NR⁷R⁸, Cyano, Nitro und Azido;
R⁷ und R⁸ jeweils gleich oder unterschiedlich sind und jeweils unabhängig ausgewählt werden aus der Gruppe, bestehend aus H, Alkyl, Alkenyl, Cycloalkyl, Cycloalkenyl, -OR⁹, -C(O)R⁹, -CO₂R⁹, -C(O)NR⁹R¹¹, -C(S)NR⁹R¹¹, -C(NH)NR⁹R¹¹, -SO₂R¹⁰, -SO₂NR⁹R¹¹, -R¹⁰-Cycloalkyl, -R¹⁰OR⁹, -R¹⁰C(O)R⁹, -R¹⁰CO₂R⁹, -R¹⁰C(O)NR⁹R¹¹, -R¹⁰C(S)NR⁹R¹¹, -R¹⁰C(NH)NR⁹R¹¹, -R¹⁰SO₂R¹⁰, -R¹⁰SO₂NR⁹R¹¹, -R¹⁰NR⁹R¹¹, -R¹⁰NHCOR⁹, -R¹⁰NHC(NH)NR⁹R¹¹, -R¹⁰NHSO₂R⁹ und -R¹⁰SO₂NHCOR⁹;
R⁹ und R¹¹ jeweils gleich oder unterschiedlich sind und jeweils unabhängig ausgewählt werden aus der Gruppe, bestehend aus H, Alkyl, Cycloalkyl, -R¹⁰-Cycloalkyl, -R¹⁰OH, -R¹⁰(OR¹⁰)_{w}, worin w 1 bis 10 ist, und -R¹⁰NR¹⁰R¹⁰;
jedes R¹⁰ ist gleich oder unterschiedlich und ist unabhängig ausgewählt aus der Gruppe, bestehend aus Alkyl, Alkenyl, Alkinyl, Cycloalkyl und Cycloalkenyl;
Ay ist Aryl;
Het ist eine 5- oder 6-gliedrige Heterocyclyl- oder Heteroaryl-Gruppe;
n ist 0, 1 oder 2;
R² ist ausgewählt aus der Gruppe, bestehend aus Het, -OR⁷, -S(O)ₙR⁹, -NR⁷R⁸, -NHHet und -NHR¹⁰Het;
Y ist N oder CH;
R³ und R⁴ sind gleich oder verschieden und werden jeweils unabhängig ausgewählt aus der Gruppe, bestehend aus H, Halogen, Alkyl, Alkenyl, Cycloalkyl, Ay, Het, -OR⁷, -OAy, -C(O)R⁷, C(O)Ay, -CO₂R⁷, -CO₂Ay, -SO₂NHR⁹, -NR⁷R⁸, -NR⁷Ay, -NHHet, -NHR¹⁰Het, -R¹⁰OR⁷, -R¹⁰OAy, -R¹⁰NR⁷R⁸ und -R¹⁰NR⁷Ay;
q ist 0, 1, 2, 3, 4 oder 5;
jeder R⁵ ist gleich oder unterschiedlich und wird unabhängig ausgewählt aus der Gruppe, bestehend aus Halogen, Alkyl, Alkenyl, Alkinyl, Cycloalkyl, Cycloalkenyl, Ay, Het, -OR⁷, -OAy, -OHet, -C(O)R⁹, -C(O)Ay, -C(O)Het, -C(O)NR⁷R⁸, -C(S)NR⁹R¹¹, -C(O)NR⁷Ay, -C(O)NHR¹⁰Het, -CO₂R⁹, -C(NH)NR⁷R⁸, -C(NH)NR⁷Ay, -S(O)ₙR⁹, -S(O)ₙAy, -S(O)ₙHet, -S(O)₂NR⁷R⁸, -S(O)₂NR⁷Ay, -NR⁷R⁸, -NR⁷Ay, -NHHet, -NHR¹⁰Ay, -NHR¹⁰Het, -R¹⁰-Cycloalkyl, -R¹⁰OR⁹, -R¹⁰C(O)R⁹, -R¹⁰CO₂R⁹, -R¹⁰C(O)NR⁹R¹¹, -R¹⁰C(S)NR⁹R¹¹, -R¹⁰C(NH)NR⁹R¹¹, -R¹⁰SO₂R⁹, -R¹⁰SO₂NR⁹R¹¹, -R¹⁰SO₂NHCOR⁹, -R¹⁰NR⁷R⁸, -R¹⁰NR⁷Ay, -R¹⁰NHC(NH)NR⁹R¹¹, Cyano, Azido und Nitro; oder
zwei benachbarte R⁵-Gruppen bilden zusammen mit den Atomen, an die sie gebunden sind ein C₅₋₆-Cycloalkyl oder Aryl;
r ist 1, 2 oder 3;
jeder R¹³ und R¹⁴ sind dieselben oder unterschiedlich und werden jeweils unabhängig ausgewählt aus der Gruppe, bestehend aus H, Alkyl, Alkenyl, Cycloalkyl, Cycloalkenyl, Ay, Het, -C(O)R⁹, -CO₂R⁹, -C(O)NR⁹R¹¹, -C(S)NR⁹R¹¹, -C(NH)NR⁹R¹¹, -SO₂R¹⁰, -SO₂NR⁹R¹¹, -R¹⁰-Cycloalkyl, -R¹⁰OR⁹, -R¹⁰C(O)R⁹, -R¹⁰CO₂R⁹, -R¹⁰C(O)NR⁹R¹¹, -R¹⁰C(S)NR⁹R¹¹, -R¹⁰C(NH)NR⁹R¹¹, -R¹⁰SO₂R¹⁰, -R¹⁰SO₂NR⁹R¹¹, -R¹⁰NR⁹R¹¹, -R¹⁰NHCOR⁹, -R¹⁰NHC(NH)NR⁹R¹¹, -R¹⁰NHSO₂R⁹ und -R¹⁰SO₂NHCOR⁹;
p ist 0, 1 oder 2, worin p + r ≤ 3 und
jeder R⁶ ist derselbe oder unterschiedlich und wird unabhängig ausgewählt aus der Gruppe, bestehend aus Halogen, Alkyl, Het, -OR⁷, -C(O)NR⁷R⁸, -S(O)ₙR⁹, -S(O)₂NR⁷R⁸ und Cyano; oder
zwei benachbarte R⁶-Gruppen bilden zusammen mit den Atomen, an die sie gebunden sind eine C₅₋₆-Cycloalkyl- oder 5- oder 6-gliedrige Heterocyclyl-Gruppe, enthaltend 1 oder 2 Heteroatome;
worin, wenn Y CH ist, R³ nicht -NR⁷Ay ist;
und pharmazeutisch annehmbare Salze und Solvate davon.

2. Verbindung gemäß Anspruch 1, worin R¹ ausgewählt ist aus der Gruppe, bestehend aus Alkyl, -OR⁷, -C(O)NR⁷R⁸ und S(O)ₙR⁹.

3. Verbindung gemäß einem der Ansprüche 1 bis 2, worin R² ausgewählt ist aus der Gruppe, bestehend aus Het, -NR⁷R⁸ und NHHet.

4. Verbindung gemäß einem der Ansprüche 1 bis 3, worin Y N ist.

5. Verbindung gemäß einem der Ansprüche 1 bis 3, worin Y CH ist.

6. Verbindung gemäß einem der Ansprüche 1 bis 5, worin R³ und R⁴ dieselben oder unterschiedlich sind und jeweils unabhängig ausgewählt werden aus der Gruppe, bestehend aus H, Halogen, Alkyl, Ay, -OR⁷, -CO₂R⁷, -NR⁷R⁸, -R¹⁰OR⁷ und -R¹⁰NR⁷R⁸.

7. Verbindung gemäß einem der Ansprüche 1 bis 5, worin R³ und R⁴ jeweils H sind.

8. Verbindung gemäß einem der Ansprüche 1 bis 7, worin q 0, 1 oder 2 ist.

9. Verbindung gemäß einem der Ansprüche 1 bis 8, worin jeder R⁵ derselbe oder unterschiedlich ist und unabhängig ausgewählt ist aus der Gruppe, bestehend aus Halogen, Alkyl, Alkenyl, Ay, Het, -OR⁷, -OAy, -CO₂R⁹, -C(O)NR⁷R⁸, -C(O)NR⁷Ay, -S(O)₂NR⁷R⁸, -NR⁷R⁸, -NR⁷Ay, -NHR¹⁰Ay, Cyano, Nitro und Azido.

10. Verbindung gemäß einem der Ansprüche 1 bis 8, worin jeder R⁵ derselbe oder unterschiedlich ist und unabhängig ausgewählt wird aus der Gruppe, bestehend aus Halogen, Alkyl, -OR⁷, -NR⁷R⁸ und Cyano.

11. Verbindung gemäß einem der Ansprüche 1 bis 10, worin p 0 oder 1 ist.

12. Verbindung gemäß einem der Ansprüche 1 bis 11, worin r 1 ist.

13. Verbindung gemäß einem der Ansprüche 1 bis 12, worin jeder R¹³ und R¹⁴ derselbe oder unterschiedlich ist und jeweils unabhängig ausgewählt wird aus der Gruppe, bestehend aus H, Alkyl, Cycloalkyl, -C(O)R⁹, -CO₂R⁹, -R¹⁰-Cycloalkyl, -R¹⁰OR⁹ und -R¹⁰CO₂R⁹, -R¹⁰NR⁹R¹¹.

14. Verbindung gemäß einem der Ansprüche 1 bis 12, worin jeder R¹³ und R¹⁴ derselbe oder unterschiedlich ist, und jeweils unabhängig ausgewählt ist aus der Gruppe, bestehend aus H, Alkyl und Cycloalkyl.

15. Verbindung, ausgewählt aus der Gruppe, bestehend aus:
N-Cyclopentyl-3-[2-(cyclopentylamino)-4-pyrimidinyl]-7-(ethylsulfanyl)-2-(4-fluorphenyl)pyrazololo[1,5-a]pyridin-5-amin;
N-Cyclopentyl-3-[2-(cyclopentylamino)-4-pyrimidinyl]-2-(4-fluorphenyl)-7-methylpyrazolo[1,5-a]pyridin-5-amin;
N-Cyclopentyl-3-[2-(cyclopentylamino)-4-pyrimidinyl]-7-ethoxy-2-(4-fluorphenyl)pyrazolo[1,5-a]pyridin-5-amin;
N-Cyclopentyl-3-[2-(cyclopentylamino)-4-pyrimidinyl]-2-(4-fluorphenyl)-7-(methylsulfanyl)-pyrazolo[1,5-a]pyridin-5-amin;
N-Cyclopentyl-3-[2-(cyclopentylamino)-4-pyrimidinyl]-2-(4-fluorphenyl)-7-(2-methoxyethoxy)-pyrazolo[1,5-a]pyridin-5-amin;
N-Cyclopentyl-3-[2-(cyclopentylamino)-4-pyrimidinyl]-2-(4-methoxyphenyl)-7-(methylsulfanyl)-pyrazolo[1,5-a]pyridin-5-amin;
N-Cyclopentyl-3-[2-(cyclopentylamino)-4-pyrimidinyl]-7-(ethylsulfanyl)-2-(4-methoxyphenyl)-pyrazolo[1,5-a]pyridin-5-amin;
N-Cyclopentyl-3-[2-cyclopentylamino)-4-pyrimidinyl]-7-(isopropylsulfanyl)-2-(4-methoxyphenyl)pyrazolo[1,5-a]pyridin-5-amin;
N-Cyclopentyl-3-[2-(cyclopentylamino)-4-pyrimidinyl]-2-(4-methoxyphenyl)-7-(phenylsulfanyl)-pyrazolo[1,5-a]pyridin-5-amin und
N-Cyclopentyl-3-[2-(cyclopentylamino)-4-pyrimidinyl]-2-(4-fluorphenyl)-7-(isopropylsulfanyl)-pyrazolo[1,5-a]pyridin-5-amin; und
pharmazeutisch annehmbaren Salzen und Solvaten davon.

16. Pharmazeutische Zusammensetzung, umfassend eine Verbindung gemäß einem der Ansprüche 1 bis 15.

17. Pharmazeutische Zusammensetzung gemäß Anspruch 16, weiterhin umfassend einen pharmazeutisch annehmbaren Träger oder ein Verdünnungsmittel.

18. Pharmazeutische Zusammensetzung gemäß Anspruch 16, weiterhin umfassend ein antivirales Mittel, ausgewählt aus der Gruppe, bestehend aus Acyclovir, Valacyclovir und pharmazeutisch annehmbaren Salzen davon.

19. Verbindung gemäß einem der Ansprüche 1 bis 15 zur Verwendung in der Therapie.

20. Verbindung gemäß einem der Ansprüche 1 bis 15 zur Verwendung in der Prophylaxe oder Behandlung einer viralen Herpesinfektion bei einem Tier.

21. Verbindung gemäß einem der Ansprüche 1 bis 15 zur Verwendung bei der Prophylaxe oder Behandlung eines Zustands oder einer Erkrankung, die mit einer viralen Herpesinfektion bei einem Tier assoziiert ist.

22. Verwendung einer Verbindung gemäß einem der Ansprüche 1 bis 15 für die Herstellung eines Medikaments für die Prophylaxe oder Behandlung einer viralen Herpesinfektion bei einem Tier.

23. Verwendung gemäß Anspruch 22, wobei die virale Herpesinfektion ausgewählt ist aus der Gruppe, bestehend aus Herpes simplex Virus 1, Herpes simplex Virus 2, Cytomegalievirus, Epstein-Barr-Virus, Herpes zoster Virus, menschliches Herpesvirus 6, menschliches Herpesvirus 7 und menschliches Herpesvirus 8.

24. Verwendung einer Verbindung gemäß einem der Ansprüche 1 bis 15 für die Herstellung eines Medikaments für die Prophylaxe oder Behandlung eines Zustands oder einer Erkrankung, die mit einer viralen Herpesinfektion bei einem Tier assoziiert sind.

25. Pharmazeutische Zusammensetzung, umfassend eine Verbindung gemäß einem der Ansprüche 1 bis 15 zur Verwendung bei der Herstellung eines Medikaments für die Prophylaxe oder Behandlung einer viralen Herpesinfektion bei einem Tier.

26. Verfahren zur Herstellung einer Verbindung gemäß einem der Ansprüche 1 bis 15, worin Y N ist; R² ausgewählt ist aus der Gruppe, bestehend aus Het, -OR⁷, -S(O)ₙR⁹, -NR⁷R⁸, -NHHet und -NHR¹⁰Het, umfassend die folgenden Schritte:
a) Umsetzen einer Verbindung der Formel (XI): 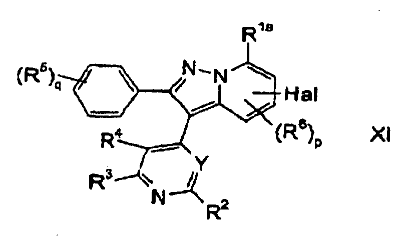 worin Hal Halogen ist; und
R^{1a} ausgewählt ist aus der Gruppe, bestehend aus H, Alkyl, Cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰-Cycloalkyl, -R¹⁰OR⁹, -R¹⁰NR⁷R⁸, Cyano, Nitro und Azido;
mit einem Amin oder Imin zur Herstellung einer Verbindung der Formel (XII): 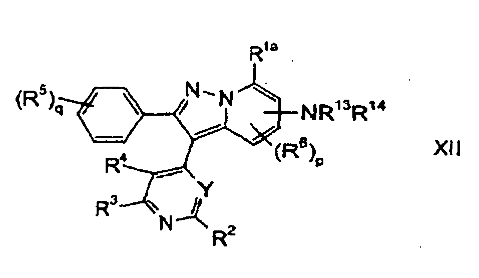 und
b) in der Ausführungsform, in der R^{1a} H ist, Umwandlung der Verbindung der Formel (XII) in eine Verbindung der Formel (I).

27. Verfahren zur Herstellung einer Verbindung gemäß einem der Ansprüche 1 bis 15, worin Y N ist; R² ausgewählt ist aus der Gruppe, bestehend aus Het, -OR⁷, -S(O)ₙR⁹,
- NR⁷R⁸, -NHHet und -NHR¹⁰Het; R³ ist H und R⁴ ist H,
wobei das Verfahren die folgenden Schritte umfaßt:
a) Umsetzen einer Verbindung der Formel (IX): 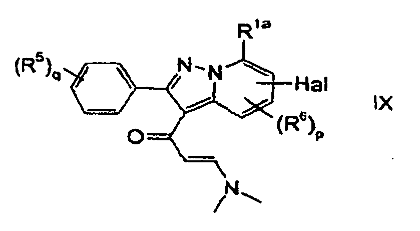 worin Hal Halogen ist; und
R^{1a} ausgewählt ist aus der Gruppe, bestehend aus H, - Alkyl, Cycloalkyl, -OR⁷, -OAy, -C(O)R⁹, -CO₂R⁹, -C(0)NR⁷R⁸, -S(O)ₙR⁹, -S(O)ₙAy, -S(O)ₙHet, -S(O)₂NR⁷R⁸, -R¹⁰-Cycloalkyl, -R¹⁰OR⁹, -R¹⁰NR⁷R⁸, Cyano, Nitro und Azido;
mit einer Verbindung der Formel (X): 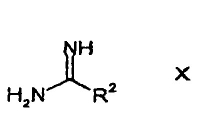 zur Herstellung einer Verbindung der Formel (XI): 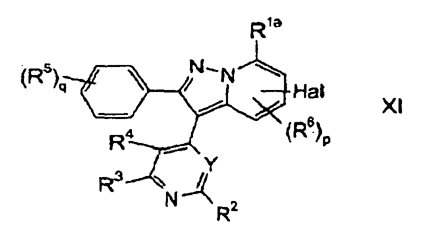
b) Umsetzen der Verbindung der Formel (XI) mit einem Amin oder Imin zur Herstellung einer Verbindung der Formel (XII): 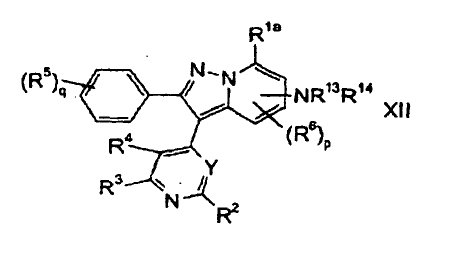 und
c) in der Ausführungsform, in der R^{1a} H ist, Umwandlung der Verbindung der Formel (XII) in eine Verbindung der Formel (I).

28. Verfahren zur Herstellung einer Verbindung gemäß einem der Ansprüche 1 bis 15, worin Y N ist, R² ausgewählt ist aus der Gruppe, bestehend aus Het, -OR⁷, -S(O)ₙR⁹, -NR⁷R⁸, -NHHet und -NHR¹⁰Het; R³ ausgewählt ist aus der Gruppe, bestehend aus H, Alkyl, Alkenyl, Cycloalkyl, Ay, Het, -C(O)R⁷, -C(O)Ay, -CO₂R⁷, -CO₂Ay, -SO₂NHR⁹, -NR⁷R⁸, worin R⁷ und R⁸ nicht H sind, -NR⁷Ay ist, worin R⁷ nicht H ist; -R¹⁰OR⁷, -R¹⁰OAy, -R¹⁰NR⁷R⁸ und -R¹⁰NR⁷Ay; und R⁴ H ist;
wobei das Verfahren die folgenden Schritte umfaßt:
a) Umsetzen einer Verbindung der Formel (XXIX): 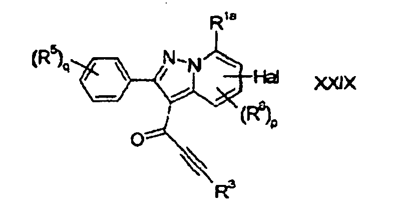 worin Hal Halogen ist und
R^{1a} ausgewählt ist aus der Gruppe, bestehend aus H, Alkyl, Cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰-Cycloalkyl, -R¹⁰OR⁹, -R¹⁰NR⁷R⁸, Cyano, Nitro und Azido;
mit einer Verbindung der Formel (X): 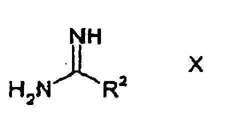 zur Herstellung einer Verbindung der Formel (XI): 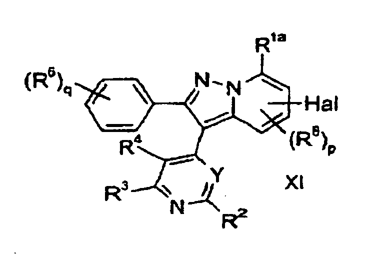
b) Umsetzen der Verbindung der Formel (XI) mit einem Amin oder Imin zur Herstellung einer Verbindung der Formel (XII): 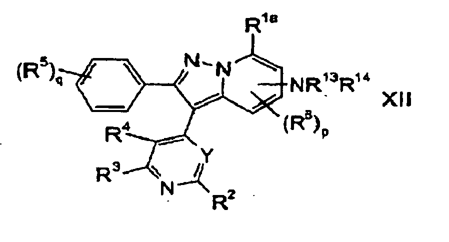 und
c) in der Ausführungsform, in der R^{1a} H ist, Umwandlung der Verbindung der Formel (XII) in eine Verbindung der Formel (I).

29. Verfahren zur Herstellung einer Verbindung gemäß einem der Ansprüche 1 bis 15, worin Y N ist, und R² ausgewählt ist aus der Gruppe, bestehend aus Het, -OR⁷, -S(O)ₙR⁹, -NR⁷R⁸, -NHHet und -NHR¹⁰Het, wobei das Verfahren die folgenden Schritte umfaßt:
a) Umsetzen einer Verbindung der Formel (XXXV): 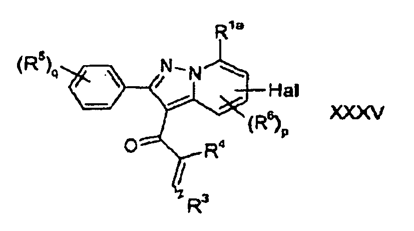 worin Hal Halogen ist; und
R^{1a} ausgewählt ist aus der Gruppe, bestehend aus H, Alkyl, Cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)_{2N}R⁷R⁸, -R¹⁰-Cycloalkyl, -R¹⁰OR⁹, -R¹⁰NR⁷R⁸, Cyano, Nitro und Azido;
mit einer Verbindung der Formel (X): 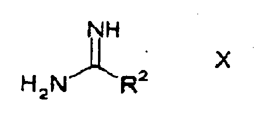 gefolgt von einer oxidativen Aromatisierung zur Herstellung einer Verbindung der Formel (XI): 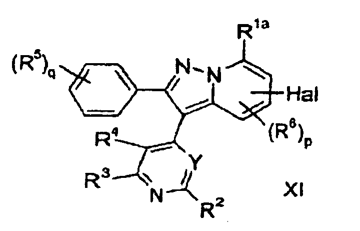
b) Umsetzen der Verbindung der Formel (XI) mit einem Amin oder Imin zur Herstellung einer Verbindung der Formel (XII): 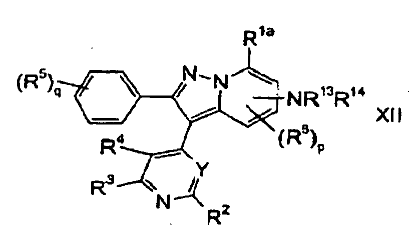 und
c) in der Ausführungsform, in der R^{1a} H ist, Umwandlung der Verbindung der Formel (XII) in eine Verbindung der Formel (I).

30. Verfahren zur Herstellung einer Verbindung gemäß einem der Ansprüche 1 bis 15, wobei das Verfahren die folgenden Schritte umfaßt:
a) Umsetzen einer Verbindung der Formel (XL): 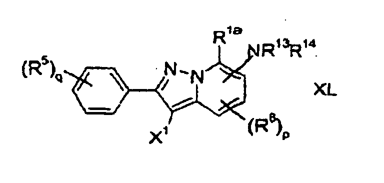 worin X¹ Halogen ist; und
worin R^{1a} ausgewählt ist aus der Gruppe, bestehend aus H, Alkyl, Cycloalkyl, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰-Cycloalkyl, -R¹⁰OR⁹, -R¹⁰NR⁷R⁸, Cyano, Nitro und Azido;
mit einer Verbindung der Formel (XLI): 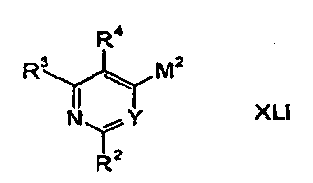 worin M² ausgewählt ist aus der Gruppe, bestehend aus -B(OH)₂,-B(ORa)₂, -B(Ra)₂, -Sn(Ra)₃, Zn-Halogenid, ZnRa, Mg-Halogenid, worin Ra Alkyl oder Cycloalkyl ist und Halogenid ist Halogen;
zur Herstellung einer Verbindung der Formel (XII): 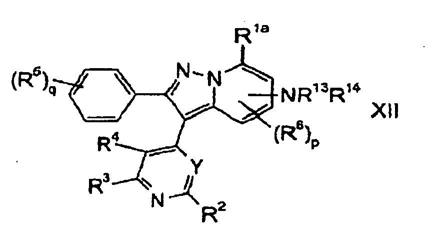 und
c) in der Ausführungsform, in der R^{1a} H ist, Umwandlung der Verbindung der Formel (XII) in eine Verbindung der Formel (I).

31. Verfahren gemäß einem der Ansprüche 26 bis 30, weiterhin umfassend den Schritt der Umwandlung einer Verbindung der Formel (XII) in eine Verbindung der Formel (XII-D): 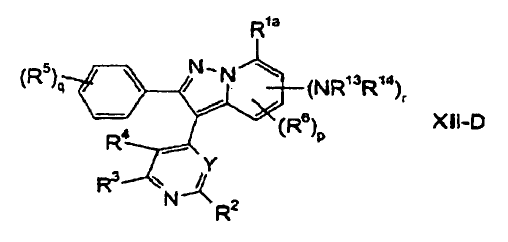 worin r 2 oder 3 ist.

32. Verfahren gemäß einem der Ansprüche 26 bis 31, weiterhin umfassend den Schritt der Umwandlung einer Verbindung der Formel (I) in ein pharmazeutisch annehmbares Salz oder Solvat davon.

33. Verfahren gemäß einem der Ansprüche 26 bis 32, weiterhin umfassend den Schritt der Umwandlung einer Verbindung der Formel (I) oder eines pharmazeutisch annehmbaren Salzes oder Solvats oder physiologischen Derivats davon in eine andere Verbindung der Formel (I) oder ein pharmazeutisch annehmbares Salz oder Solvat davon.

## Revendications

1. Composé de formule (I) : 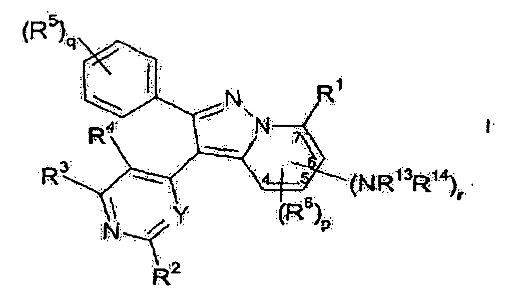 dans laquelle:
R¹ est choisi dans le groupe consistant en alkyle, cycloalkyle, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyle, -R¹⁰OR⁹, -R¹⁰NR⁷R⁸, cyano, nitro et azido ;
chacun des R⁷ et R⁸ sont identiques ou différents et sont chacun choisis indépendamment dans le groupe consistant en H, alkyle, alcényle, cycloalkyle, cycloalcényle, -OR⁹, -C(O)R⁹, -CO₂R⁹, -C(O)NR⁹R¹¹, -C(S)NR⁹R¹¹, -C(NH)NR⁹R¹¹, -SO₂R¹⁰, -SO₂NR⁹R¹¹, -R¹⁰cycloalkyle, -R¹⁰OR⁹, -R¹⁰C(O)R⁹, -R¹⁰CO₂R⁹, -R¹⁰C(O)NR⁹R¹¹, -R¹⁰C(S)NR⁹R¹¹, -R¹⁰C(NH)NR⁹R¹¹, -R¹⁰SO₂R¹⁰, -R¹⁰SO₂NR⁹R¹¹, -R¹⁰NR⁹R¹¹, -R¹⁰NHCOR⁹, -R¹⁰NHC (NH)NR⁹R¹¹, -R¹⁰NHSO₂R⁹ et -R¹⁰SO₂NHCOR⁹;
chacun des R⁹ et R¹¹ sont identiques ou différents et sont chacun choisis indépendamment dans le groupe consistant en H, alkyle, cycloalkyle, -R¹⁰cycloalkyle, -R¹⁰OH, -R¹⁰(OR¹⁰)_{w} où w est 1 à 10, et -R¹⁰NR¹⁰R¹⁰ ;
chaque R¹⁰ est identique ou différent et est choisi indépendamment dans le groupe consistant en alkyle, alcényle, alcynyle, cycloalkyle et cycloalcényle ;
Ay est aryle ; Het est un groupe hétérocyclique ou hétéroaryle à 5 ou 6 chaînons ;
n est 0, 1 ou 2 ;
R² est choisi dans le groupe consistant en Het, -OR⁷, -S(O)ₙR⁹, -NR⁷R⁸, -NHHet et -NHR¹⁰Het ;
Y est N ou CH ;
R³ et R⁴ sont identiques ou différents et sont chacun choisis indépendamment dans le groupe consistant en H, halo, alkyle, alcényle, cycloalkyle, Ay, Het, -OR⁷, -OAy, -C(O)R⁷, -C(O)Ay, -CO₂R⁷, -CO₂Ay, -SO₂NHR⁹, -NR⁷R⁸, -NR⁷Ay, -NHHet, -NHR¹⁰Het, -R¹⁰OR⁷, -R¹⁰OAy, -R¹⁰NR⁷R⁸ et -R¹⁰NR⁷Ay;
q est 0, 1, 2, 3, 4 ou 5 ;
chaque R⁵ est identique ou différent et est choisi indépendamment dans le groupe consistant en halo, alkyle, alcényle, alcynyle, cycloalkyle, cycloalcényle, Ay, Het, -OR⁷, -OAy, -OHet, -C(O)R⁹, -C(O)Ay, -C(O)Het, -C(O)NR⁷R⁸, -C(S)NR⁹R¹¹, -C(O)NR⁷Ay, -C(O)NHR¹⁰Het, -CO₂R⁹, -C(NH)NR⁷R⁸, -C(NH)NR⁷Ay, -S(O)ₙR⁹, -S(O)ₙAy, -S(O)ₙHet, -S(O)₂NR⁷R⁸, -S(O)₂NR⁷Ay, -NR⁷R⁸, -NR⁷Ay, -NHHet, -NHR¹⁰Ay, -NHR¹⁰Het, -R¹⁰cycloalkyle, -R¹⁰OR⁹, -R¹⁰C(O)R⁹, -R¹⁰CO₂R⁹, -R¹⁰C(O)NR⁹R¹¹, -R¹⁰C(S)NR⁹R¹¹, -R¹⁰C(NH)NR⁹R¹¹, -R¹⁰SO₂R⁹, -R¹⁰SO₂NR⁹R¹¹, -R¹⁰SO₂NHCOR⁹, -R¹⁰NR⁷R⁸, -R¹⁰NR⁷Ay, -R¹⁰NHC(NH)NR⁹R¹¹, cyano, azido et nitro ; ou bien deux groupes R⁵ adjacents forment ensemble avec les atomes auxquels ils sont liés un groupe cycloalkyle en C₅₋₆ ou aryle ;
r est 1, 2 ou 3 ;
chacun des R¹³ et R¹⁴ sont identiques ou différents et sont chacun choisis indépendamment dans le groupe consistant en H, alkyle, alcényle, cycloalkyle, cycloalcényle, Ay, Het, -C(O)R⁹, -CO₂R⁹, -C(O)NR⁹R¹¹, -C(S)NR⁹R¹¹, -C(NH)NR⁹R¹¹, -SO₂R¹⁰, -SO₂NR⁹R¹¹, -R¹⁰cycloalkyle, -R¹⁰OR⁹, -R¹⁰C(O)R⁹, -R¹⁰CO₂R⁹, -R¹⁰C(O)NR⁹R¹¹, -R¹⁰C(S)NR⁹R¹¹, -R¹⁰C(NH)NR⁹R¹¹, -R¹⁰SO₂R¹⁰, -R¹⁰SO₂NR⁹R¹¹, -R¹⁰NR⁹R¹¹, -R¹⁰NHCOR⁹, -R¹⁰NHC(NH)NR⁹R¹¹, -R¹⁰NHSO₂R⁹ et -R¹⁰SO₂NHCOR⁹;
p est 0, 1 ou 2, où p + r ≤ 3; et
chaque R⁶ est identique ou différent et est choisi indépendamment dans le groupe consistant en halo, alkyle, Het, -OR⁷, -C(O)NR⁷R⁸, -S(O)ₙR⁹, -SO₂NR⁷R⁸ et cyano ; ou bien deux groupes R⁶ adjacents forment ensemble avec les atomes auxquels ils sont liés un groupe cycloalkyle en C₅₋₆ ou un groupe hétérocyclique de 5 à 6 chaînons contenant 1 ou 2 hétéroatomes ;
dans laquelle lorsque Y est CH, R³ n'est pas -NR⁷Ay ;
et sels et solvates de ceux-ci acceptables du point de vue pharmaceutique.

2. Composé selon la revendication 1, dans lequel R¹ est choisi dans le groupe consistant en alkyle, -OR⁷, -C(O)NR⁷R⁸ et -S(O)ₙR⁹.

3. Composé selon l'une quelconque des revendications 1 et 2, dans lequel R² est choisi dans le groupe consistant en Het -NR⁷R⁸ et NHHet.

4. Composé selon l'une quelconque des revendications 1 à 3, dans lequel Y est N.

5. Composé selon l'une quelconque des revendications 1 à 3, dans lequel Y est CH.

6. Composé selon l'une quelconque des revendications 1 à 5, dans lequel R³ et R⁴ sont identiques ou différents et sont chacun choisi indépendamment dans le groupe consistant en H, halo, alkyle, Ay, -OR⁷, -CO₂R⁷, -NR⁷R⁸, -R¹⁰OR⁷ et -R¹⁰NR⁷R⁸.

7. Composé selon l'une quelconque des revendications 1 à 5, dans lequel R³ et R⁴ sont chacun H.

8. Composé selon l'une quelconque des revendications 1 à 7, dans lequel q est 0, 1 ou 2.

9. Composé selon l'une quelconque des revendications 1 à 8, dans lequel chaque R⁵ est identique ou différent et est choisi indépendamment dans le groupe consistant en halo, alkyle, alcényle, Ay, Het, -OR⁷, -OAy, -CO₂R⁹, -C(O)NR⁷R⁸, -C(O)NR⁷Ay, -S(O)₂NR⁷R⁸, -NR⁷R⁸, -NR⁷Ay, -NHR¹⁰Ay, cyano, nitro et azido.

10. Composé selon l'une quelconque des revendications 1 à 8, dans lequel chaque R⁵ est identique ou différent et est choisi indépendamment dans le groupe consistant en halo, alkyle, -OR⁷, -NR⁷R⁸ et cyano.

11. Composé selon l'une quelconque des revendications 1 à 10, dans lequel p est 0 ou 1.

12. Composé selon l'une quelconque des revendications 1 à 11, dans lequel r est 1.

13. Composé selon l'une quelconque des revendications 1 à 12, dans lequel chacun des R¹³ et R¹⁴ sont identiques ou différents et sont chacun choisis indépendamment dans le groupe consistant en H, alkyle, cycloalkyle, -C(O)R⁹, -CO₂R⁹, -R¹⁰cycloalkyle, -R¹⁰OR⁹ et -R¹⁰CO₂R⁹, -R¹⁰NR⁹R¹¹.

14. Composé selon l'une quelconque des revendications 1 à 12, dans lequel chacun des R¹³ et R¹⁴ sont identiques ou différents et sont chacun choisis indépendamment dans le groupe consistant en H, alkyle et cycloalkyle.

15. Composé choisi dans le groupe consistant en :
*N*-cyclopentyl-3-[2-(cyclopentylamino)-4-pyrimidinyl]-7-(éthylsulfanyl)-2-(4-fluorophényl)pyrazolo[1,5-a]pyridin-5-amine ;
*N*-cyclopentyl-3-[2-(cyclopentylamino)-4-pyrimidinyl]-2-(4-fluorophényl)-7-méthylpyrazolo[1,5-a]pyridin-5-amine ;
*N*-cyclopentyl-3-[2-(cyclopentylamino)-4-pyrimidinyl]-7-éthoxy-2-(4-fluorophényl)pyrazolo[1,5-*a*]pyridin-5-amine ;
*N*-cyclopentyl-3-[2-(cyclopentylamino)-4-pyrimidinyl]-2-(4-fluorophényl)-7-(méthylsulfanyl)pyrazolo[1,5-*a*]pyridin-5-amine ;
*N*-cyclopentyl-3-[2-(cyclopentylamino)-4-pyrimidinyl]-2-(4-fluorophényl)-7-(2-méthoxyéthoxy)pyrazolo[1,5-*a*]pyridin-5-amine ;
*N*-cyclopentyl-3-[2-(cyclopentylamino)-4-pyrimidinyl]-2-(4-méthoxyphényl)-7-(méthylsulfanyl)pyrazolo[1,5-*a*]pyridin-5-amine ;
*N*-cyclopentyl-3-[2-(cyclopentylamino)-4-pyrimidinyl]-7-(éthylsulfanyl)-2-(4-méthoxyphényl)pyrazolo[1,5-*a*]pyridin-5-amine ;
*N*-cyclopentyl-3-[2-(cyclopentylamino)-4-pyrimidinyl]-7-(isopropylsulfanyl)-2-(4-méthoxyphényl)pyrazolo[1,5-*a*]pyridin-5-amine ;
*N*-cyclopentyl-3-[2-(cyclopentylamino)-4-pyrimidinyl]-2-(4-méthoxyphényl)-7-(phénylsulfanyl)lpyrazolo[1,5-*a*]pyridin-5-amine ; et
*N*-cyclopentyl-3-[2-(cyclopentylamino)-4-pyrimidinyl]-2-(4-fluorophényl)-7-(isopropylsulfanyl)lpyrazolo[1,5-*a*]pyridin-5-amine ; et
sels et solvates de ceux-ci acceptables du point de vue pharmaceutique.

16. Composition pharmaceutique comprenant un composé selon l'une quelconque des revendications 1 à 15.

17. Composition pharmaceutique selon la revendication 16 comprenant de plus un support ou diluant acceptable du point de vue pharmaceutique.

18. Composition pharmaceutique selon la revendication 16 comprenant de plus un agent antiviral choisi dans le groupe consistant en acyclovir, valacyclovir et sels de ceux-ci acceptables du point de vue pharmaceutique.

19. Composé selon l'une quelconque des revendications 1 à 15 utile en thérapie.

20. Composé selon l'une quelconque des revendications 1 à 15 utile dans la prophylaxie ou le traitement d'une infection virale herpétique chez un animal.

21. Composé selon l'une quelconque des revendications 1 à 15 utile dans la prophylaxie ou le traitement d'états ou de maladies associés à une infection virale herpétique chez un animal.

22. Utilisation d'un composé selon l'une quelconque des revendications 1 à 15 pour la préparation d'un médicament destiné à la prophylaxie ou au traitement d'une infection virale herpétique chez un animal.

23. Utilisation selon la revendication 22, dans laquelle ladite infection virale herpétique est choisie dans le groupe consistant en Herpès simplex virus de type 1, Herpès simplex virus de type 2, Cytomégalovirus, virus Epstein-Barr, virus varicelle-zona, Herpèsvirus humain de type 6, Herpès virus humain de type 7 et Herpèsvirus humain de type 8.

24. Utilisation d'un composé selon l'une quelconque des revendications 1 à 15 pour la préparation d'un médicament destiné à la prophylaxie ou au traitement d'états ou de maladies associés à une infection virale herpétique chez un animal.

25. Composition pharmaceutique comprenant un composé selon l'une quelconque des revendications 1 à 15 utile dans la préparation d'un médicament destiné à la prophylaxie ou au traitement d'une infection virale herpétique chez un animal.

26. Procédé de préparation d'un composé selon l'une quelconque des revendications 1 à 15, dans lequel Y est N, R² est choisi dans le groupe consistant en Het, -OR⁷, -S(O)ₙR⁹, -NR⁷R⁸, -NHHet et -NHR¹⁰Het,
comprenant les étapes de :
a) réaction d'un composé de formule (XI) : 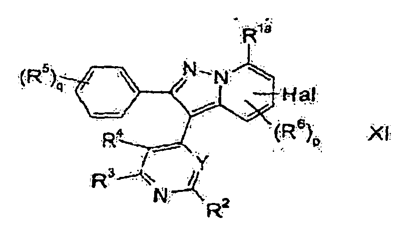 dans laquelle :
Hal est halo et R^{1a} est choisi dans le groupe consistant en H, alkyle, cycloalkyle, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyle, -R¹⁰OR⁹, -R¹⁰NR⁷R⁸, cyano, nitro et azido ;
avec une amine ou une imine pour préparer un composé de formule (XII) : 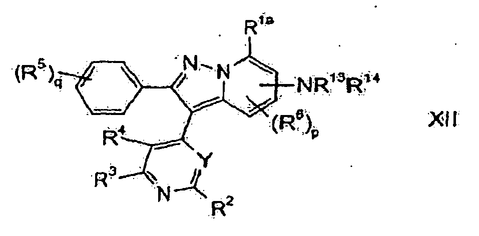 et
b) dans le mode de réalisation où R^{1a} est H, conversion du composé de formule (XII) en un composé de formule (I).

27. Procédé de préparation d'un composé selon l'une quelconque des revendications 1 à 15, dans lequel Y est N ; R² est choisi dans le groupe consistant en Het, -OR⁷, -S(O)ₙR⁹, -NR⁷R⁸, -NHHet et -NHR¹⁰Het; R³ est H et R⁴ est H,
ledit procédé comprenant les étapes de :
a) réaction d'un composé de formule (IX) 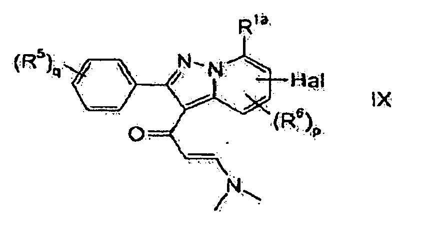 dans laquelle :
Hal est halo ; et R^{1a} est choisi dans le groupe consistant en H, alkyle, cycloalkyle, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyle, -R¹⁰OR⁹, -R¹⁰NR⁷R⁸, cyano, nitro et azido ;
avec un composé de formule (X) : 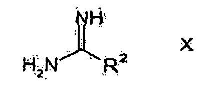 pour préparer un composé de formule (XI): 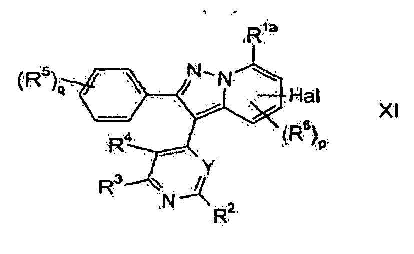
b) réaction du composé de formule (XI) avec une amine ou une imine pour préparer un composé de formule (XII) : 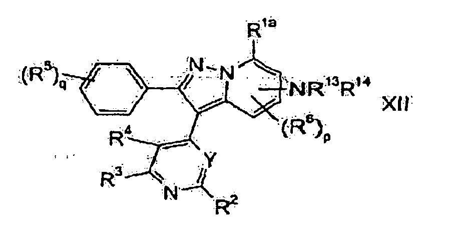 et
c) dans le mode de réalisation où R^{1a} est H, conversion du composé de formule (XII) en un composé de formule (I).

28. Procédé de préparation d'un composé selon l'une quelconque des revendications 1 à 15, dans lequel Y est N ; R² est choisi dans le groupe consistant en Het, -OR⁷, -S(O)ₙR⁹, -NR⁷R⁸, -NHHet et -NHR¹⁰Het ; R³ est choisi dans le groupe consistant en H, alkyle, alcényle, cycloalkyle, Ay, Het, -C(O)R⁷, -C(O)Ay, -CO₂R⁷, -CO₂Ay, -SO₂NHR⁹, -NR⁷R⁸ où R⁷ et R⁸ ne sont pas H, -NR⁷Ay où R⁷ n'est pas H, -R¹⁰OR⁷, -R¹⁰OAy, -R¹⁰NR⁷R⁸ et -R¹⁰NR⁷Ay ; et R⁴ est H,
ledit procédé comprenant les étapes de :
a) réaction d'un composé de formule (XXIX) 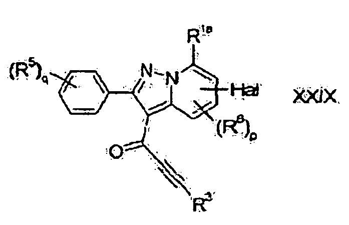 dans laquelle :
Hal est halo ; et R^{1a} est choisi dans le groupe consistant en H, alkyle, cycloalkyle, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyle, -R¹⁰OR⁹, -R¹⁰NR⁷R⁸, cyano, nitro et azido ;
avec un composé de formule (X) : 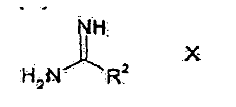 pour préparer un composé de formule (XI) : 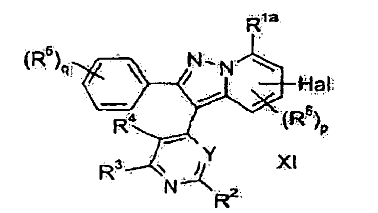
b) réaction du composé de formule (XI) avec une amine ou une imine pour préparer un composé de formule (XII) : 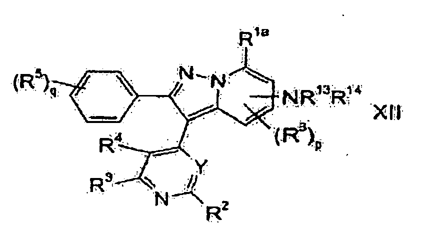 et
c) dans le mode de réalisation où R^{1a} est H, conversion du composé de formule (XII) en un composé de formule (I).

29. Procédé de préparation d'un composé selon l'une quelconque des revendications 1 à 15, dans lequel Y est N ; R² est choisi dans le groupe consistant en Het, -OR⁷, -S(O)ₙR⁹, -NR⁷R⁸, -NHHet et -NHR¹⁰Het ;
ledit procédé comprenant les étapes de :
a) réaction d'un composé de formule (XXXV) 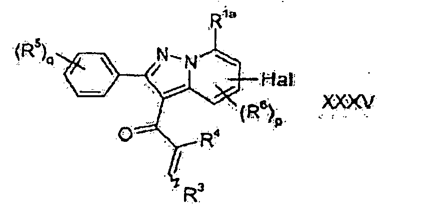 dans laquelle :
Hal est halo ; et R^{1a} est choisi dans le groupe consistant en H, alkyle, cycloalkyle, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰cycloalkyle, -R¹⁰OR⁹, -R¹⁰NR⁷R⁸, cyano, nitro et azido ;
avec un composé de formule (X) : 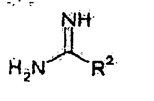 suivie d'une aromatisation, pour préparer un composé de formule (XI) : 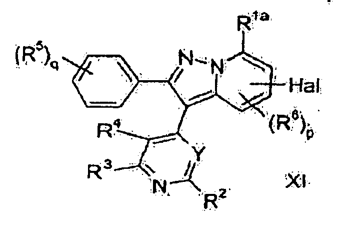
b) réaction du composé de formule (XI) avec une amine ou une imine pour préparer un composé de formule (XII): 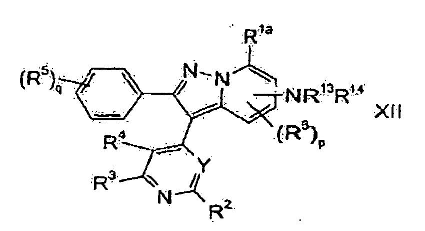 et
c) dans le mode de réalisation où R^{1a} est H, conversion du composé de formule (XII) en un composé de formule (I).

30. Procédé de préparation d'un composé selon l'une quelconque des revendications 1 à 15,
ledit procédé comprenant les étapes de :
a) réaction d'un composé de formule (XL) 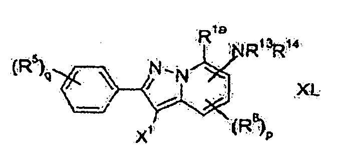 dans laquelle :
X¹ est halo ; et dans laquelle R^{1a} est choisi dans le groupe consistant en H, alkyle, cycloalkyle, -OR⁷, -OAy, -C(O)R⁹, -CO₂R⁹, -C(O)NR⁷R⁸, -S(O)ₙR⁹, -S(O)ₙAy, -S(O)ₙHet, -S(O)₂NR⁷R⁸, -R¹⁰ cycloalkyle, -R¹⁰OR⁹, -R¹⁰NR⁷R⁸, cyano, nitro et azido ;
avec un composé de formule (XLI) : 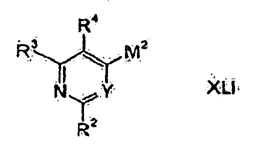 dans laquelle :
M² est choisi dans le groupe consistant en -B(OH)₂, -B(ORa)₂, -B(Ra)₂, -Sn(Ra)₃, halogénure de Zn, ZnRa, halogénure de Mg, où Ra est un groupe alkyle ou cycloalkyle et halogénure est un groupe halo ; pour préparer un composé de formule (XII) : 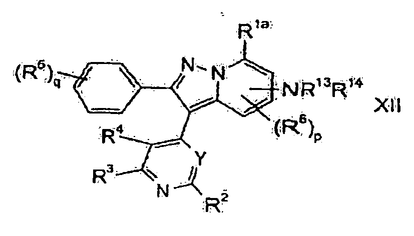 et
c) dans le mode de réalisation où R^{1a} est H, conversion du composé de formule (XII) en un composé de formule (1).

31. Procédé selon l'une quelconque des revendications 26 à 30 comprenant de plus l'étape de conversion d'un composé de formule (XII) en un composé de formule (XII-D) : 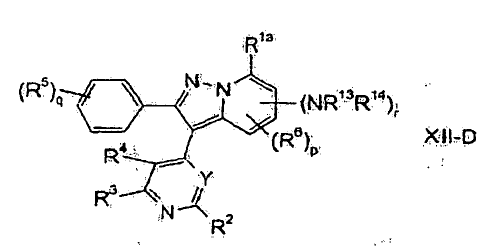 dans laquelle r est 2 ou 3.

32. Procédé selon l'une quelconque des revendications 26 à 31 comprenant de plus l'étape de conversion d'un composé de formule (I) en un sel ou solvate de ceux-ci acceptable du point de vue pharmaceutique.

33. Procédé selon l'une quelconque des revendications 26 à 32 comprenant de plus l'étape de conversion d'un composé de formule (I) ou d'un sel ou solvate de ceux-ci acceptable du point de vue physiologique en un autre composé de formule (I) ou sel ou solvate de ceux-ci acceptable du point de vue pharmaceutique.
